# EUROPEAN PATENT APPLICATION

(11) **EP 4 850 732 A2**
(43) Date of publication of application: **09.12.2026**
(21) Application number: 26212242.7
(22) Date of filing: 04.12.2013
(51) Int. Cl.: A61M 16/08

(54) **MEDICAL TUBES AND METHODS OF MANUFACTURE**

(30) Priority: 04.12.2012 US 201261733360 P; 04.12.2012 US 201261733359 P; 13.09.2013 US 201361877622 P; 13.09.2013 US 201361877566 P; 13.09.2013 US 201361877784 P; 13.09.2013 US 201361877736 P
(62) Document number(s) of the earlier application(s) in accordance with Art. 76 EPC: 25217637.5 / 4 714 483; 23217345.0 / 4 316 559; 20172616.3 / 3 708 212; 13859885.9 / 2 928 533
(71) Applicant: Fisher & Paykel Healthcare Limited, East Tamaki, Auckland 2013 (NZ)
(72) Inventor: Oosthuysen, Helgard, 2013 Auckland (NZ); Stoks, Elmo Benson, 2013 Auckland (NZ); North, Charles Christopher, 2013 Auckland (NZ); Sujau, Mahran Maumoon, 2013 Auckland (NZ); Stroobant, Joshua Daniel, 2013 Auckland (NZ); Millar, Gavin Walsh, 2013 Auckland (NZ); Buswell, Matthew Liam, 2013 Auckland (NZ); Al-Tiay, Ibrahim, 2013 Auckland (NZ); McCauley, David Leon, 2013 Auckland (NZ); Van Schalkwyk, Andre, 2013 Auckland (NZ); Munkelt, Katja, 2013 Auckland (NZ); Wilson, Matthew Robert, 2013 Auckland (NZ); Patel, Sanjay Parag, 2013 Auckland (NZ); D'Andrea, Dominique Richard, 2013 Auckland (NZ); Dover, Martin Grant, 2013 Auckland (NZ); Barker, Dean Antony, 2013 Auckland (NZ); Harwood, Jonathan David, 2013 Auckland (NZ); Amadio, Christopher Jaye, 2013 Auckland (NZ)
(74) Representative: Bird & Bird LLP

(57) **Abstract**

The present invention relates to a breathing tube for delivering humidified gases to a patient, the breathing tube comprising a first end and a second end; a connector at the first end of the tube comprising a lumen extending along a longitudinal axis and a wall surrounding the lumen, the lumen configured to define a gas-flow path when in use; a circuit board assembly comprising: a wiring portion comprising electrical contacts configured to be coupled to at least two conductive wires of the breathing tube, the wiring portion projecting outwardly of the connector in a direction away from the lumen; an extending portion extending across the lumen along a diameter or chord line and comprising a sensor portion, the sensor portion being positioned within the gas-flow path and comprising at least one temperature sensor; one or more conductive tracks configured to electrically connect the at least one temperature sensor to the electrical contacts; and, an overmolding composition provided over at least over the sensor portion; and, at least one diode electrically coupled to the at least one temperature sensor; the circuit board assembly being configured to position the at least one temperature sensor apart from the at least one diode to thermally insulate the at least one temperature sensor from the at least one diode.

## Description

### INCORPORATION BY REFERENCE

This application claims the benefit of priority under 35 U.S.C. § 119(e) to U.S. Provisional Application No. 61/733,359, entitled MEDICAL TUBES AND METHODS OF MANUFACTURE, filed on December 4, 2012; U.S. Provisional Application No. 61/733,360, entitled MEDICAL TUBES AND METHODS OF MANUFACTURE, filed on December 4, 2012; U.S. Provisional Application No. 61/877,622, entitled MEDICAL TUBES AND METHODS OF MANUFACTURE, filed on September 13, 2013; U.S. Provisional Application No. 61/877,566, entitled HUMIDIFICATION SYSTEM, filed on September 13, 2013; U.S. Provisional Application No. 61/877,784, entitled CONNECTIONS FOR HUMIDIFICATION SYSTEM, filed on September 13, 2013; and U.S. Provisional Application No. 61/877,736, entitled ZONE HEATING FOR RESPIRATORY CIRCUITS, filed on September 13, 2013, each of which is incorporated herein by reference in its entirety.

In addition, PCT Application No. PCT/IB2012/001786, entitled MEDICAL TUBES AND METHODS OF MANUFACTURE, filed May 30, 2012, is also incorporated herein by reference in its entirety.

### BACKGROUND

### Field

This disclosure relates generally to tubes suitable for medical use, and in particular to tubes for use in medical circuits suitable for providing gases to and/or removing gases from a patient, such as in positive airway pressure (PAP), respirator, anaesthesia, ventilator, and insufflation systems.

### Description of the Related Art

In medical circuits, various components transport warm and/or humidified gases to and from patients. For example, in some breathing circuits such as PAP or assisted breathing circuits, gases inhaled by a patient are delivered from a heater-humidifier through an inspiratory tube. As another example, tubes can deliver humidified gas (commonly CO₂) into the abdominal cavity in insufflation circuits. This can help prevent "drying out" of the patient's internal organs, and can decrease the amount of time needed for recovery from surgery. Unheated tubing allows significant heat loss to ambient cooling. This cooling may result in unwanted condensation or "rainout" along the length of the tubing transporting warm, humidified air. A need remains for tubing that insulates against heat loss and, for example, allows for improved temperature and/or humidity control in medical circuits. Accordingly, it is an object of the invention to overcome or ameliorate one or more of the disadvantages of the prior art or to at least provide the public with a useful choice.

### SUMMARY

Medical tubes and methods of manufacturing medical tubes are disclosed herein in various embodiments. In some embodiments, the tube may be a composite structure made of two or more distinct components that are spirally wound to form an elongate tube. For example, one of the components may be a spirally wound elongate hollow body, and the other component may be an elongate structural component also spirally wound between turns of the spirally wound hollow body In other embodiments, the tube need not be made from distinct components. For instance, an elongate hollow body formed (e.g., extruded) from a single material may be spirally wound to form an elongate tube. The elongate hollow body itself may in transverse cross-section have a thin wall portion and a relatively thicker or more rigid reinforcement portion. The tubes can be incorporated into a variety of medical circuits or may be employed for other medical uses.

In at least one embodiment, a composite tube can comprise a first elongate member comprising a hollow body spirally wound to form at least in part an elongate tube having a longitudinal axis, a lumen extending along the longitudinal axis, and a hollow wall surrounding the lumen. A second elongate member may be spirally wound and joined between adjacent turns of the first elongate member, the second elongate member forming at least a portion of the lumen of the elongate tube. The name "first elongate member" and "second elongate member" do not necessarily connote an order, such as the order in which the components are assembled. As described herein, the first elongate member and the second elongate member can also be portions of a single tube-shaped element.

In various embodiments, the foregoing component has one, some, or all of the following properties, as well as properties described elsewhere in this disclosure.

The first elongate member can be a tube. The first elongate member can form in longitudinal cross-section a plurality of bubbles with a flattened surface at the lumen. Adjacent bubbles can be separated by a gap above the second elongate member, or may not be directly connected to each other. The bubbles can have perforations. The second elongate member can have a longitudinal cross-section that is wider proximal the lumen and narrower at a radial distance from the lumen. Specifically, the second elongate member can have a longitudinal cross-section that is generally triangular, generally T-shaped, or generally Y-shaped. One or more conductive filaments can be embedded or encapsulated in the second elongate member. The one or more conductive filaments can be heating filaments (or more specifically, resistance heating filaments) and/or sensing filaments. The tube can comprise pairs of conductive filaments, such as two or four conductive filaments. Pairs of conductive filaments can be formed into a connecting loop at one end of the composite tube. The one or more conductive filaments can be spaced from the lumen wall. In at least one embodiment, the second elongate member can have a longitudinal cross-section that is generally triangular, generally T-shaped, or generally Y-shaped, and one or more conductive filaments can be embedded or encapsulated in the second elongate member on opposite sides of the triangle, T-shape, or Y-shape.

The foregoing component according to any or all of the preceding embodiments can be incorporated into a medical circuit component, an inspiratory tube, an expiratory tube, a PAP component, an insufflation circuit, an exploratory component, or a surgical component, among other applications.

A method of manufacturing a composite tube is also disclosed. The resulting tube can have one, some, or all of the properties described above or anywhere in this disclosure. In at least one embodiment, the method comprises providing a first elongate member comprising a hollow body and a second elongate member configured to provide structural support for the first elongate member. The second elongate member is spirally wrapped around a mandrel with opposite side edge portions of the second elongate member being spaced apart on adjacent wraps, thereby forming a second-elongate-member spiral. The first elongate member is spirally wrapped around the second-elongate-member spiral, such that portions of the first elongate member overlap adjacent wraps of the second-elongate-member spiral and a portion of the first elongate member is disposed adjacent the mandrel in the space between the wraps of the second-elongate-member spiral, thereby forming a first-elongate-member spiral.

In various embodiments, the foregoing method can comprise one, some, or all of the following. The method can comprise supplying air at a pressure greater than atmospheric pressure to an end of the first elongate member. The method can comprise cooling the second-elongate-member spiral and the first-elongate-member spiral, thereby forming a composite tube having a lumen extending along a longitudinal axis and a hollow space surrounding the lumen. The method can comprise forming the first elongate member. The method can comprise extruding the first elongate member with a first extruder. The method can comprise forming the second elongate member. The method can comprise extruding the second elongate member with a second extruder. The second extruder can be configured to encapsulate one or more conductive filaments in the second elongate member. Forming the second elongate member can comprise embedding conductive filaments in the second elongate member. The conductive filaments can be non-reactive with the second elongate member. The conductive filaments can comprise alloys of aluminum or copper or other conductive materials. The method can comprise forming pairs of conductive filaments into a connecting loop at one end of the composite tube. The first extruder can be distinct from the second extruder.

A medical tube is also disclosed. In at least one embodiment, the tube comprises an elongate hollow body spirally wound to form an elongate tube having a longitudinal axis, a lumen extending along the longitudinal axis, and a hollow wall surrounding the lumen, wherein the elongate hollow body has in transverse cross-section a wall defining at least a portion of the hollow body. The tube can further comprise a reinforcement portion extending along a length of the elongate hollow body being spirally positioned between adjacent turns of the elongate hollow body, wherein the reinforcement portion forms a portion of the lumen of the elongate tube. The reinforcement portion can be relatively thicker or more rigid than the wall of the elongate hollow body.

In various embodiments, the foregoing tube has one, some, or all of the following properties, as well as properties described elsewhere in this disclosure. The reinforcement portion can be formed from the same piece of material as the elongate hollow body. The elongate hollow body in transverse cross-section can comprise two reinforcement portions on opposite sides of the elongate hollow body, wherein spiral winding of the elongate hollow body joins adjacent reinforcement portions to each other such that opposite edges of the reinforcement portions touch on adjacent turns of the elongate hollow body. Opposite side edges of the reinforcement portions can overlap on adjacent turns of the elongate hollow body. The reinforcement portion can be made of a separate piece of material than the elongate hollow body. The hollow body can form in longitudinal cross-section a plurality of bubbles with a flattened surface at the lumen. The bubbles can have perforations. The medical tube can also comprise one or more conductive filaments embedded or encapsulated within the reinforcement portion. The conductive filament can be a heating filament and/or or sensing filament. The medical tube can comprise two conductive filaments, wherein one conductive filament is embedded or encapsulated in each of the reinforcement portions. The medical tube can comprise two conductive filaments positioned on only one side of the elongate hollow body. Pairs of conductive filaments can be formed into a connecting loop at one end of the elongate tube. The one or more filaments can be spaced from the lumen wall.

The foregoing tube according to any or all of the preceding embodiments can be incorporated into a medical circuit component, an inspiratory tube, an expiratory tube, a PAP component, an insufflation circuit, an exploratory component, or a surgical component, among other applications.

A method of manufacturing a medical tube is also disclosed. In at least one embodiment, the method comprises spirally winding an elongate hollow body around a mandrel to form an elongate tube having a longitudinal axis, a lumen extending along the longitudinal axis, and a hollow wall surrounding the lumen, wherein the elongate hollow body has in transverse cross-section a wall defining at least a portion of the hollow body and two reinforcement portions on opposite sides of the elongate body forming a portion of the wall of the lumen, the two reinforcement portions being relatively thicker or more rigid than the wall defining at least a portion of the hollow body. The method can further comprise joining adjacent reinforcement portions to each other such that opposite edges of the reinforcement portions touch on adjacent turns of the elongate hollow body.

In various embodiments, the foregoing method can comprise one, some, or all of the following or any other properties described elsewhere in this disclosure. Joining adjacent reinforcement portions to each other can cause edges of the reinforcement portions to overlap. The method can further comprise supplying air at a pressure greater than atmospheric pressure to an end of the elongate hollow body. The method can further comprise cooling the elongate hollow body to join the adjacent reinforcement portions to each other. The method can further comprise extruding the elongate hollow body. The method can further comprise embedding conductive filaments in the reinforcement portions. The method can further comprise forming pairs of conductive filaments into a connecting loop at one end of the elongate tube.

A breathing tube is also disclosed. In at least one embodiment, the tube comprises a first elongate member comprising a hollow body spirally wound to form at least in part an elongate tube having a longitudinal axis, a lumen extending along the longitudinal axis, and a hollow wall surrounding the lumen, the wall having an inner portion proximal the lumen and an outer portion facing away from the lumen, wherein the inner portion of the wall has a smaller thickness than the outer portion of the wall.

In various embodiments, the foregoing breathing tube can comprise one, some, or all of the following properties or any other properties described elsewhere in this disclosure. The breathing tube can further comprising a second elongate member spirally wound and joined between adjacent turns of the first elongate member, the second elongate member forming at least a portion of the lumen of the elongate tube. The thickness of the outer portion of the wall can be in the range of about 0.14 mm and about 0.44 mm. The thickness of the outer portion of the wall can be about 0.24 mm. The thickness of the inner portion of the wall can be in the range of about 0.05 mm and about 0.30 mm. The thickness of the inner portion of the wall can be about 0.10 mm.

A breathing tube is also disclosed. In at least one embodiment, the tube comprises a first elongate member comprising a hollow body spirally wound to form at least in part an elongate tube having a longitudinal axis, a lumen extending along the longitudinal axis, and a hollow wall surrounding the lumen, the hollow body forming in longitudinal cross section a plurality of bubbles, a bubble having a maximum width along the longitudinal axis and a maximum height perpendicular to the longitudinal axis between the outward-facing apex of the wall and the lumen, wherein the ratio of the maximum height to the maximum width is at least about 0.16.

In various embodiments, the foregoing breathing tube can comprise one, some, or all of the following properties or any other properties described elsewhere in this disclosure. The breathing tube can further comprise a second elongate member spirally wound and joined between adjacent turns of the first elongate member, the second elongate member forming at least a portion of the lumen of the elongate tube. The maximum height can be in the range of about 1.2 mm and about 8.2 mm. The maximum height can be about 3.2 mm. The maximum width can be in the range of about 3.5 mm and about 7.5 mm. The maximum width can be about 5.5 mm. The ratio of the maximum height to the maximum width can be greater than 1.0.

A breathing tube is also disclosed. In at least one embodiment, the tube comprises a first elongate member comprising a hollow body spirally wound to form at least in part an elongate tube having a longitudinal axis, a lumen extending along the longitudinal axis, and a hollow wall surrounding the lumen, the hollow body forming in longitudinal cross section a plurality of bubbles, wherein a vertical distance between corresponding points on adjacent bubbles defines a pitch, wherein the ratio of pitch to the maximum outer diameter of the composite tube is less than about 0.35.

In various embodiments, the foregoing breathing tube can comprise one, some, or all of the following properties or any other properties described elsewhere in this disclosure. The breathing tube can further comprising a second elongate member spirally wound and joined between adjacent turns of the first elongate member, the second elongate member forming at least a portion of the lumen of the elongate tube. The pitch can be in the range of about 1.2 mm and about 8.1 mm. The pitch can be about 5.1 mm. The maximum outer diameter can be in the range of about 19.5 mm and 25.5 mm. The maximum outer diameter can be about 22.5 mm.

A composite tube is also disclosed. In at least one embodiment, the tube comprises a first elongate member comprising a hollow body spirally wound to form at least in part an elongate tube having a longitudinal axis, a lumen extending along the longitudinal axis, and a hollow wall surrounding the lumen, the hollow body forming in longitudinal cross section a plurality of bubbles, a bubble having a maximum height, perpendicular to the longitudinal axis, between the outward-facing apex of the wall and the lumen that defines the maximum height of the first elongate member; and a second elongate member spirally wound and joined between adjacent turns of the first elongate member, the second elongate member forming at least a portion of the lumen of the elongate tube, the second elongate member having a maximum height, perpendicular to the longitudinal axis, between the outward-facing apex of the second elongate member and the lumen, wherein the ratio of the difference between the maximum height of the first elongate member and the maximum height of the second elongate member to the maximum outer diameter of the composite tube is less than about 0.049: 1.

In various embodiments, the foregoing composite tube can comprise one, some, or all of the following properties or any other properties described elsewhere in this disclosure. The wall can have an inner portion proximal the lumen and an outer portion facing away from the lumen and the inner portion of the wall has a smaller thickness than the outer portion of the wall.

A composite tube is also disclosed. In at least one embodiment, the tube comprises a a first elongate member comprising a hollow body spirally wound to form at least in part an elongate tube having a longitudinal axis, a lumen extending along the longitudinal axis, and a hollow wall surrounding the lumen, the wall having an inner portion proximal the lumen and an outer portion facing away from the lumen; and a second elongate member spirally wound between adjacent turns of the first elongate member, the second elongate member forming at least a portion of the lumen of the elongate tube and the first elongate member being joined at connection points on adjacent turns of the second elongate member; wherein the composite tube's bend radius is limited by the length of the outer portion between the connection points.

In various embodiments, the foregoing composite tube can comprise one, some, or all of the following properties or any other properties described elsewhere in this disclosure. The wall has an inner portion proximal the lumen and an outer portion facing away from the lumen and the inner portion of the wall has a smaller thickness than the outer portion of the wall.

A breathing tube is also disclosed. In at least one embodiment, the tube comprises a first elongate member comprising a hollow body component, wherein the weight per length of the tube within at least a portion of the 300 mm nearest an end of the tube is less than about 0.08 g/mm.

In various embodiments, the foregoing breathing tube can comprise one, some, or all of the following properties or any other properties described elsewhere in this disclosure. The first elongate member can comprise a hollow body spirally wound to form at least in part an elongate tube having a longitudinal axis, a lumen extending along the longitudinal axis, and a hollow wall surrounding the lumen. The breathing tube can further comprise a second elongate member spirally wound and joined between adjacent turns of the first elongate member, the second elongate member forming at least a portion of the lumen of the elongate tube. The breathing tube can comprise one or more conductive filaments embedded or encapsulated within the second elongate member. At least one of the one or more conductive filaments can be a heating filament. At least one of the one or more conductive filaments can be a sensing filament. The tube mass in the 300 mm nearest an end of the tube can be less than about 24 g. The weight per length of the tube within at least a portion of the 300 mm nearest an end of the tube can be less than about 0.06 g/mm. The tube mass in the 300 mm nearest an end of the tube can be less than about 16 g. The thickness of the wall can be at most about 0.50 mm.

A breathing tube is also disclosed. In at least one embodiment, the tube comprises a first elongate member comprising a hollow body spirally wound to form at least in part an elongate tube having a longitudinal axis, a lumen extending along the longitudinal axis, and a hollow wall surrounding the lumen, the wall having an inner portion proximal the lumen and an outer portion facing away from the lumen, wherein, in at least a portion of the composite tube, when force is applied to the outer portion of the wall with a 2.5-mm probe until the outer portion of the wall contacts the inner portion, the outer portion deflects by a vertical distance that satisfies the equation: *D* > 0.5×*F*_{2.5}, where *D* represents the vertical distance in millimeters, and *F*_{2.5} represents the force in Newtons applied by the 2.5-mm probe.

In various embodiments, the foregoing breathing tube can comprise one, some, or all of the following properties or any other properties described elsewhere in this disclosure. The breathing tube can further comprise a second elongate member spirally wound and joined between adjacent turns of the first elongate member, the second elongate member forming at least a portion of the lumen of the elongate tube. The outer portion can deflect more than about 1 mm when a force of about 1 N is applied with the 2.5-mm probe.

A conduit suitable for use with a tube for delivering humidified gases to a patient is also disclosed. In at least one embodiment, the conduit comprises a connector configured to connect to the tube, the connector comprising a lumen extending along a longitudinal axis and walls surrounding the lumen, the lumen defining a flow path for the humidified gases when in use; and a printed circuit board assembly comprising a printed circuit board and further comprising a dividing portion embedded in the walls of the connector and extending across the lumen of the connector along a diameter or chord line, such that the dividing portion generally bisects at least part of the flow path, at least part of the dividing portion being overmolded by an overmolding composition, a wiring portion adjacent the dividing portion and projecting outward from the wall of the connector in a direction away from the lumen of the connector, and a sensor portion disposed in the lumen of the connector and projecting from the dividing portion along the longitudinal axis, the sensor portion comprising at least one sensor, and the sensor portion being overmolded by the overmolding composition.

In various embodiments, the foregoing conduit can comprise one, some, or all of the following properties or any other properties described elsewhere in this disclosure. The printed circuit board assembly can further comprise a support portion adjacent the dividing portion and projecting outward from the connector in a direction away from the lumen and in a direction opposite the wiring portion. The wiring portion can be configured to electrically connect to one or more heater wires from the conduit. The at least one sensor can comprise a thermistor. The sensor portion can project upstream of the flow path. The at least one sensor can comprise a sensor adjacent an upstream leading edge of the sensor portion. The sensor portion can project downstream of the flow path. The at least one sensor can comprise a sensor adjacent a downstream leading edge of the sensor portion. The overmolding composition proximal the sensor portion can have a tapered shape extending along the longitudinal axis. The overmolding can be thinnest proximal a leading edge of the sensor portion. The sensor portion can have an airfoil shape extending along the longitudinal axis. The sensor portion can have a bullet or torpedo shape.

A respiratory conduit is also disclosed. In at least one embodiment, the conduit comprises a lumen extending along a longitudinal axis and a wall surrounding the lumen, the lumen defining a gas-flow path when in use; and an overmolded printed circuit board assembly secured to the wall, the printed circuit board assembly comprising a printed circuit board and further comprising a mount portion disposed in the lumen of the connector and projecting along the longitudinal axis, and a temperature sensor on a surface of the mount portion.

In various embodiments, the foregoing conduit can comprise one, some, or all of the following properties or any other properties described elsewhere in this disclosure. The temperature sensor can be a thermistor.

A respiratory conduit is also disclosed. In at least one embodiment, the conduit comprises a lumen extending along a longitudinal axis and walls surrounding the lumen, the lumen defining a gas-flow path when in use; and a component secured to the walls and extending across the lumen along a diameter or chord line, such that the component generally bisects at least part of the flow path, the component comprising a mount portion disposed in the lumen and projecting along the longitudinal axis, a temperature sensor on a surface of the mount portion, and electrical connection to the sensor.

In various embodiments, the foregoing conduit can comprise one, some, or all of the following properties or any other properties described elsewhere in this disclosure. The temperature sensor can be a thermistor. The component can be a printed circuit board. The electrical connection can span the component's length along the diameter or chord line.

A respiratory conduit is also disclosed. In at least one embodiment, the conduit comprises a lumen extending along a longitudinal axis and a wall surrounding the lumen, the lumen defining a gas-flow path when in use; and an overmolded printed circuit board assembly secured to the wall, the printed circuit board assembly comprising a printed circuit board and further comprising a mount portion disposed in the lumen and projecting along the longitudinal axis, and a temperature sensor on a surface of the mount portion, wherein the overmolding proximal the mount portion has a tapered shape.

In various embodiments, the foregoing conduit can comprise one, some, or all of the following properties or any other properties described elsewhere in this disclosure. The temperature sensor can be a thermistor.

A respiratory conduit is also disclosed. In at least one embodiment, the conduit comprises a lumen extending along a longitudinal axis and a wall surrounding the lumen, the lumen defining a gas-flow path when in use; and a component connected to the wall and comprising a mount portion disposed in the lumen and projecting along the longitudinal axis, the mount portion comprising a temperature sensor positioned longitudinally upstream from the connection to the wall.

In various embodiments, the foregoing breathing tube can comprise one, some, or all of the following properties or any other properties described elsewhere in this disclosure. The temperature sensor can be a thermistor. The temperature sensor can be proximal an upstream extreme of the mount portion. The mount portion can be overmolded. The overmolding can be thinnest proximal the temperature sensor. The mount can project longitudinally downstream. The mount can have an airfoil shape extending along the longitudinal axis. The mount can have a bullet or torpedo shape. A vertical distance between the mount and the wall can be at least 30% of the lumen's diameter.

A respiratory conduit segment is also disclosed. In at least one embodiment, the segment comprises a lumen extending along a longitudinal axis and a wall surrounding the lumen, the lumen defining a gas-flow path when in use; and a printed circuit board assembly comprising a printed circuit board and comprising a first portion extending across the lumen along a diameter or chord line, such that a portion of the printed circuit board assembly generally bisects at least part of the flow path, the first portion being overmolded by an overmolding composition, a second portion adjacent the first portion projecting outward from the wall in a direction away from the lumen, the second portion comprising one or more connection pads on the printed circuit board configured to receive one or more wires from a first assembly, a third portion adjacent the first portion projecting outward from the wall in a direction away from the lumen and in a direction opposite the second portion, the third portion comprising one or more connection pads on the printed circuit board configured to receive one or more wires from a second assembly that is distinct from the first assembly, and one or more conductive tracks on the printed circuit board electrically coupled to the one or more connection pads of the second portion and to the one or more connection pads of the third portion and configured to provide electrical connectivity between the first assembly and the second assembly.

In various embodiments, the foregoing segment can comprise one, some, or all of the following properties or any other properties described elsewhere in this disclosure. The first assembly can be a breathing tube. The second assembly can be a breathing tube. The printed circuit board assembly can further comprise a mount portion disposed in the lumen of the connector and projecting along the longitudinal axis, and a temperature sensor on a surface of the mount portion.

In various embodiments, a breathing tube comprises a first elongate member comprising a hollow body spirally wound to form at least in part an elongate tube having a longitudinal axis, a lumen extending along the longitudinal axis, and a hollow wall surrounding the lumen, the hollow body forming in longitudinal cross section a plurality of bubbles, a bubble having a maximum width along the longitudinal axis and a maximum height perpendicular to the longitudinal axis between the outward-facing apex of the wall and the lumen, wherein the ratio of the maximum height to the maximum width is at least about 0.16. A second elongate member may be spirally wound and joined between adjacent turns of the first elongate member, the second elongate member forming at least a portion of the lumen of the elongate tube. The maximum height may be in the range of about 0.7 mm and about 7.7 mm. The maximum height may be about 2.7 mm. The maximum width may be in the range of about 2.0 mm and about 6.0 mm. The maximum width may be about 4.0 mm. The maximum height to the maximum width may be greater than 1.0.

In various embodiments, a breathing tube comprises a first elongate member comprising a hollow body spirally wound to form at least in part an elongate tube having a longitudinal axis, a lumen extending along the longitudinal axis, and a hollow wall surrounding the lumen, the hollow body forming in longitudinal cross section a plurality of bubbles, wherein a vertical distance between corresponding points on adjacent bubbles defines a pitch, wherein the ratio of pitch to the maximum outer diameter of the composite tube is less than about 0.35. A second elongate member may be spirally wound and joined between adjacent turns of the first elongate member, the second elongate member forming at least a portion of the lumen of the elongate tube. The pitch may be in the range of about 1.2 mm and about 8.1 mm. The pitch may be about 5.1 mm. The maximum outer diameter may be in the range of about 19.5 mm and 25.5 mm. The maximum outer diameter may be about 22.5 mm.

In various embodiments, a composite tube comprises a first elongate member comprising a hollow body spirally wound to form at least in part an elongate tube having a longitudinal axis, a lumen extending along the longitudinal axis, and a hollow wall surrounding the lumen, the hollow body forming in longitudinal cross section a plurality of bubbles, a bubble having a maximum height, perpendicular to the longitudinal axis, between the outward-facing apex of the wall and the lumen that defines the maximum height of the first elongate member; and a second elongate member spirally wound and joined between adjacent turns of the first elongate member, the second elongate member forming at least a portion of the lumen of the elongate tube, the second elongate member having a maximum height, perpendicular to the longitudinal axis, between the outward-facing apex of the second elongate member and the lumen, wherein the ratio of the difference between the maximum height of the first elongate member and the maximum height of the second elongate member to the maximum outer diameter of the composite tube is less than about 0.049:1. The wall may have an inner portion proximal the lumen and an outer portion facing away from the lumen and the inner portion of the wall may have a smaller thickness than the outer portion of the wall.

In various embodiments, a composite tube comprises a first elongate member comprising a hollow body spirally wound to form at least in part an elongate tube having a longitudinal axis, a lumen extending along the longitudinal axis, and a hollow wall surrounding the lumen, the wall having an inner portion proximal the lumen and an outer portion facing away from the lumen; and a second elongate member spirally wound between adjacent turns of the first elongate member, the second elongate member forming at least a portion of the lumen of the elongate tube and the first elongate member being joined at connection points on adjacent turns of the second elongate member; wherein the composite tube's bend radius is limited by the length of the outer portion between the connection points. The wall may have an inner portion proximal the lumen and an outer portion facing away from the lumen and the inner portion of the wall may have a smaller thickness than the outer portion of the wall.

In various embodiments, a conduit suitable for use with a tube for delivering humidified gases to a patient is provided, the conduit comprising a connector configured to connect to the tube, the connector comprising a lumen extending along a longitudinal axis and walls surrounding the lumen, the lumen defining a flow path for the humidified gases when in use; and a printed circuit board assembly comprising a printed circuit board and further comprising a dividing portion embedded in the walls of the connector and extending across the lumen of the connector along a diameter or chord line, such that the dividing portion generally bisects at least part of the flow path, at least part of the dividing portion being overmolded by an overmolding composition, a wiring portion adjacent the dividing portion and projecting outward from the wall of the connector in a direction away from the lumen of the connector, and a sensor portion disposed in the lumen of the connector and projecting from the dividing portion along the longitudinal axis, the sensor portion comprising at least one sensor, and the sensor portion being overmolded by the overmolding composition. The printed circuit board assembly may further comprise a support portion adjacent the dividing portion and projecting outward from the connector in a direction away from the lumen and in a direction opposite the wiring portion. The wiring portion may be configured to electrically connect to one or more heater wires from the conduit. The at least one sensor may comprise a thermistor. The sensor portion may project upstream of the flow path. The at least one sensor may comprise a sensor adjacent an upstream leading edge of the sensor portion. The sensor portion may project downstream of the flow path. The at least one sensor may comprise a sensor adjacent a downstream leading edge of the sensor portion. The overmolding composition proximal the sensor portion may have a tapered shape extending along the longitudinal axis. The overmolding may be thinnest proximal a leading edge of the sensor portion. The sensor portion may have an airfoil shape extending along the longitudinal axis. The sensor portion may have a bullet or torpedo shape.

In various embodiments, a respiratory conduit comprises a lumen extending along a longitudinal axis and a wall surrounding the lumen, the lumen defining a gas-flow path when in use; and an overmolded printed circuit board assembly secured to the wall, the printed circuit board assembly comprising a printed circuit board and further comprising a mount portion disposed in the lumen and projecting along the longitudinal axis, and a temperature sensor on a surface of the mount portion, wherein the overmolding proximal the mount portion has a tapered shape. The temperature sensor may be a thermistor.

In various embodiments, a respiratory conduit comprises a lumen extending along a longitudinal axis and a wall surrounding the lumen, the lumen defining a gas-flow path when in use; and a component connected to the wall and comprising a mount portion disposed in the lumen and projecting along the longitudinal axis, the mount portion comprising a temperature sensor positioned longitudinally upstream from the connection to the wall. The temperature sensor may be a thermistor. The temperature sensor may be proximal an upstream extreme of the mount portion. The mount portion may be overmolded. The overmolding may be thinnest proximal the temperature sensor. The mount may project longitudinally downstream. The mount may have an airfoil shape extending along the longitudinal axis. The mount may have a bullet or torpedo shape. A vertical distance between the mount and the wall may be at least 30% of the lumen's diameter.

In various embodiments, a respiratory conduit segment comprises a lumen extending along a longitudinal axis and a wall surrounding the lumen, the lumen defining a gas-flow path when in use; and a printed circuit board assembly comprising a printed circuit board and comprising a first portion extending across the lumen along a diameter or chord line, such that a portion of the printed circuit board assembly generally bisects at least part of the flow path, the first portion being overmolded by an overmolding composition, a second portion adjacent the first portion projecting outward from the wall in a direction away from the lumen, the second portion comprising one or more connection pads on the printed circuit board configured to receive one or more wires from a first assembly, a third portion adjacent the first portion projecting outward from the wall in a direction away from the lumen and in a direction opposite the second portion, the third portion comprising one or more connection pads on the printed circuit board configured to receive one or more wires from a second assembly that is distinct from the first assembly, and one or more conductive tracks on the printed circuit board electrically coupled to the one or more connection pads of the second portion and to the one or more connection pads of the third portion and configured to provide electrical connectivity between the first assembly and the second assembly. The first assembly may be a breathing tube. The second assembly may be a breathing tube. The printed circuit board assembly may further comprise a mount portion disposed in the lumen of the connector and projecting along the longitudinal axis, and a temperature sensor on a surface of the mount portion.

In various embodiments, a composite tube comprises a first elongate member comprising a hollow body spirally wound to form at least in part an elongate tube having a longitudinal axis, a lumen extending along the longitudinal axis, and a hollow wall surrounding the lumen; a second elongate member spirally wound and joined between adjacent turns of the first elongate member, the second elongate member forming at least a portion of the lumen of the elongate tube; wherein at least a portion of the first elongate member is formed of a breathable material. In one example, the composite tube may be provided with a source of humidification fluid and/or be pre-charged with a volume of humidification fluid, and a heater provided to heat the fluid such that fluid vapour passes through the breathable material into or from the lumen. The heater may comprise one or more heating filaments disposed in the second elongate member.

For purposes of summarizing the invention, certain aspects, advantages and novel features of the invention have been described herein. It is to be understood that not necessarily all such advantages may be achieved in accordance with any particular embodiment of the invention. Thus, the invention may be embodied or carried out in a manner that achieves or optimizes one advantage or group of advantages as taught herein without necessarily achieving other advantages as may be taught or suggested herein.

### BRIEF DESCRIPTION OF THE DRAWINGS

Example embodiments that implement the various features of the disclosed systems and methods will now be described with reference to the drawings. The drawings and the associated descriptions are provided to illustrate embodiments and not to limit the scope of the disclosure.
FIG. 1 shows a schematic illustration of a medical circuit incorporating one or more medical tubes.
FIG. **2A** shows a side-plan view of a section of an example composite tube.
FIG. **2B** shows a longitudinal cross-section of a top portion a tube similar to the example composite tube of FIG. **2A****.**
FIG. **2C** shows another longitudinal cross-section illustrating a first elongate member in the composite tube.
FIG. **2D** shows another longitudinal cross-section of a top portion of a tube.
FIG. **2E** shows another longitudinal cross-section of a top portion of a tube.
FIG. **2F** shows a tube with a portion exposed in longitudinal cross-section.
FIG. **2G** shows a longitudinal cross-section of a portion of a tube similar to the example tube of FIG. **2F****.**
FIG. **2H** shows a longitudinal cross-section of a top portion of a tube.
FIG. **3** shows a jig suitable for determining bubble deflection.
FIG. **4** shows a curve of force vs. bubble deflection.
FIGS. **5A-5C** show examples of first elongate member shapes configured to improve thermal efficiency.
FIGS. **5D-5F** show examples of filament arrangements configured to improve thermal efficiency.
FIG. **6A** shows a longitudinal cross section of a portion of a composite tube in a neutral position.
FIG. **6B** shows the portion of the composite tube of FIG. **6A** in a bent position, in which the composite tube has been bent to a ∩-shape.
FIG. **6C** shows a composite tube that has been bent to a ∩-shape.
FIG. **6D** shows a composite tube that has been bent beyond the minimum radius of curvature.
FIG. **7A** shows a transverse cross-section of a second elongate member in the composite tube.
FIG. **7B** shows another transverse cross-section of a second elongate member.
FIG. **7C** shows another example second elongate member.
FIG. **7D** shows another example second elongate member.
FIG. **7E** shows another example second elongate member.
FIG. **7F** shows another example second elongate member.
FIG. **7G** shows another example second elongate member.
FIG. **8A** shows a schematic of a composite tube with a variable pitch.
FIG. **8B** is a graph depicting an example temperature profile in a variable-pitch composite tube.
FIG. **9A** shows a front-plan cross-sectional schematic of a flexibility jig.
FIG. **9B** shows a detailed front-plan cross-sectional schematic of rollers on the flexibility jig of FIG. **9A****.**
FIGS. **9C-9F** show a flexibility jig in use. FIGS. **9C** and **9E** show a front-perspective view of samples under testing in the jig. FIGS. **9D** and **9F** show a rear-perspective view of samples under testing in the jig.
FIG. **10A** shows a crush resistance testing jig.
FIG. **10B** shows a plot of load vs. extension, used for determining crush stiffness.
FIGS. **11A-11D** demonstrate radius of curvature properties of tubes.
FIGS. **12A-12C** show examples of first elongate member stacking.
FIG. **13** shows an alternative embodiment of the second elongate member.
FIGS. **14A-14E** show variations of a tube, adapted to provide increased lateral stretch in the tube.
FIGS. **15A-15E** show a stretched state of the tubes shown in FIGS. **13A-E,** respectively.
FIG. **16** shows an example medical circuit according to at least one embodiment.
FIG. **17** shows an insufflation system according to at least one embodiment.
FIG. **18** is a schematic illustration of a coaxial tube, according to at least one embodiment.
FIGS. **19A-19B** show a composite tube in use with a patient interface.
FIG. **20A** shows a composite tube in use with a full face mask.
FIG. **20B** shows a composite tube in use with a nasal mask.
FIG. **20C** shows a composite tube in use with a nasal/pillow mask.
FIG. **21A** shows an aspect in a method for forming the composite tube.
FIG. **21B** shows a spiral-wound second elongate member.
FIG. **21C** shows another aspect in a method for forming the composite tube.
FIG. **21D** shows another aspect in a method for forming the composite tube.
FIG. **21E** shows another aspect in a method for forming the composite tube.
FIG. **21F** shows another aspect in a method for forming the composite tube.
FIG. **22A-22C** show example configurations of longitudinal cross sections of tubes.
FIGS. **23A-23H** show an alternative method of forming a tube.
FIGS. **24A-24B** show another example illustrating a single elongate hollow body being spirally wound to form a medical tube.
FIGS. **24C-24F** show examples of other single elongate hollow bodies being spirally wound to form a medical tube.
FIGS. **25A-25L** show a general flow chart and more detailed schematics and photographs relating to a method for attaching a connector to the end of the tube that is configured in use to connect to a humidifier.
FIGS. **26A-26E** show a connector for attaching filaments to an electrical connector.
FIGS. **27A-27E** show a clamshell suitable for use with the connector of FIGS. **25A-25L****.**
FIGS. **28A-28F** and **29A-29L** show connectors that can be used for medical circuits having electrical wires running therethrough and associated methods of assembly.
FIGS. **30A-30O** show schematics relating to a connector suitable for attaching a tube to a patient interface.
FIGS. **31A-31B** show a stop portion suitable for use with the connector of FIGS. **30A-30O****.**
FIGS. **32A-32D** show an anti-rotation feature suitable for use with the connector of FIGS. **30A-30O****.**
FIGS. **33A-33D** illustrate an example PCB assembly.
FIG. **34** illustrates a segmented inspiratory limb for use with a humidification system, the segmented inspiratory limb having an intermediate connector configured to couple heating filaments and/or temperature sensors in the two segments.
FIGS. **35A-35E** show schematics relating to a connector suitable for attaching a tube to a humidifier port, patient interface, or any other suitable component.
FIGS. **36A-36K** show schematics relating to another connector suitable for attaching a tube to a humidifier port, patient interface, or any other suitable component.
FIG. **37A** shows a longitudinal cross-section of a top portion of a tube comprising two first elongate members.
FIG. **37B** shows another longitudinal cross-section of a top portion of a tube comprising two first elongate members.

Generally throughout the drawings, reference numbers are reused to indicate correspondence between referenced (or similar) elements. Nevertheless, corresponding referenced (or similar) elements may have different reference numbers in some circumstances. In addition, the first digit(s) of each reference number generally indicate the figure in which the element first appears.

### DETAILED DESCRIPTION

Details regarding several illustrative embodiments for implementing the apparatuses and methods described herein are described below with reference to the figures. The invention is not limited to these described embodiments.

### Breathing Circuit Comprising One Or More Medical Tubes

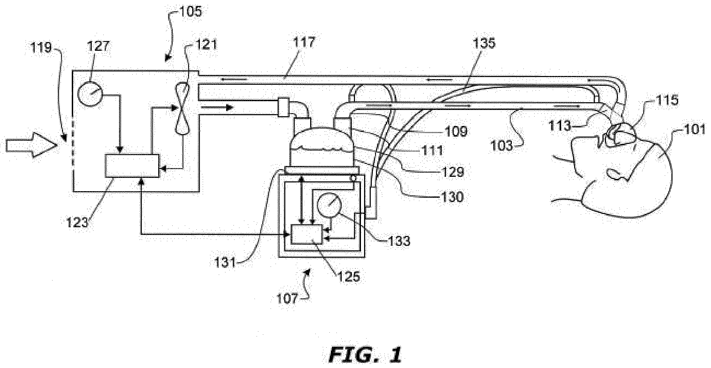

For a more detailed understanding of the disclosure, reference is first made to FIG. 1, which shows a breathing circuit according to at least one embodiment, which includes one or more medical tubes. Tube is a broad term and is to be given its ordinary and customary meaning to a person of ordinary skill in the art (that is, it is not to be limited to a special or customized meaning) and includes, without limitation, cylindrical and non-cylindrical passageways. Certain embodiments may incorporate a composite tube, which may generally be defined as a tube comprising two or more portions, or, specifically, in some embodiments, two or more components, as described in greater detail below. Such a breathing circuit can be a continuous, variable, or bi-level positive airway pressure (PAP) system or other form of respiratory therapy.

Gases can be transported in the circuit of FIG. **1** as follows. Dry gases pass from a ventilator/blower **105** to a humidifier **107,** which humidifies the dry gases. The humidifier **107** connects to the inlet **109** (the end for receiving humidified gases) of the inspiratory tube **103** via a port **111,** thereby supplying humidified gases to the inspiratory tube **103.** An inspiratory tube is a tube that is configured to deliver breathing gases to a patient, and may be made from a composite tube as described in further detail below. The gases flow through the inspiratory tube **103** to the outlet **113** (the end for expelling humidified gases), and then to the patient **101** through a patient interface **115** connected to the outlet **113.**

An expiratory tube **117** optionally connects to the patient interface **115.** An expiratory tube is a tube that is configured to move exhaled humidified gases away from a patient. Here, the expiratory tube **117** returns exhaled humidified gases from the patient interface **115** to the ventilator/blower **105.**

In this example, dry gases enter the ventilator/blower **105** through a vent **119.** A fan **121** can improve gas flow into the ventilator/blower by drawing air or other gases through vent **119.** The fan **121** can be, for instance, a variable speed fan, where an electronic controller **123** controls the fan speed. In particular, the function of the electronic controller **123** can be controlled by an electronic master controller **125** in response to inputs from the master controller **125** and a user-set predetermined required value (preset value) of pressure or fan speed or gases flow rate via a dial **127.**

The humidifier **107** comprises a humidification chamber **129** containing a volume of water **130** or other suitable humidifying liquid. Preferably, the humidification chamber **129** is removable from the humidifier **107** after use. Removability allows the humidification chamber **129** to be more readily sterilized or disposed. However, the humidification chamber **129** portion of the humidifier **107** can be a unitary construction. The body of the humidification chamber **129** can be formed from a non-conductive glass or plastics material. But the humidification chamber **129** can also include conductive components. For instance, the humidification chamber **129** can include a highly heat-conductive base (for example, an aluminum base) contacting or associated with a heater plate **131** on the humidifier **107.**

The humidifier **107** can also include electronic controls. In this example, the humidifier **107** includes an electronic, analog or digital master controller **125.** Preferably, the master controller **125** is a microprocessor-based controller executing computer software commands stored in associated memory. In response to the user-set humidity or temperature value input via a user interface **133,** for example, and other inputs, the master controller **125** determines when (or to what level) to energize heater plate **131** to heat the water **130** within humidification chamber **129.**

Any suitable patient interface **115** can be incorporated. Patient interface is a broad term and is to be given its ordinary and customary meaning to a person of ordinary skill in the art (that is, it is not to be limited to a special or customized meaning) and includes, without limitation, masks (such as tracheal mask, face masks and nasal masks), cannulas, and nasal pillows. A temperature probe **135** can connect to the inspiratory tube **103** near the patient interface **115,** or to the patient interface **115.** The temperature probe **135** monitors the temperature near or at the patient interface **115.** A heating filament (not shown) associated with the temperature probe can be used to adjust the temperature of the patient interface **115** and/or inspiratory tube **103** to raise the temperature of the inspiratory tube **103** and/or patient interface **115** above the saturation temperature, thereby reducing the opportunity for unwanted condensation.

In FIG. **1****,** exhaled humidified gases are returned from the patient interface **115** to the ventilator/blower **105** via the expiratory tube **117.** The expiratory tube **117** can also be a composite tube, as described in greater detail below. However, the expiratory tube **117** can also be a medical tube as previously known in the art. In either case, the expiratory tube **117** can have a temperature probe and/or heating filament, as described above with respect to the inspiratory tube **103,** integrated with it to reduce the opportunity for condensation. Furthermore, the expiratory tube **117** need not return exhaled gases to the ventilator/blower **105.** Alternatively, exhaled humidified gases can be passed directly to ambient surroundings or to other ancillary equipment, such as an air scrubber/filter (not shown). In certain embodiments, the expiratory tube is omitted altogether.

### Composite Tubes

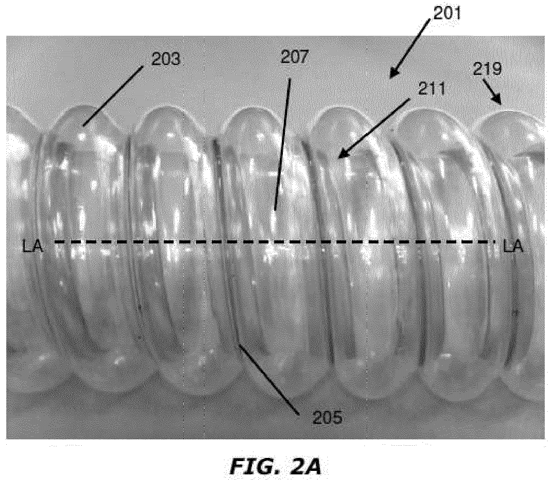

FIG. **2A** shows a side-plan view of a section of example composite tube **201.** In general, the composite tube **201** comprises a first elongate member **203** and a second elongate member **205.** Member is a broad term and is to be given its ordinary and customary meaning to a person of ordinary skill in the art (i.e., it is not to be limited to a special or customized meaning) and includes, without limitation, integral portions, integral components, and distinct components. Thus, although FIG. **2A** illustrates an embodiment made of two distinct components, it will be appreciated that in other embodiments (such as described in below), the first elongate member **203** and second elongate member **205** can also represent regions in a tube formed from a single material. Thus, the first elongate member **203** can represent a hollow portion of a tube, while the second elongate member **205** represents a structural supporting or reinforcement portion of the tube which adds structural support to the hollow portion. The hollow portion and the structural supporting portion can have a spiral configuration, as described herein.

The composite tube **201** may be used to form the inspiratory tube **103** and/or the expiratory tube **117** in a breathing circuit, as described above, a coaxial tube as described below, or any other tubes as described elsewhere in this disclosure. In certain embodiments, the composite tube **201** is at least an inspiratory tube **103.**

The following describes components and properties of example composite tubes **201** in greater detail. Sub-headings are used, such as "first elongate member" and "second elongate member." These sub-headings are not, and should not be construed as, limiting. For example, aspects of one or more embodiments described under the first-elongate-member subheading can also apply to one or more embodiments described under the second-elongate-member subheading, and the reverse is also true.

### First Elongate Member

In FIG. **2A****,** the first elongate member **203** comprises a hollow body spirally wound to form, at least in part, an elongate tube having a longitudinal axis **LA-LA** and a lumen **207** (tube bore) extending along the longitudinal axis **LA-LA.** The first elongate member **203** has an inner portion **211** near the lumen **207.** In certain embodiments, a surface of the inner portion **211** forms the lumen **207.** The first elongate member **203** also has an outer portion **219** opposite the inner portion and facing away from the lumen **207** in the radial direction. As discussed in greater detail below, the first elongate member **203** can form in longitudinal cross-section a plurality of bubbles. In certain embodiments, the bubbles have a cross-sectional profile resembling the letter "D." The bubbles can be arced at the outward-facing surface. The bubbles can be flatter at the surface at the lumen **207.** In at least one embodiment, the first elongate member **203** is a tube.

Preferably, the first elongate member **203** is flexible. Flexible refers to the ability to bend. Furthermore, the first elongate member **203** is preferably transparent or, at least, semi-transparent or semi-opaque. A degree of optical transparency allows a caregiver or user to inspect the lumen **207** for blockage or contaminants or to confirm the presence of moisture.

A variety of plastics, including medical grade plastics, are suitable for the body of the first elongate member **203.** Examples of suitable materials include Polyolefin elastomers, Polyether block amides, Thermoplastic co-polyester elastomers, EPDM-Polypropylene mixtures, and Thermoplastic polyurethanes. In certain embodiments, the material is selected such that the material density of the resulting first elongate member **203** is less than or equal to 1 g/cm³ (or about 1 g/cm³).

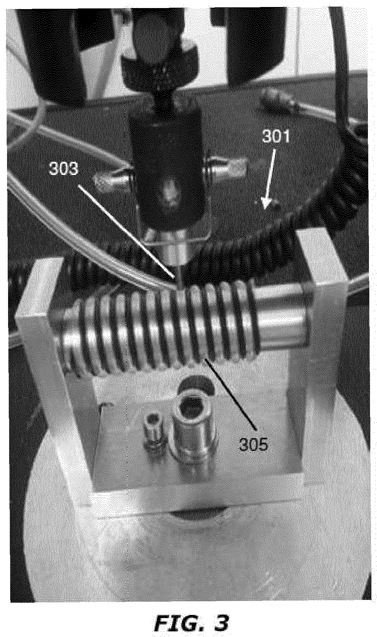

The first elongate member **203** material is preferably soft. Softness reflects the amount the material "gives" or compresses upon application of a force. A soft material gives or compresses more than a firm material. Bubble deflection can be used to qu
antify the softness of the first elongate member **203** material. Bubble deflection is distance that the outer portion **219** of the first elongate member **203** vertically deflects (that is, displaces radially inward in the direction of the lumen **207)** upon application of a force. Bubble deflection can be tested, for example, using a bubble deflection jig, such as the jig **301** shown in the photograph of FIG. **3****.**

In one softness test, four samples of composite tubes having the properties shown in TABLE **1** (hereinafter "Type 1") and four samples of composite tubes having the properties shown in TABLE **2** (hereinafter "Type 2") were each tested on the jig **301** of FIG. **3****.**

**Table 1**

| Feature | Dimension (mm) | Range (±) |
|---|---|---|
| Lumen diameter | 17.2 | 5.0 |
| Pitch | 5.1 | 3.0 |
| Bubble width | 4.0 | 2.0 |
| Bead width | 2.3 | +3.0/-2.0 |
| Bubble height | 2.7 | +5.0/-2.0 |
| Bead height | 1.6 | 1.5 |
| Bubble thickness on top, farthest from lumen (outer wall thickness) | 0.24 | +0.20/-0.10 |
| Bubble thickness adjacent lumen (inner wall thickness) | 0.10 | +0.20/-0.05 |
| Outer diameter of tube | 22.5 | 3.0 |

**Table 2**

| Feature | Dimension (mm) | Range(±) |
|---|---|---|
| Lumen diameter | 18.25 | 0.25 |
| Pitch | 8.2 | 0.15 |
| Bubble width | 7.0 | 1 |
| Bead width | 2.30 | 0.15 |
| Bubble height | 4.0 | 0.05 |
| Bead height | 1.95 | 0.15 |
| Bubble thickness on top, farthest from lumen (outer wall thickness) | 0.42 | 0.04 |
| Bubble thickness adjacent lumen (inner wall thickness) | 0.22 | 0.04 |
| Outer diameter of tube | 26 | 0.5 |

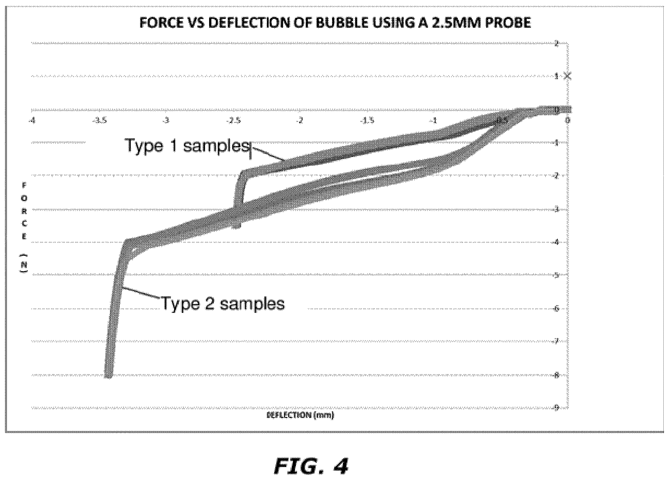

A probe **303** with a 2.5-mm diameter applied a force to each sample **305** and bubble deflection was measured. The resulting curves are plotted in FIG. **4****.** Until their respective outer portion **219** contacted the inner portion **211,** Type 1 samples generally required less force to achieve a similar bubble deflection as Type 2 samples. In certain embodiments, until the outer portion **219** contacts the inner wall **211,** the bubble deflection can satisfy the equation: *D* > 0.5×*F*_{2.5}, where *D* represents the bubble deflection in millimeters and *F*_{2.5} represents the force in Newtons applied by a 2.5-mm probe. For example, the first elongate member **203** can deflect more than 1 mm when a force of 1 N is applied with a 2.5-mm probe **303,** until the outer portion **219** contacts the inner portion **211.**

It should be appreciated that, although the configuration in TABLE **1** may be preferred in certain embodiments, other configurations and variations, may be used in other embodiments as may be desired.

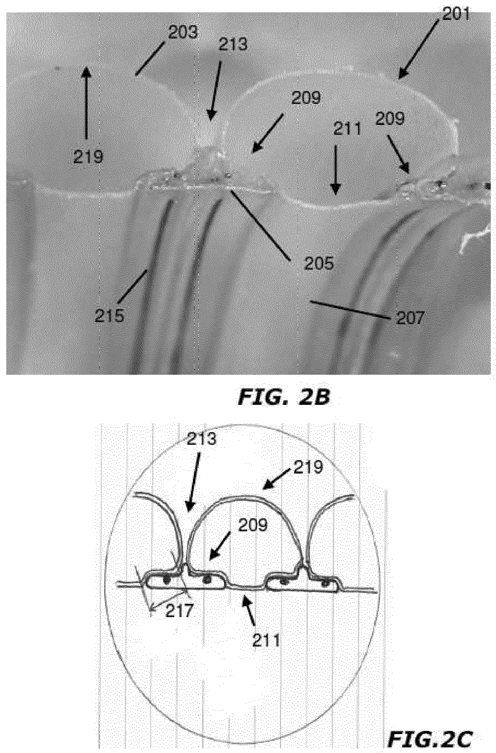

FIG. **2B** shows a longitudinal cross-section of a top portion of the example composite tube **201** of FIG. **2A****.** FIG. **2B** has the same orientation as FIG. **2A****.** This example further illustrates the hollow-body shape of the first elongate member **203.** As seen in this example, the first elongate member **203** forms in longitudinal cross-section a plurality of hollow bubbles. Thus, in this description, the term "bubble" refers to cross-sectional shape of a wind or turn of the first elongate member **203.** Portions **209** of the first elongate member **203** overlap adjacent wraps of the second elongate member **205.** An inner portion **211** of the first elongate member **203** forms the wall of the lumen **207.**

The hollow body structure of the first elongate member **203** contributes to the sound damping properties to the composite tube **201.** In at least one embodiment, the outer diameter of the first elongate member **203** is larger than the outer diameter of the second elongate member **205.** The bubble-shaped structure forms a cushion. Thus, the fluid (gas or liquid) filled bubble-shaped first elongate member **203** can muffle the noise made when the composite tube **201** is dragged over an object, such as the edge of a desk or bedside table. In this way, the composite tube **201** can be quieter compared with one-piece solid-body corrugated tubes,
The hollow body structure of the first elongate member **203** also contributes to the insulating properties to the composite tube **201.** An insulating composite tube **201** is desirable because, as explained above, it prevents heat loss. This can allow the composite tube **201** to deliver gas from a heater-humidifier to a patient while maintaining the gas's conditioned state with minimal energy consumption.

It was discovered that having a gap **213** between adjacent turns of the first elongate member **203,** that is, between adjacent bubbles, unexpectedly improved the overall insulating properties of the composite tube **201.** Thus, in certain embodiments, adjacent bubbles are separated by a gap **213.** Furthermore, certain embodiments include the realization that providing a gap **213** between adjacent bubbles increases the heat transfer resistivity (the R value) and, accordingly, decreases the heat transfer conductivity of the composite tube **201.** This gap configuration was also found to improve the flexibility of the composite tube **201** by permitting shorter-radius bends. A triangular second elongate member **205** or a T-shaped second elongate member **205,** as shown in FIG. **2B****,** can help maintain a gap **213** between adjacent bubbles. Nevertheless, in certain embodiments, adjacent bubbles are touching. For example, adjacent bubbles can be bonded together.

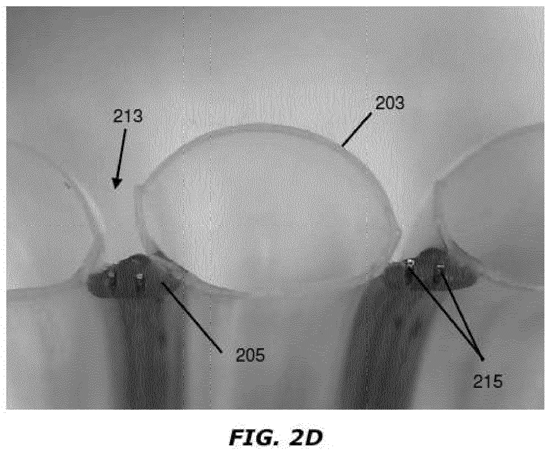

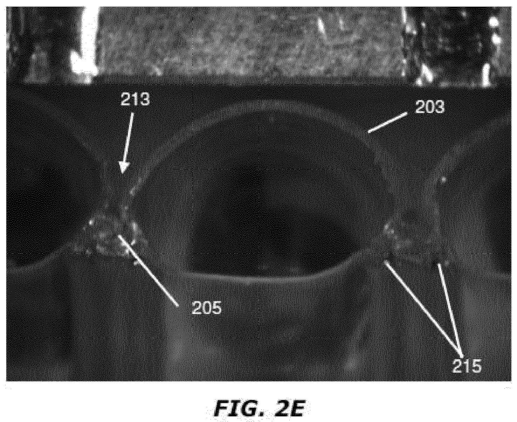

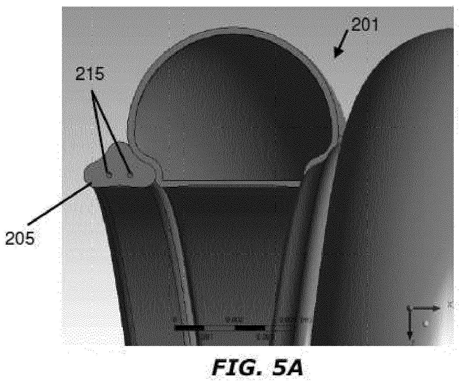

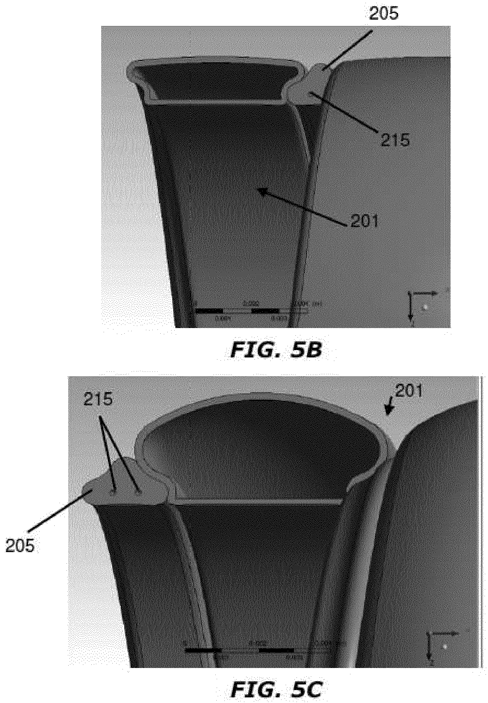

FIG. **2C** shows a longitudinal cross-section of the bubbles in FIG. **2B****.** As shown, the portions **209** of the first elongate member **203** overlapping adjacent wraps of the second elongate member **205** are characterized by a degree of bond region **217.** A larger bond region improves the tube's resistance to delamination at the interface of the first and second elongate members. Additionally or alternatively, the shape of the bead and/or the bubble can be adapted to increase the bond region **217.** For example, FIG. **2D** shows a relatively small bonding area on the left-hand side. FIG. **5B** also demonstrates a smaller bonding region. In contrast, FIG. **2E** has a much larger bonding region than that shown in FIG. **2D****,** because of the size and shape of the bead. FIGS. **5A** and **5C** also illustrate a larger bonding region. Each of these figures is discussed in more detail below. It should be appreciated that, although the configurations in FIGS. **2E****, 3A,** and **5C** may be preferred in certain embodiments, other configurations, including those of FIGS. **2D****,** **5B****,** and other variations, may be used in other embodiments as may be desired.

FIG. **2D** shows a longitudinal cross-section of a top portion of another composite tube. FIG. **2D** has the same orientation as FIG. **2B****.** This example further illustrates the hollow-body shape of the first elongate member **203** and demonstrates how the first elongate member **203** forms in longitudinal cross-section a plurality of hollow bubbles. In this example, the bubbles are completely separated from each other by a gap **213.** A generally triangular second elongate member **205** supports the first elongate member **203.**

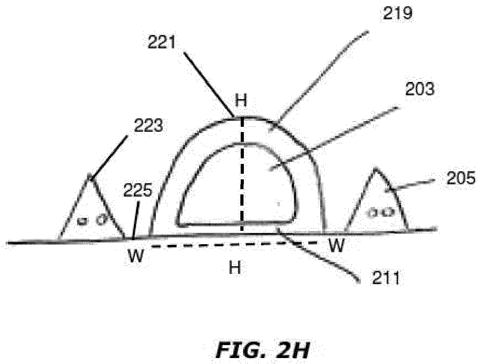

FIG. **2H** shows a longitudinal cross-section of a top portion of another composite tube. FIG. **2H** has the same orientation as FIG. **2B****.**

In the example of FIG. **2H****,** the cross-sectional thickness of the inner portion **211** of the first elongate member **203** forming the wall of the lumen is less than the thickness of the outer portion **219.** Because the first elongate member **203** has a D-shaped bubble profile, the outer-facing portion of the first elongate member **203** has material slack between adjacent winds of the second elongate member, which facilitates movement and stretching as the composite tube **201** is bent into a ∩-shape. Because the configuration of FIG. **2H** results in a thinner bubble near the lumen **207,** such a configuration allows the inner portion **211** to compress or "bunch" more readily when the composite tube **201** is bent into a ∩-shape. Thus, certain embodiments include the realization that a configuration in which the cross-sectional thickness of the inner portion **211** is less than the cross-sectional thickness of the outer portion **219** can improve the flexibility of the composite tube **201** by permitting shorter radius bends. In addition, certain embodiments include the realization that overall tube flexibility can be improved by providing a first elongate member **203** with a variable cross-sectional wall thickness. Desirably, the thickness of the inner portion **211** is less than the thickness of the outer portion **219.**

In at least one embodiment, the thickness of the inner portion **211** is at least 20% (or about 20%) less than the thickness of the outer portion **219.** For example, in certain embodiments, the thickness of the inner portion **211** is at least 30% (or about 30%), at least 40% (or about 40%), at least 50% (or about 50%), or at least 60% (or about 60%) less than the thickness of the outer portion **219.** In certain embodiments, the thickness of the inner portion **211** is 27% (or about 27%) less than the thickness of the outer portion **219.** In certain embodiments, the thickness of the inner portion **211** is 32% (or about 32%) less than the thickness of the outer portion **219.** In certain embodiments, the thickness of the inner portion **211** is 58% (or about 58%) less than the thickness of the outer portion **219.** In certain embodiments, the thickness of the inner portion **211** is 64% (or about 64%) less than the thickness of the outer portion **219.**

The thickness of the outer portion **219** can be in the range of 0.14 mm (or about 0.14 mm) and 0.44 mm (or about 0.44 mm), such as 0.22 mm (or about 0.22 mm) or 0.24 mm (or about 0.24 mm). The thickness of the inner portion **211** can be in the range of 0.05 mm (or about 0.05 mm) and 0.30 mm (or about 0.30 mm), and preferably 0.10 mm (or about 0.10 mm) or 0.16 mm (or about 0.16 mm).

Referring again to FIG. **2H****,** the height (designated as **H-H)** of a single longitudinal cross-sectional bubble of the first elongate member **203** can be greater than the width (designated as **W-W)** of a single longitudinal cross-sectional bubble of the first elongate member **203.** Because a greater height increases the amount of material slack in the outer wall of the bubble of the first elongate member **203,** such configuration can improve the flexibility of the composite tube **201** by permitting shorter radius bends. Accordingly, certain embodiments include the realization that overall tube flexibility can be improved by providing a first elongate member **203** with a longitudinal cross-sectional height that is greater than the longitudinal cross-sectional width. It should be appreciated that, although this example configuration may be preferred in certain embodiments, other configurations and variations, may be used in other embodiments as may be desired. For example, the height of a longitudinal cross-sectional bubble of the first elongate member **203** can be less than its width.

In at least one embodiment, the bubble height **(H-H)** can be in the range of 1.2 mm (or about 1.2 mm) and 8.2 mm (or about 8.2 mm), such as 1.2 mm (or about 1.2 mm), 1.7 mm (or about 1.7 mm), 1.8 mm (or about 1.8 mm), 2.7 mm (or about 2.7 mm), 2.8 mm (or about 2.8 mm), 3 mm (or about 3 mm), 3.2 mm (or about 3.2 mm), 3.5 mm (or about 3.5 mm), 3.8 mm (or about 3.8 mm), 4 mm (or about 4 mm), 4.5 mm (or about 4.5 mm), 7.7 mm (or about 7.7 mm), or 8.2 mm (or about 8.2 mm). In at least one embodiment, the bubble width **(W-W)** can be in the range of 1.7 mm (or about 1.7 mm) and 8 mm (or about 8 mm), such as 1.7 mm (or about 1.7 mm), 3.2 mm (or about 3.2 mm), 3.5 mm (or about 3.5 mm), 4.0 mm (or about 4.0 mm), 4.2 mm (or about 4.2 mm), 5.2 mm (or about 5.2 mm), 5.5 mm (or about 5.5 mm), 6 mm (or about 6 mm), 7 mm (or about 7 mm), 7.5 mm (or about 7.5 mm), or 8 mm (or about 8 mm).

The relationship between bubble height **(H-H)** and bubble width **(W-W)** can be expressed as a ratio. A ratio of bubble height **(H-H)** to bubble width **(W-W)** equal to 0 is least flexible. Flexibility increases as the ratio increases. In at least one embodiment, the ratio of bubble height **(H-H)** to bubble width **(W-W)** can be in the range of 0.15 (or about 0.15) and 1.5 mm (or about 1.5), such as 0.16 (or about 0.16), 0.34 (or about 3.4), 0.50 (or about 0.50), 0.56 (or about 0.56), 0.57 (or about 0.57), 0.58 (or about 0.58), 0.67 (or about 0.67), 0.68 (or about 0.68), 0.73 (or about 0.73), 0.85 (or about 0.85), 1.1 (or about 1.1). and 1.3 (or about 1.3).

It can be desirable for the outer profile of the corrugated tube to be relatively smooth. Relative smoothness, as used in this description, relates to the ridges between the first elongate member **203** and second elongate member **205** along the length of the composite tube **201.** A relatively smoother corrugated tube has flatter, more closely spaced, or otherwise less pronounced ridges. A relatively smoother profile can advantageously reduce noise when the corrugated tube is dragged across an object, such as a desk or table edge.

An example parameter for quantifying relative smoothness is the vertical difference between an outer radial apex **221** of first elongate member **203** and an outer radial apex **223** of the second elongate member **205** of a composite tube **201** (as shown, for example, in FIG. **2H****).** As the distance between the outer radial apex **221** and the outer radial apex **223** decreases, the composite tube **201** feels relatively smoother. In at least one embodiment, the vertical distance is in the range of 1 mm (or about 1 mm) and 4.6 mm (or about 4.6 mm), such as 1.0 mm (or about 1.0 mm), 1.1 mm (or about 1.1 mm), 1.3 mm (or about 1.3 mm), 1.4 mm (or about 1.4 mm), 1.6 mm (or about 1.6 mm), 1.9 mm (or about 1.9 mm), 2.0 mm (or about 2.0 mm), 2.3 mm (or about 2.3 mm), 2.4 mm (or about 2.4 mm), 3.0 mm (or about 3.0 mm), 3.3 mm (or about 3.3 mm), or 4.6 mm (or about 4.6 mm). It is also possible to quantify relative smoothness as the vertical distance between an outer radial apex **221** of the first elongate member **203** and an outer radial nadir **225** of the second elongate member **205** of the composite tube **201.** For example, the vertical distance can be 1.5 mm (or about 1.5 mm).

Another example parameter for quantifying relative smoothness is the ratio of the vertical difference between a radial apex **221** of first elongate member **203** and a radial apex **223** (or a radial nadir **225)** of the second elongate member **205** of a composite tube **201** to the maximum outer diameter of the composite tube **201** (that is, from outer radial apex **221** to outer radial apex **221** on the opposite side of the tube **201).** As the maximum outer diameter increases, the vertical difference between the outer radial apex **221** and the outer radial apex **223** or nadir **225** has less effect on relative smoothness. In at least one embodiment, the ratio is in the range of 0.04 to 0.18, such as 0.04, 0.05, 0.07, 0.08, 0.09, 0.10, 0.11, 0.12, 0.16, 0.17, or 0.18 or thereabout.

As another example, the distance between corresponding points from one turn to the next (that is, the pitch) can be selected to quantify relative smoothness. In certain embodiments, the pitch can be in the range of 2.1 mm (or about 2.1 mm) and 9.5 mm (or about 9.5 mm), such as 2.1 mm (or about 2.1 mm), 3.8 mm (or about 3.8 mm), 4.8 mm (or about 4.8 mm), 5.1 mm (or about 5.1 mm), 5.5 mm (or about 5.5 mm), 5.8 mm (or about 5.8 mm), 6.4 mm (or about 6.4 mm), 7.5 mm (or about 7.5 mm), 8.1 mm (or about 8.1 mm), or 9.5 mm (or about 9.5 mm).

The ratio of the pitch of the composite tube **201** to the vertical difference between a radial apex **221** of the first elongate member **203** and a radial apex **223** of the second elongate member **205** of the composite tube **201** to can be selected to quantify relative smoothness. In certain embodiments, the ratio is in the range of 1.3 (or about 1.3) and 4.8 (or about 4.8), such as 1.31 (or about 1.31), 1.76 (or about 1.76), 2.39 (or about 2.39), 2.42 (or about 2.42), 2.53 (or about 2.53), 2.71 (or about 2.71), 2.75 (or about 2.75), 3.26 (or about 3.26), 3.75 (or about 3.75), 4.13 (or about 4.13), 4.64 (or about 4.64), or 4.75 (or about 4.75).

The ratio of pitch to maximum outer diameter can also be selected to improve relative smoothness. In certain embodiments, the ratio of pitch to the outer diameter of the tube can be in the range of 0.10 (or about 0.10) and 0.35 (or about 0.32), such as 0.11 (or about 0.11), 0.23 (or about 0.23), 0.28 (or about 0.28), 0.29 (or about 0.29), 0.30 (or about 0.30), 0.31 (or about 0.31), or 0.32 (or about 0.32).

As discussed above, the hollow portion of the first elongate member 203 can be filled with a fluid, that is, a liquid or gas. The first elongate member 203 can be substantially sealed so as to prevent the quantity of fluid escaping. The first elongate member 203 can also be open at one or both ends to allow a continuous flow of liquid or gas.

The gas can be air, which is desirable because of its low thermal conductivity (2.62x10⁻² W/m·K at 300K). A gas that is more viscous than air may also be used advantageously, as a higher viscosity reduces heat transfer under conditions of natural convection. Thus, gases such as argon (17.72x10⁻³ W/m K at 300K), krypton (9.43x10⁻³ W/m·K at 300K), and xenon (5.65x10⁻³ W/ m·K at 300K) can increase insulating performance. Each of these gases is non-toxic, chemically inert, fire-inhibiting, and commercially available. The hollow portion of the first elongated member **203** can be sealed at both ends of the tube, causing the gas within to be substantially stagnant. Alternatively, the hollow portion can be a secondary pneumatic connection, such as a pressure sample line for conveying pressure feedback from the patient-end of the tube to a controller.

Examples of liquids can include water or other biocompatible liquids with a high thermal capacity. For instance, nanofluids can be used. An example nanofluid with suitable thermal capacity comprises water and nanoparticles of substances such as aluminum.

In use, the fluid in the hollow portion of the first elongate member **203** can be configured to be used to measure one or more properties of the tube **201,** the first elongate member **203,** the second elongate member **205,** and/or the gas in the tube **201** lumen **207.** In at least one embodiment, the pressure of gas passing along the tube lumen ("lumen gas") can be measured. A reference measurement of the pressure of the fluid in the hollow portion of the first elongate member **203** ("hollow fluid") is made before the lumen gas begins to circulate. As the lumen gas begins to pass through the tube **201,** the pressure of the lumen gas will tend to cause a proportional rise in the pressure of the hollow fluid within the first elongate member **203.** By comparing a measurement taken in use with the reference measurement, the pressure of the lumen gas within the tube **201** can be determined. In another embodiment, a hollow fluid is chosen that changes one or more properties based on the operational heat range of the lumen gas within the tube **201.** In this manner, by measuring the property of the hollow fluid, the temperature of the lumen gas can be determined. For example, a hollow fluid which expands with temperature can be used. In use, the temperature of the hollow fluid will tend towards the temperature of the lumen gas flow. By then measuring the pressure of the hollow fluid, the temperature of the lumen gas can be determined. This may have particular benefit when the temperature of the lumen gas flow is difficult or undesirable to measure directly.

In at least one embodiment, the extrudate used to form the first elongate member **203** further comprises a mineral filler. The extrusion process is described in greater detail below. Talc or hydrous magnesium silicate is suitable mineral filler. In addition to talc, other suitable mineral fillers include calcium carbonate, calcium magnesium carbonate such as dolomite, barium sulfate, wollastonite, kaolin, and mica, each of which can be added alone or in combination. Suitable mineral fillers can also have particle sizes less than 10 µm (or about 10 mm), or less than 2.5 *µ*m (or about 2.5 mm).

It was discovered that the addition of mineral filler to the plastic extrudate reduces the stickiness of the resultant first elongate member **203.** Stickiness refers to the tactile gumminess or clinginess of the first elongate member **203** material. A stickier material feels gummier than a less sticky material. A stickier material can also tend to cling to more unwanted matter, such as dirt or hair, than a less sticky material. The addition of mineral filler was discovered to reduce the noise the tube makes when it is moved, flexed, and so forth by reducing the extent to which adjacent bubbles stick (and unstick) to each other when bunched (and unbunched) around the vicinity of a bend.

It was also discovered that the addition of mineral filler to the extrudate can further reduce the noise made when the first elongate member **203** is dragged over an object, such as the edge of a desk or bedside table. The mineral filler may help reflect sound within the surrounding polymer so that the sound does not pass straight through. The improved sound reflection also may give the polymer phase more opportunity to absorb the sound energy, the mineral filler thereby providing intrinsic sound damping. The mineral filler may also reduce the hardness of the plastic extrudate and thereby improve sound damping properties.

In certain embodiments, the mineral filler is in the range of 1.5 to 10 (or about 1.5 to about 10) weight percent of the total extrudate. In certain embodiments, the mineral filler is in the range of 1.5 to 5 (or about 1.5 to about 5) weight percent of the total extrudate. In certain embodiments, the mineral filler is in the range of 10 (or about 10) weight percent or less of the total extrudate. In certain embodiments, the mineral filler is in the range of 5 (or about 5) weight percent or less of the total extrudate. In certain embodiments, the mineral filler is in the range of 1.5 (or about 1.5) weight percent or more of the total extrudate.

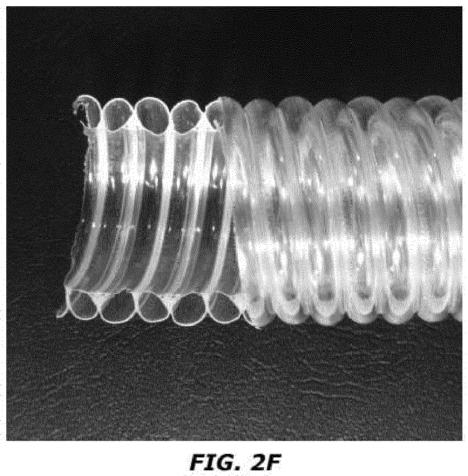

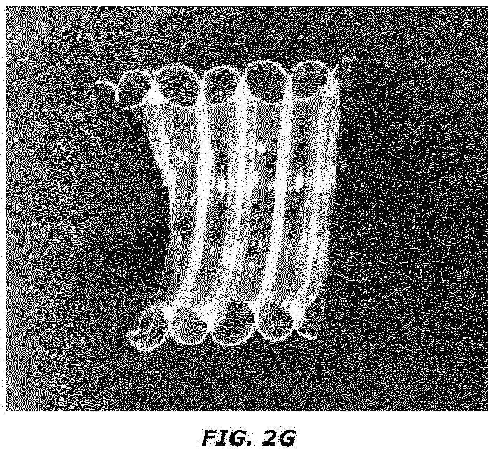

In FIG. **2F****,** the first elongate member **203** forms in longitudinal cross-section a plurality of hollow bubbles. In this example, there are a plurality of bubbles, and more specifically, two adjacent wraps of the first elongate member **203,** between wraps of the second elongate member **205.** This configuration is shown in greater detail in FIG. **2G****.** As described and shown elsewhere in this disclosure, certain configurations can implement greater than two, for example, three, wraps of the first elongate member **203** between wraps of the second elongate member **205.**

Embodiments comprising a plurality of adjacent wraps of the first elongate member **203** between wraps of the second elongate member **205** can be advantageous because of improvements in overall tube flexibility. As described below, the substantially solid second elongate member **205** is generally less flexible than the hollow first elongate member **203.** Accordingly, certain embodiments include the realization that overall tube flexibility can be improved by increasing the number of bubbles of first elongate member **203** between wraps of the second elongate member **205.**

Another advantage of embodiments comprising a plurality of adjacent wraps of the first elongate member **203** between wraps of the second elongate member **205** is improved recovery from crushing. It was observed that, after crushing, samples having multiple bubbles between wraps of the first elongate member **203** recovered their shape more quickly than samples having a single bubble between wraps of the first elongate member **203.**

Yet another advantage of embodiments comprising a plurality of adjacent wraps of the first elongate member **203** between wraps of the second elongate member **205** is improved resistance to crushing. Crush resistance is a mechanical property that plays an important role in the resilience of the tube while in service. The hospital environment can be harsh, as the tube can be subjected to crushing by a patient's arm or leg, bed frames, and other equipment. Example crush resistance properties are discussed in greater detail below.

Yet another advantage to the multiple-bubble configuration is that the configuration imparts the ability to hold or transport additional fluids. As explained above, the hollow portion of the first elongate member **203** can be filled with a gas. The multiple discrete bubbles or hollow portions can be filled with multiple discrete gases. For example, one hollow portion can hold or transport a first gas and a second hollow portion can be used as a secondary pneumatic connection, such as a pressure sample line for conveying pressure feedback from the patient-end of the tube to a controller. As another example, multiple discrete bubbles or hollow portions can be filled with a combination of liquids, or a combination of liquids and gases. A first bubble can hold or transport a gas, and a second bubble can hold or transport a liquid, for instance. Suitable liquids and gases are described above.

It should be appreciated that, although the configurations in FIGS. **2F** and **2G** may be preferred in certain embodiments, other configurations, may be utilized in other embodiments as may be desired.

### Second Elongate Member

Referring again to FIGS. **2A** and **2B****,** the second elongate member **205** is also spirally wound and joined to the first elongate member **203** between turns of the first elongate member **203.** The second elongate member **205** can form at least a portion of the lumen **207** of the elongate tube. The second elongate member **205** acts as structural support for the first elongate member **203.**

CPAP machines ordinarily weigh in the range of 2 and 4 kg (or about 2 and 4 kg). Thus, the break strength of the composite tube **201** (the horizontal tensile load or force required to cause separation of the first elongate member **203** and the second elongate member **205)** desirably is high enough to prevent separation if a user attempts to use the composite tube **201** to lift a CPAP machine connected to the composite tube **201.** Thus, the break strength is preferably greater than 20 N (or about 20 N) and, more preferably, greater than 30 N (or about 30 N). In certain embodiments, the break strength is in the range of 75 and 80 N (or about 75 and 80 N). The yield strength (the maximum stress that can be developed without causing plastic deformation) can be in the range of 55 and 65 N (or about 55 and 65 N). In certain embodiments, the composite tube 201 will not stretch (horizontally deflect) more than 0.5 mm (or about 0.5 mm) when a lateral force of 2 N is applied.

In at least one embodiment, the second elongate member **205** is wider at the base (proximal the lumen **207)** and narrower at the top. For example, the second elongate member can be generally triangular in shape, generally T-shaped, or generally Y-shaped. However, any shape that meets the contours of the corresponding first elongate member **203** is suitable.

Preferably, the second elongate member **205** is flexible, to facilitate bending of the tube. Desirably, the second elongate member **205** is less flexible than the first elongate member **203.** This improves the ability of the second elongate member **205** to structurally support the first elongate member **203.** For example, the modulus of the second elongate member **205** is preferably 30 - 50MPa (or about 30 - 50 MPa). The modulus of the first elongate member **203** is less than the modulus of the second elongate member **205.** The second elongate member **205** can be solid or mostly solid.

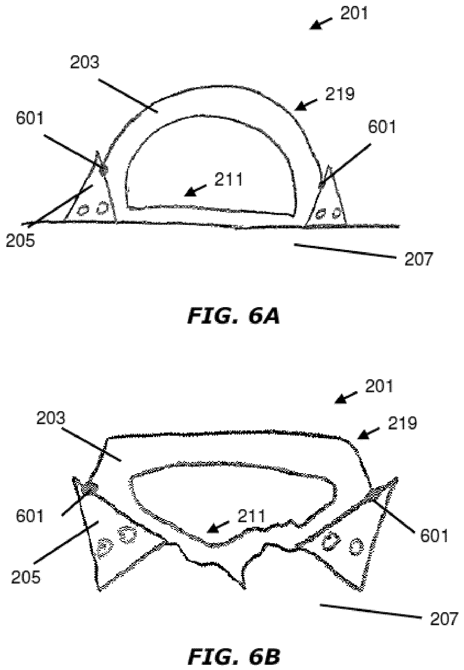

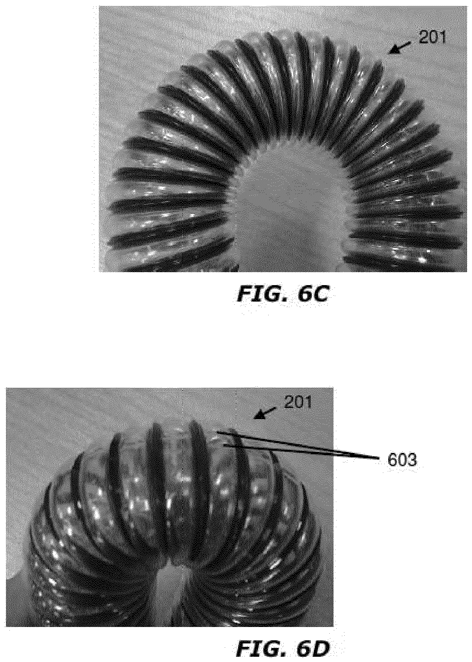

FIG. **6A** shows a longitudinal cross section of a composite tube **201** in a neutral position. FIG. **6A** focuses on one wind or bubble of the first elongate member **203** and two winds of the second elongate member **205.** The first elongate member **203** and second elongate member **205** have a radially-outermost connection point **601.** In this example, the inner portion **211** of the first elongate member **203** is thinner than the outer portion **219** of the first elongate member **203.** Also in this example, the second elongate member **205** has a triangular cross section. The lumen **207** is situated under the base of the first elongate member **203** and second elongate member **205.** FIG. **6B** shows the composite tube **201** of FIG. **6A** in a bent position, in which the composite tube **201** has been bent to a ∩-shape (as shown in FIG. **6C****).** FIG. **6B** again focuses on one wind or bubble of the first elongate member **203** and two winds of the second elongate member **205.** More specifically, FIG. **6B** focuses on the wind or bubble of the first elongate member **203** at the top of the ∩-shape, that is, at the location of maximum bend. The radius of curvature of the composite tube **201** is constrained by the length of the section of the outer portion **219** between adjacent outermost connection points **601.** If the composite tube **201** is bent beyond the minimum radius of curvature, the outer wall forms dimples **605,** as shown in FIG. **6D****.**

A variety of polymers and plastics, including medical grade plastics, are suitable for the body of the second elongate member **205.** Examples of suitable materials include Polyolefin elastomers, Polyether block amides, Thermoplastic co-polyester elastomers, EPDM-Polypropylene mixtures and Thermoplastic polyurethanes. In certain embodiments, the first elongate member **203** and the second elongate member **205** may be made from the same material. The second elongate member **205** may also be made of a different color material from the first elongate member **203,** and may be transparent, translucent or opaque. For example, in one embodiment the first elongate member **203** may be made from a clear plastic, and the second elongate member **205** may be made from an opaque blue, black, or other color plastic.

This spirally-wound structure comprising a flexible, hollow body and an integral support can provide crush resistance, while leaving the tube wall flexible enough to permit short-radius bends without kinking, occluding or collapsing. Preferably, the tube can be bent around a 25 mm diameter metal cylinder without kinking, occluding, or collapsing, as defined in the test for increase in flow resistance with bending according to ISO 5367:2000(E).

This structure also can provide a smooth lumen **207** surface, which helps keep the tube free from deposits and improves gas flow. The hollow body has been found to improve the insulating properties of a tube, while allowing the tube to remain light weight.

In some embodiments, the second elongate member **205** can be made of a material that wicks water. For example, an absorbent sponge-like material can be used. In such embodiments, the second elongate member **205** can be connected to a water source, such as a water bag. In use, water would be conveyed along at least a portion of the length of the second elongate member **205** (preferably, substantially the whole length). As gas passes along the second elongate member **205,** water vapor will tend to be picked up by the gases in the lumen **207,** thereby humidifying the gas flow.

In some embodiments, the one or more heating filaments **215** embedded in the second elongate member **205,** as shown in FIG. **2B****,** can be controlled to alter the rate of evaporation and thereby alter the level of humidification provided to the gas flow. Although FIG. **2B** specifically shows heating filaments **215,** it should be understood that the second elongate member **205** can encapsulate or house other conductive material(s), such as one or more filaments, and specifically sensors (not shown). Such conductive materials can be disposed in the second elongate member **205** for heating or sensing the gas flow. Heating filaments **215** can minimize the cold surfaces onto which condensate from moisture-laden air can form. Heating filaments **215** can also be used to alter the temperature profile of gases in the lumen **207** of composite tube **201.**

In the example of FIG. **2B****,** two heating filaments **215** are encapsulated in the second elongate member **205,** one on either side of the vertical portion of the "T." The heating filaments **215** comprise conductive material, such as alloys of Aluminum (Al) and/or Copper (Cu), or conductive polymer. Preferably, the material forming the second elongate member **205** is selected to be non-reactive with the metal in the heating filaments **215** when the heating filaments **215** reach their operating temperature. The filaments **215** may be spaced away from lumen **207** so that the filaments are not exposed to the lumen **207.** At one end of the composite tube, pairs of filaments can be formed into a connecting loop.

In at least one embodiment, a plurality of filaments are disposed in the second elongate member **205.** The filaments can be electrically connected together to share a common rail. For example, a first filament, such as a heating filament, can be disposed on a first side of the second elongate member **205.** A second filament, such as a sensing filament, can be disposed on a second side of the second elongate member **205.** A third filament, such as a ground filament, can be disposed between the first and second filaments. The first, second, and/or third filaments can be connected together at one end of the second elongate member **205.**

FIG. **2E** shows a longitudinal cross-section of a top portion of another composite tube. FIG. **2E** has the same orientation as FIG. **2B****.** In the example of FIG. **2E****,** the heating filaments **215** are spaced farther apart from each other than the filaments **215** in FIG. **2B****.** It was discovered that increasing the space between heating filaments can improve heating efficiency, and certain embodiments include this realization. Heating efficiency refers to the ratio of the amount of heat input to the tube to the amount of energy output or recoverable from the tube. Generally speaking, the greater the energy (or heat) that is dissipated to ambient atmosphere from the tube, the lower the heating efficiency. For improved heating performance, the heating filaments **215** can be equally (or about equally) spaced along the bore of the tube. Alternatively, the filaments **215** can be positioned at extremities of the second elongate member **205,** which may provide simpler manufacturing.

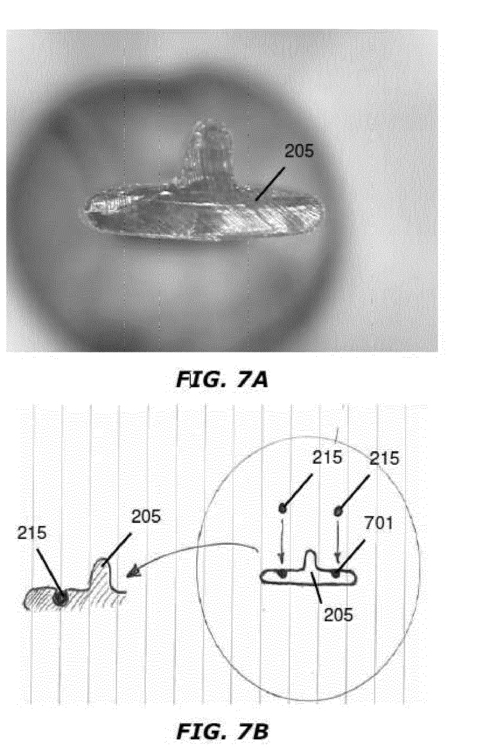

Reference is next made to FIGS. **7A** through **7G** which demonstrate example configurations for the second elongate member **205.** FIG. **7A** shows a cross-section of a second elongate member **205** having a shape similar to the T-shape shown in FIG. **2B****.** In this example embodiment, the second elongate member **205** does not have heating filaments. Other shapes for the second elongate member **205** may also be utilized, including variations of the T-shape as described below and triangular shapes.

FIG. **7B** shows another example second elongate member **205** having a T-shape cross-section. In this example, heating filaments **215** are embedded in cuts **701** in the second elongate member **205** on either side of the vertical portion of the "T." In some embodiments, the cuts **701** can be formed in the second elongate member **205** during extrusion. The cuts **701** can alternatively be formed in the second elongate member **205** after extrusion. For example, a cutting tool can form the cuts in the second elongate member **205.** Preferably, the cuts are formed by the heating filaments **215** as they are pressed or pulled (mechanically fixed) into the second elongate member **205** shortly after extrusion, while the second elongate member **205** is relatively soft. Alternatively, one or more heating filaments can be mounted (e.g., adhered, bonded, or partially embedded) on the base of the elongate member, such that the filament(s) are exposed to the tube lumen. In such embodiments, it can be desirable to contain the filament(s) in insulation to reduce the risk of fire when a flammable gas such as oxygen is passed through the tube lumen.

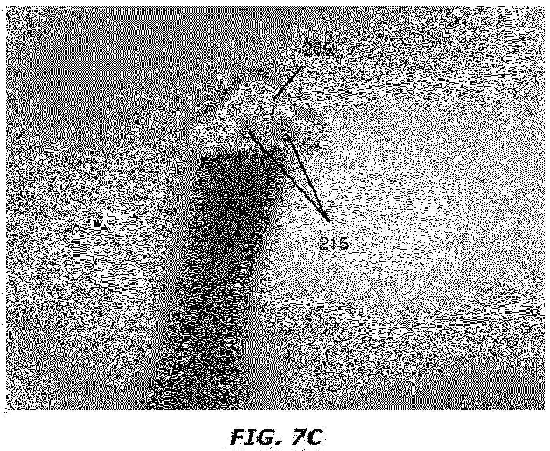

FIG. **7C** shows yet another example second elongate member **205** in cross-section. The second elongate member **205** has a generally triangular shape. In this example, heating filaments **215** are embedded on opposite sides of the triangle.

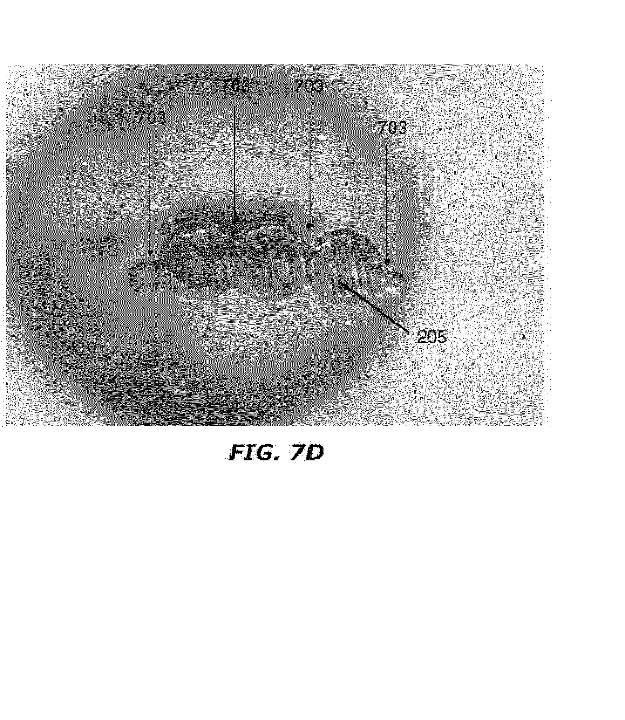

FIG. **7D** shows yet another example second elongate member **205** in cross-section. The second elongate member **205** comprises four grooves **703.** The grooves **703** are indentations or furrows in the cross-sectional profile. In some embodiments, the grooves **703** can facilitate the formation of cuts (not shown) for embedding filaments (not shown). In some embodiments, the grooves **703** facilitate the positioning of filaments (not shown), which are pressed or pulled into, and thereby embedded in, the second elongate member **205.** In this example, the four initiation grooves **703** facilitate placement of up to four filaments, for example, four heating filaments, four sensing filaments, two heating filaments and two sensing filaments, three heating filaments and one sensing filament, or one heating filament and three sensing filaments. In some embodiments, heating filaments can be located on the outside of the second elongate member **205.** Sensing filaments can be located on the inside.

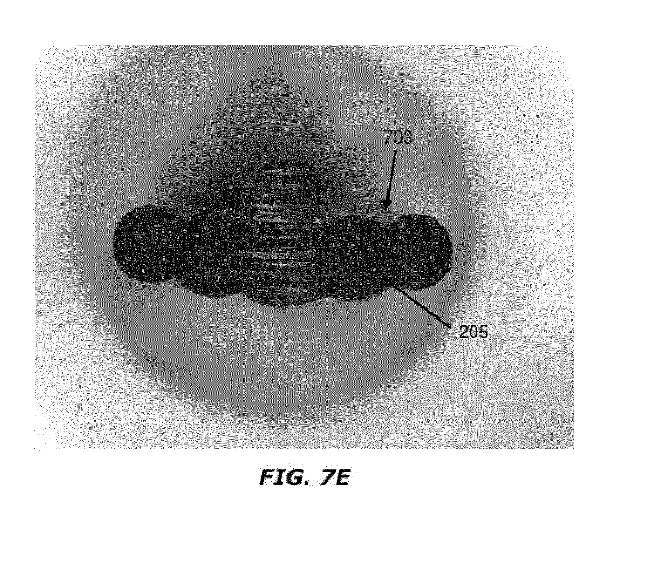

FIG. **7E** shows still another example second elongate member **205** in cross-section. The second elongate member **205** has a T-shape profile and a plurality of grooves **303** for placing heating filaments.

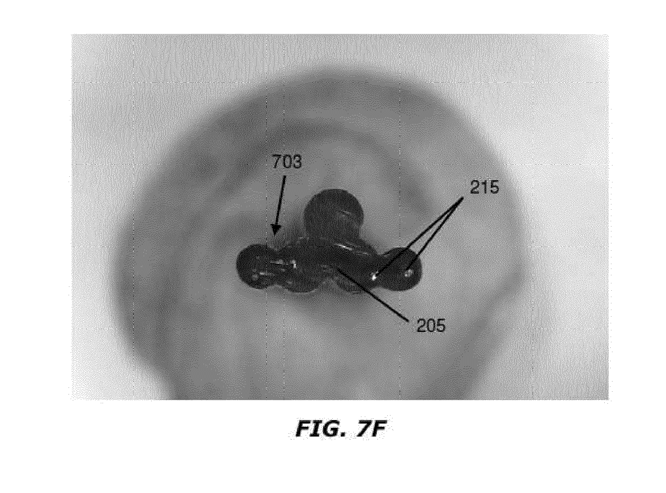

FIG. **7F** shows yet another example second elongate member **205** in cross-section. Four filaments **215** are encapsulated in the second elongate member **205,** two on either side of the vertical portion of the "T." As explained in more detail below, the filaments are encapsulated in the second elongate member **205** because the second elongate member **205** was extruded around the filaments. No cuts were formed to embed the heating filaments **215.** In this example, the second elongate member **205** also comprises a plurality of grooves **703.** Because the heating filaments **215** are encapsulated in the second elongate member **205,** the grooves **703** are not used to facilitate formation of cuts for embedding heating filaments. In this example, the grooves **703** can facilitate separation of the embedded heating filaments, which makes stripping of individual cores easier when, for example, terminating the heating filaments.

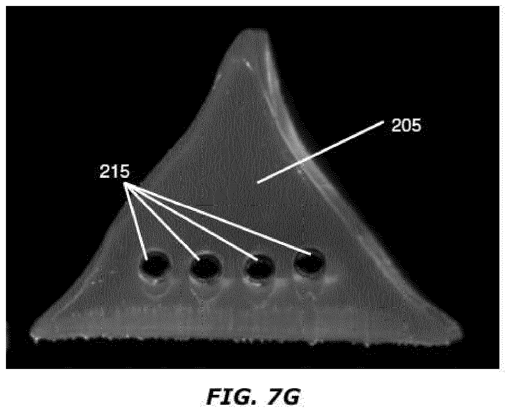

FIG. **7G** shows yet another example second elongate member **205** in cross-section. The second elongate member **205** has a generally triangular shape. In this example, the shape of the second elongate member **205** is similar to that of FIG. 7C, but four filaments **215** are encapsulated in the second elongate member **205,** all of which are central in the bottom third of the second elongate member **205** and disposed along a generally horizontal axis.

As explained above, it can be desirable to increase the distance between filaments to improve heating efficiency. In some embodiments, however, when heating filaments **215** are incorporated into the composite tube **201,** the filaments **215** can be positioned relatively central in the second elongate member **205.** A centralized position promotes robustness of the composite tubing for reuse, due in part to the position reducing the likelihood of the filament breaking upon repeating flexing of the composite tube **201.** Centralizing the filaments **215** can also reduce the risk of an ignition hazard because the filaments **215** are coated in layers of insulation and removed from the gas path.

As explained above, some of the examples illustrate suitable placements of filaments **215** in the second elongate member **205.** In the foregoing examples comprising more than one filament **215,** the filaments **215** are generally aligned along a horizontal axis. Alternative configurations are also suitable. For example, two filaments can be aligned along a vertical axis or along a diagonal axis. Four filaments can be aligned along a vertical axis or a diagonal axis. Four filaments can be aligned in a cross-shaped configuration, with one filament disposed at the top of the second elongate member, one filament disposed at the bottom of the second elongate member (near the tube lumen), and two filaments disposed on opposite arms of a "T," "Y," or triangle base.

### Dimensions

TABLES **3** and **4** show some example dimensions of medical tubes described herein, as well as some ranges for these dimensions. The dimensions refer to a transverse cross-section of a tube. In these tables, lumen diameter represents the inner diameter of a tube. Pitch represents the distance between two repeating points measured axially along the tube, namely, the distance between the tip of the vertical portions of adjacent "T"s of the second elongate member. Bubble width represents the width (maximum outer diameter) of a bubble. Bubble height represents the height of a bubble from the tube lumen. Bead height represents the maximum height of the second elongate member from the tube lumen (e.g., the height of the vertical portion of the "T"). Bead width represents the maximum width of the second elongate member (e.g., the width of the horizontal portion of the "T"). Bubble thickness represents the thickness of the bubble wall.

**Table 3**

| Feature | Infant | | Adult | |
|---|---|---|---|---|
| | Dimension (mm) | Range (+) | Dimension (mm) | Range (±) |
| Lumen diameter | 11 | 1 | 18 | 5 |
| Pitch | 4.8 | 1 | 7.5 | 2 |
| Bubble width | 4.2 | 1 | 7 | 1 |
| Bead width | 2.15 | 1 | 2.4 | 1 |
| Bubble height | 2.8 | 1 | 3.5 | 0.5 |
| Bead height | 0.9 | 0.5 | 1.5 | 0.5 |
| Bubble thickness | 0.4 | 0.35 | 0.2 | 0.15 |

**Table 4**

| Feature | Infant | | Adult | |
|---|---|---|---|---|
| | Dimension (mm) | Range (±) | Dimension (mm) | Range (±) |
| Lumen diameter | 11 | 1 | 18 | 5 |
| Pitch | 4.8 | 1 | 7.5 | 2 |
| Bubble width | 4.2 | 1 | 7 | 1 |
| Bead width | 2.15 | 1 | 3.4 | 1 |
| Bubble height | 2.8 | 1 | 4.0 | 0.5 |
| Bead height | 0.9 | 0.5 | 1.7 | 0.5 |
| Bubble thickness | 0.4 | 0.35 | 0.2 | 0.15 |

In another example embodiment, a medical tube has the approximate dimensions shown in TABLE 5.

**Table 5**

| Feature | Dimension (mm) |
|---|---|
| Pitch | 6.4 |
| Bubble width | 5.5 |
| Bubble height | 3.2 |
| Bubble thickness on top, farthest from lumen (outer wall thickness) | 0.22 |
| Bubble thickness adjacent lumen (inner wall thickness) | 0.16 |

In another example embodiment, a medical tube has the approximate dimensions shown in TABLE 6.

**Table 6**

| Feature | Dimension (mm) | Range (±) |
|---|---|---|
| Lumen diameter | 17.2 | 5.0 |
| Pitch | 5.1 | 3.0 |
| Bubble width | 4.0 | 2.0 |
| Bead width | 2.3 | +3.0/-2.0 |
| Bubble height | 2.7 | +5.0/-2.0 |
| Bead height | 1.6 | 1.5 |
| Bubble thickness on top, farthest from lumen (outer wall thickness) | 0.24 | +0.20/-0.10 |
| Bubble thickness adjacent lumen (inner wall thickness) | 0.10 | +0.20/-0.05 |
| Outer diameter of tube | 22.5 | 3.0 |

Preferably, the low ends of the ranges of TABLE 6 correspond to each other, and the high ends of the ranges of Table 6 correspond to each other.

The embodiments of TABLES 5 and 6 can be particularly advantageous for obstructive sleep apnea applications.

TABLES 7, 8, and 9 provide example ratios between the dimensions of tube features for the tubes described in TABLES 3, 4, and 6 respectively.

**Table 7**

| Ratios | Infant | Adult |
|---|---|---|
| Lumen diameter : Pitch | 2.3 : 1 | 2.4 : 1 |
| Pitch : Bubble width | 1.1 : 1 | 1.1 : 1 |
| Pitch : Bead width | 2.2 : 1 | 3.1 : 1 |
| Bubble width : Bead width | 2.0 : 1 | 2.9 : 1 |
| Lumen diameter : Bubble height | 3.9 : 1 | 5.1 : 1 |
| Lumen diameter : Bead height | 12.2 : 1 | 12.0 : 1 |
| Bubble height : Bead height | 3.1 : 1 | 2.3 : 1 |
| Lumen diameter : Bubble thickness | 27.5 : 1 | 90.0 : 1 |

**Table 8**

| Ratios | Infant | Adult |
|---|---|---|
| Lumen diameter : Pitch | 2.3 : 1 | 2.4 : 1 |
| Pitch : Bubble width | 1.1 : 1 | 1.1 : 1 |
| Pitch : Bead width | 2.2 : 1 | 2.2 : 1 |
| Bubble width : Bead width | 2.0 : 1 | 2.1 : 1 |
| Lumen diameter : Bubble height | 3.9 : 1 | 4.5 : 1 |
| Lumen diameter : Bead height | 12.2 : 1 | 10.6 : 1 |
| Bubble height : Bead height | 3.1 : 1 | 2.4 : 1 |
| Lumen diameter : Bubble thickness | 27.5 : 1 | 90.0 : 1 |

**Table 9**

| Ratios | Value |
|---|---|
| Lumen diameter : Pitch | 3.4 : 1 |
| Pitch : Bubble width | 0.93 : 1 |
| Pitch : Bead width | 2.2 : 1 |
| Bubble width : Bead width | 1.7 : 1 |
| Lumen diameter : Bubble height | 5.4 : 1 |
| Lumen diameter : Bead height | 10.8 : 1 |
| Bubble height : Bead height | 1.7 : 1 |
| Lumen diameter : Bubble thickness on top, farthest from lumen | 71.7 : 1 |
| Lumen diameter : Bubble thickness adjacent lumen | 172 : 1 |

### Variable Pitch and/or Variable Diameter

The foregoing description discloses various constant pitch and constant diameter configurations. Certain embodiments can incorporate variable pitch and/or variable diameter, however.

A variable pitch can be desirable because it can better allow heat delivered to the gas flow to be varied along the length of the tube. The ability to control where the heat is delivered in a tube can be used to control or reduce rainout within the tube. For example, an end-of-tube temperature set point may be achieved for a given condition, yet be insufficient to prevent rainout within the tube, particularly at or near to the inlet of the tube where the gas temperature may be close to the dew point temperature (high relative humidity). Certain embodiments include the realization that redistributing the heat source to concentrate it near the entrance of the tube can help to ensure a greater axial concentration of heat in this region, Q(z) [W/m], where z is the axial displacement of the tube, beginning at the unit end.

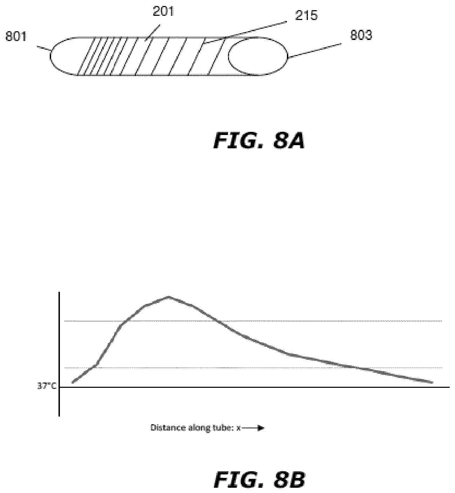

FIG. **8A** shows an example composite tube **201** with variable pitch. In this example, the tube **201** has a smaller pitch proximal the unit end **801.** Thus, the heating filaments **215** in this region will be more densely spaced, enabling greater heating at that part of the tube **201** as well as greater and more accurate temperature control. The tube **201** has a larger pitch at the patient end **803.** The greater spacing between heating filaments **215** can allow the gases to decrease in temperature as they approach the patient. This can prevent the patient from receiving gases that are too hot and can reduce rainout formation. FIG. **8B** shows the temperature profile of the composite tube of FIG. **8A****.** Other temperature profiles are also possible and can be customized to achieve specific desired effects.

The geometry of the tube **201** also affects the mechanical properties of the tube. By increasing the size of the bubble of the first elongate member, the flexibility of the tube **201** will be increase. Conversely, a smaller bubble size will produce a more rigid region of the tube **201.** By altering flexibility and rigidity, the mechanical properties of the tube **201** can be customized. By varying the diameter of the tube **201,** it is possible to have a smaller diameter near a patient interface which will increase patient comfort, improve the aesthetics, and reduce the invasiveness of the interface.

### Additional Properties

TABLES **10-13** show some example properties of a composite tube (labeled "A"), described herein, having a heating filament integrated inside the second elongate member. For comparison, properties of a Fisher & Paykel model RT100 disposable corrugated tube (labeled "B") having a heating filament helically wound inside the bore of the tube are also presented.

Measurement of resistance to flow (RTF) was carried out according to Annex A of ISO 5367:2000(E). The results are summarized in TABLE **10.** As seen below, the RTF for the composite tube is lower than the RTF for the model RT100 tube.

**Table 10**

| | RTF (cm H₂O) | | | |
|---|---|---|---|---|
| Flow rate (L/min) | 3 | 20 | 40 | 60 |
| A | 0 | 0.05 | 0.18 | 0.38 |
| B | 0 | 0.28 | 0.93 | 1.99 |

Condensate or "rainout" within the tube refers to the weight of condensate collected per day at 20 L/min gas flow rate and room temperature of 18°C. Humidified air is flowed through the tube continuously from a chamber. The tube weights are recorded before and after each day of testing. Three consecutive tests are carried out with the tube being dried in between each test. The results are shown below in TABLE **11.** The results showed that rainout is significantly lower in the composite tube than in the model RT100 tube.

**Table 11**

| Tube | A (Day 1) | A (Day 2) | A (Day 3) | B (Day 1) | B (Day 2) | B (day 3) |
|---|---|---|---|---|---|---|
| Weight before (g) | 136.20 | 136.70 | 136.70 | 111.00 | 111.10 | 111.10 |
| Weight after (g) | 139.90 | 140.00 | 139.20 | 190.20 | 178.80 | 167.10 |
| Condensate weight (g) | 3.7 | 3.3 | 2.5 | 79.20 | 67.70 | 56.00 |

The power requirement refers to the power consumed during the condensate test. In this test, the ambient air was held at 18°C. Humidification chambers (*see, e.g.,* the humidification chamber **129** in FIG. **1****)** were powered by MR850 heater bases. The heating filaments in the tubes were powered independently from a DC power supply. Different flow rates were set and the chamber was left to settle to 37°C at the chamber output. Then, the DC voltage to the circuits was altered to produce a temperature of 40°C at the circuit output. The voltage required to maintain the output temperature was recorded and the resulting power calculated. The results are shown in TABLE **12.** The results show that composite Tube A uses significantly more power than Tube B. This is because Tube B uses a helical heating filament in the tube bore to heat the gas from 37°C to 40°C. The composite tube does not tend to heat gas as quickly because the heating filament is in the wall of the tube (embedded in the second elongate member). Instead, the composite tube is designed to maintain the gas temperature and prevent rainout by maintaining the tube bore at a temperature above the dew point of the humidified gas.

**Table 12**

| Flow rate (L/min) | 40 | 30 | 20 |
|---|---|---|---|
| Tube A, power required (W) | 46.8 | 38.5 | 37.8 |
| Tube B, power required (W) | 28.0 | 27.5 | 26.8 |

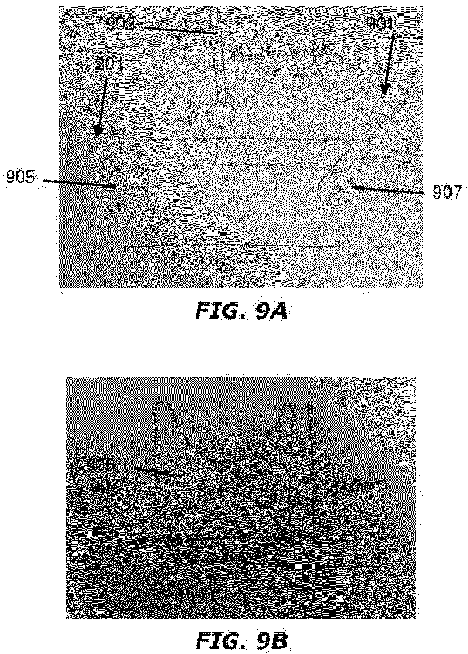

Vertical deflection can be used to quantify flexibility of a composite tube. Vertical deflection can be tested, for example, using a three-point bend test. A first 300 mm-length sample of Tube A and a second 300 mm-length sample of Tube B were each tested on a flexibility jig. A front-plan cross-sectional schematic of the flexibility jig is shown in FIG. **9A****.** The jig **901** used a 25-mm rod **903** with a fixed mass of 120 g to apply a force to each tube **201,** which was positioned between two rollers **905** and **907.** The rollers were spaced 150 mm apart. The force exerted by the rod **903** was about 1.2 N (0.12 kg × 9.81 m/s²). A detailed front-plan cross-sectional schematic of rollers **905** and **907** is shown in FIG. **9B****.** Both rollers **905** and **907** had the same dimensions, which are shown in FIG. **9B****.** An Instron 5560 Test System instrument was used to measure load and extension. Each tube sample was tested three times; measuring the extension of the tube against the applied load, to obtain average respective stiffness constants. The average stiffness constants for Tube A and Tube B are reproduced in TABLE **13.**

**Table 13**

| Tube | Stiffness (N/mm) |
|---|---|
| A | 0.028 |
| B | 0.088 |

Tube weight can be very important, particularly for CPAP applications. If a patient experiences less weight near the patient's face, the patient will be more comfortable during sleep. A lighter composite tube **201** will not pull the patient's head in a particular direction as much as a heavier tube. To ensure patient comfort, it is possible to specify that the total mass or weight in a region near the patient end of the composite tube **201** must be less than a specified value. In certain embodiments, the tube mass in the 300 mm nearest the patient end is less than 24 g (or about 24 g). Desirably, the tube mass in the 300 mm nearest the patient end is less than 16 g (or about 16 g). In certain embodiments, the tube mass in the 300 mm nearest the patient end is less than 15 g (or about 15 g). It is also possible to specify that the total mass of the composite tube be less than a specified value. In certain embodiments, the tube mass is less than 130 g (or about 130 g). Desirably, the tube mass is less than 120 g (or about 120 g). In certain embodiments, the tube mass is less than 100 g (or about 100 g).

The following discussion now describes additional properties relating to a composite tube **201** with two bubbles between wraps of the second elongate member **205,** as discussed above.

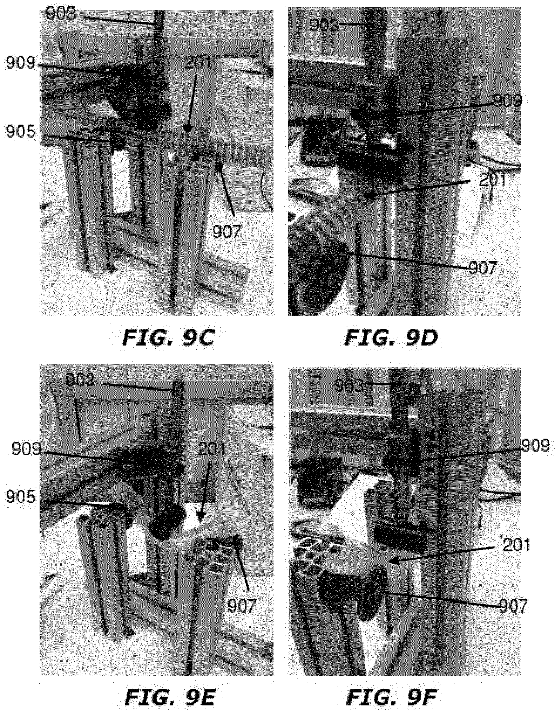

A first 300 mm-length sample of tube comprising two bubbles between wraps of the second elongate member **205** and a second 300 mm-length sample of tube comprising one bubble between wraps of the second elongate member **205** were each tested on the flexibility jig **901** discussed above. The vertical deflection was measured using the position of the fixed weight with respect to a vertical support **909** of the flexibility jig, shown in the photographs of FIGS. **9C** through **9F****.**

FIG. **9C** shows a front-perspective view of the second sample under testing in the jig **901.** FIG. **9D** shows a rear-perspective view of the second sample under testing in the jig **901.** FIG. **9E** shows a front-perspective view of the first sample under testing in the jig **901.** FIG. **9F** shows a rear-perspective view of the first sample under testing in the jig **901.** As shown in FIGS. **9C** through **9F****,** the second sample shown in FIGS. **9E** and **9F** had substantially greater vertical deflection than the first sample shown in FIGS. **9C** and **9D****.** Specifically, the second sample had a vertical deflection of 3 mm, while the first sample was much more flexible, having a vertical deflection of 42 mm.

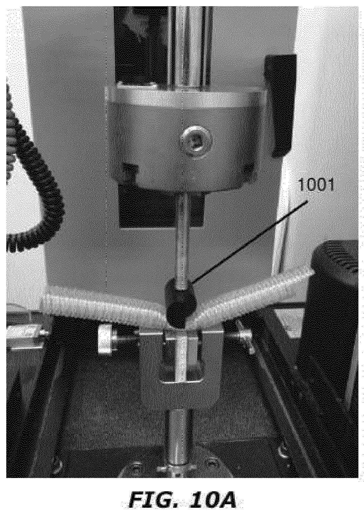

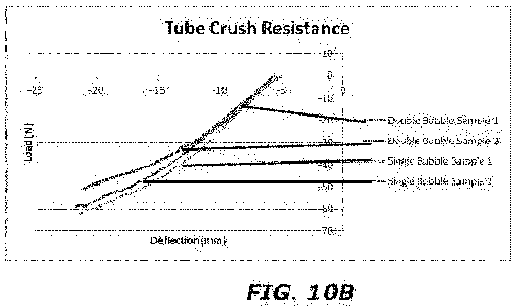

Crush resistance testing was performed on four tube samples using an Instron machine set up as shown in the photograph in FIG. **10A****.** The cylinder **1001** was plunged downwards 16 mm from the top of the tube at a rate of 60 mm/min. The Instron machine has a load cell to accurately measure force exerted on a component versus extension. The load vs. extension was plotted, as shown in FIG. **10B****.**

The crush stiffness for each sample was found by fitting a line of best fit to the data of FIG. **10B** and calculating its gradient. The calculated crush stiffness for each sample is shown in TABLE **14A.** In TABLE **14A** (and elsewhere in this disclosure), the designation "double bubble" refers to a sample of tube comprising two bubbles between wraps of the second elongate member **205,** when the sample is viewed in longitudinal cross section. The designation "single bubble" refers to a sample of tube comprising a single bubble between wraps of the second elongate member **205,** when the sample is viewed in longitudinal cross section. The average crush stiffness (measured in N/mm) represents the average maximum force per unit width which produces no crush.

**Table 14A**

| Sample | Crush Stiffness (N/mm) | Average |
|---|---|---|
| Double Bubble, Sample 1 | 3.26 | 3.21 |
| Double Bubble, Sample 2 | 3.15 | |
| Single Bubble, Sample 1 | 3.98 | 3.86 |
| Single Bubble, Sample 2 | 3.74 | |

As shown in the foregoing table, single bubble tubes had an average crush stiffness of 3.86 N/mm, while double bubble tubes had an average crush stiffness of 3.21 N/mm. In other words, the double bubble tubes had an approximately 16.8% lower resistance to crush than the single bubble tubes. Nevertheless, crush stiffness per unit thickness for the double bubble tubes was observed to be approximately 165% of the value for the single bubble tubes, as shown below in TABLE **14B.**

**Table 14B**

| | Bubble Thickness (mm) | Crush Stiffness (N/mm) | Stiffness/Bubble Thickness (N/mm²) |
|---|---|---|---|
| Double Bubble | 0.22 | 3.21 | 14.32 |
| Single Bubble | 0.43 | 3.86 | 8.70 |

Stated another way, when outer bubble thickness is taken into account, the double bubble tube is around 65% more resistant to crush than the single bubble tube variant. Similar to the bubbles shown in FIGS. **2F** and **2G****,** the tested bubbles in the double bubble configuration are taller than they are wide, which results in more material in the vertical plane. Thus, it is believed that the unexpected improvement in crush resistance per unit thickness of the bubble may be attributed to the additional vertical web between beads working in the direction of crush.

Tensile testing was also performed on the single and double bubble tube samples. Both samples were 230 mm in length and were elongated by 15 mm at a rate of 10 mm/min. The force required to elongate the samples was measured. The results are shown in TABLE **14C.**

**Table 14C**

| Sample | Peak Force at 15mm extension (N) |
|---|---|
| Double Bubble | 17.60 |
| Single Bubble | 54.65 |

As shown in TABLE **14C,** the double bubble tube was significantly more extensible in the axial (longitudinal) plane. This increase in longitudinal extensibility is believed to be due to the single bubble tube having more material between the beads working in the axial plane.

### Thermal Properties

In embodiments of a composite tube **201** incorporating a heating filament **215,** heat can be lost through the walls of the first elongate member **203,** resulting in uneven heating. As explained above, one way to compensate for these heat losses is to apply an external heating source at the first elongate member **203** walls, which helps to regulate the temperature and counter the heat loss. Other methods for optimizing thermal properties can also be used, however.

Reference is again made to FIGS. **5A** through **5C****,** which demonstrate example configurations for bubble height (that is, the cross-sectional height of the first elongate member **203** measured from the surface facing the inner lumen to the surface forming the maximum outer diameter) to improve thermal properties.

The dimensions of the bubble can be selected to reduce heat loss from the composite tube **201.** Generally, increasing the height of the bubble increases the effective thermal resistance of the tube **201,** because a larger bubble height permits the first elongate member **203** to hold more insulating air. However, it was discovered that, at a certain bubble height, changes in air density cause convection inside the tube **201,** thereby increasing heat loss. Also, at a certain bubble height the surface area becomes so large that the heat lost through surface outweighs the benefits of the increased height of the bubble. Certain embodiments include these realizations.

The radius of curvature and the curvature of the bubble can be useful for determining a desirable bubble height. The curvature of an object is defined as the inverse of the radius of curvature of that object. Therefore, the larger a radius of curvature an object has, the less curved the object is. For example, a flat surface would have a radius of curvature of ∞, and therefore a curvature of 0.

FIG. **5A** shows a longitudinal cross-section of a top portion of a composite tube. FIG. **5A** shows an embodiment of a composite tube **201** where the bubble has a large height. In this example, the bubble has a relatively small radius of curvature and therefore a large curvature. Also, the bubble is approximately three to four times greater in height than the height of the second elongate member **205.**

FIG. **5B** shows a longitudinal cross-section of a top portion of another composite tube. FIG. **5B** shows an embodiment of a composite tube **201** where the bubble is flattened on top. In this example, the bubble has a very large radius of curvature but a small curvature. Also, the bubble is approximately the same height as the second elongate member **205.**

FIG. **5C** shows a longitudinal cross-section of a top portion of another composite tube. FIG. **5C** shows an embodiment of a composite tube **201** where the width of the bubble is greater than the height of the bubble. In this example, the bubble has radius of curvature and the curvature between that of FIG. **5A** and FIG. **5B****,** and the center of the radius for the upper portion of the bubble is outside of the bubble (as compared to FIG. **5A**). The inflection points on the left and right sides of the bubble are about at the middle (heightwise) of the bubble (as opposed to in the lower portion of the bubble, as in FIG. **5A**). Also, the height of the bubble is approximately double that of the second elongate member **205,** resulting in a bubble height between that of FIG. **5A** and FIG. **5B****.**

The configuration of FIG. **5A** resulted in the lowest heat loss from the tube. The configuration of FIG. **5B** resulted in the highest heat loss from the tube. The configuration of FIG. **5C** had intermediate heat loss between the configurations of FIG. **5A** and **5B****.** However, the large external surface area and convective heat transfer in the configuration of FIG. **5A** led to inefficient heating. Thus, of the three bubble arrangements of FIGS. **5A-5C****,** FIG. **5C** was determined to have the best overall thermal properties. The practical implication of this thermal efficiency is that, when the same thermal energy was input to the three tubes, the configuration of FIG. **5C** allowed for the largest temperature rise along the length of the tube. The bubble of FIG. **5C** is sufficiently large to increase the insulating air volume, but not large enough to cause a significant convective heat loss. The configuration of FIG. **5B** was determined to have the poorest thermal properties, namely that the configuration of FIG. **5B** allowed for the smallest temperature rise along the length of the tube. The configuration of FIG. **5A** had intermediate thermal properties and allowed for a lower temperature rise than the configuration of FIG. **5C****.**

It should be appreciated that although the FIG. **5C** configuration may be preferred in certain embodiments, other configurations, including those of FIGS. **5A****,** **5B** and other variations, may be utilized in other embodiments as may be desired.

TABLE **15** shows the height of the bubble, the outer diameter of the tube, and the radius of curvature of the configurations shown in each of FIGS. **5A****,** **5B,** and **5C****.**

**Table 15**

| Tube (Fig.) | 5A | 5B | 5C |
|---|---|---|---|
| Bubble height (mm) | 3.5 | 5.25 | 1.75 |
| Outer diameter (mm) | 21.5 | 23.25 | 19.75 |
| Radius of curvature (mm) | 5.4 | 3.3 | 24.3 |

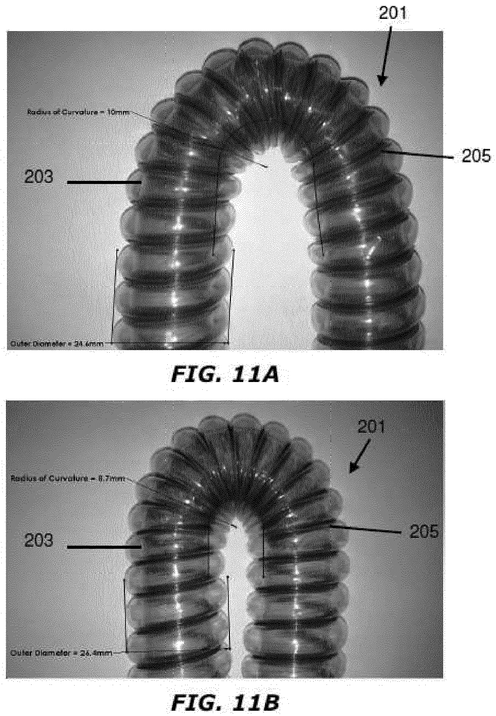

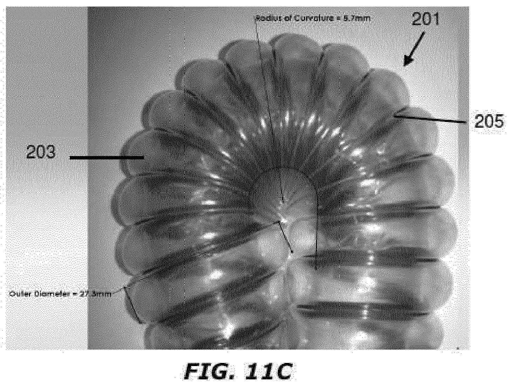

TABLE **16A** shows the height of the bubble, the outer diameter, and the radius of curvature of further configurations as shown in FIGS. **11A, 11B****,** and **11C****.**

**Table 8A**

| Tube (Fig.) | 10A | 10B | 10C |
|---|---|---|---|
| Bubble height (mm) | 6.6 | 8.4 | 9.3 |
| Outer diameter (mm) | 24.6 | 26.4 | 27.3 |
| Radius of curvature (mm) | 10 | 8.7 | 5.7 |

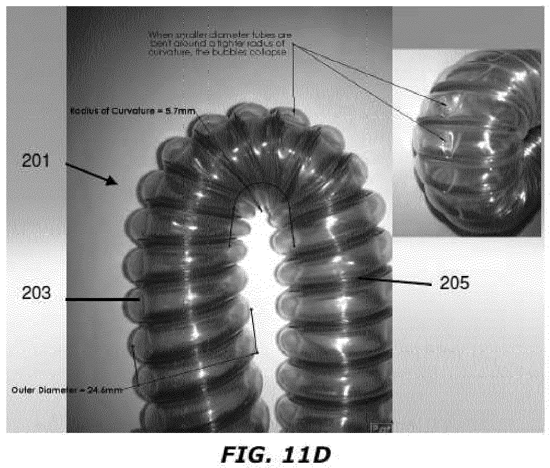

It should be noted that, in general, the smaller the radius of curvature, the tighter the tube can be bent around itself without the bubble collapsing or "kinking." For example, FIG. **11D** shows a tube that has been bent beyond its radius of curvature (specifically, it shows the tube of FIG **11A** bent around a radius of curvature of 5.7 mm), thereby causing kinking in the walls of the bubble. Kinking is generally undesirable, as it can detract from the appearance of the tube, and can impair the thermal properties of the tube.

Accordingly, in some applications, the configurations with increased bending properties (such as those shown in FIGS. **5A** or **5B**) can be desirable despite having less efficient thermal properties. In some applications, it has been found that a tube with an outer diameter of 25 mm to 26 mm (or about 25 mm to about 25 mm) provides satisfactory performance. It should be appreciated that although the configurations of FIGS. **5A** and **5B** may be preferred in certain embodiments, other configurations, including those of FIGS. **11A-11D** and other variations, may be utilized in other embodiments as may be desired.

Reference is again made to FIGS. **5C** through **5F** which demonstrate example positioning of heating element **215** with similar bubble shapes to improve thermal properties. The location of the heating element **215** can change the thermal properties within the composite tube **201.**

FIG. **5C** shows a longitudinal cross-section of a top portion of another composite tube. FIG. **5C** shows an embodiment of a composite tube **201** where the heating elements **215** are centrally located in the second elongate member **205.** This example shows the heating elements **215** close to one another and not close to the bubble wall.

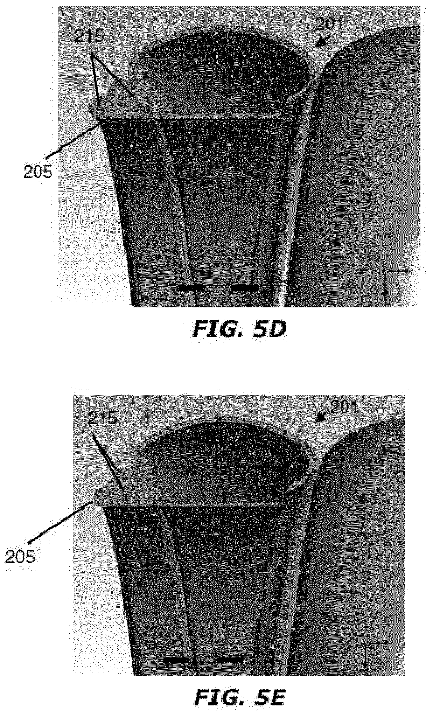

FIG. **5D** shows a longitudinal cross-section of a top portion of another composite tube. FIG. **5D** shows an embodiment of a composite tube **201** in which the heating elements **215** are spaced farther apart, as compared to FIG. **5C****,** in the second elongate member **205.** These heating elements are closer to the bubble wall and provide for better regulation of heat within the composite tube **201.**

FIG. **5E** shows a longitudinal cross-section of a top portion of another composite tube. FIG. **5E** shows an embodiment of a composite tube **201** wherein the heating elements **215** are spaced on top of each other in the vertical axis of the second elongate member **205.** In this example, the heating elements **215** are equally close to each bubble wall.

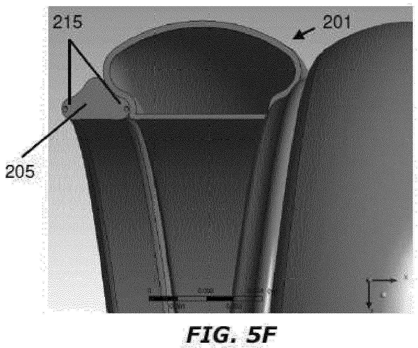

FIG. **5F** shows a longitudinal cross-section of a top portion of another composite tube. FIG. **5F** shows an embodiment of a composite tube **201** where the heating elements **215** are spaced at opposite ends of the second elongate member **205.** The heating elements **215** are close to the bubble wall, especially as compared to FIGS. **5C-5E****.**

Of the four filament arrangements of FIGS. **5C-5F****,** FIG. **5F** was determined to have the best thermal properties. Because of their similar bubble shapes, all of the configurations experienced similar heat loss from the tube. However, when the same thermal energy was input to the tubes, the filament configuration of FIG. **5F** allowed for the largest temperature rise along the length of the tube, for the bulk gas temperature within the tube. The configuration of FIG. **5D** was determined to have the next best thermal properties and allowed for the next largest temperature rise along the length of tube. The configuration of FIG. **5C** performed next best. The configuration of FIG. **5E** had the poorest performance and allowed for the smallest temperature rise along the length of the tube, when the same amount of heat was input.

It should be appreciated that although the FIG. **5F** configuration may be preferred in certain embodiments, other configurations, including those of FIGS. **5C****,** **5D, 5E****,** and other variations, may be utilized in other embodiments as may be desired.

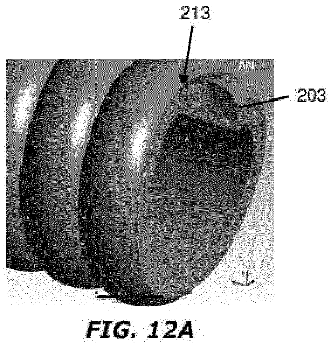

Reference is next made to FIGS. **12A** through **12C****,** which demonstrate example configurations for stacking of the first elongate member **203.** It was discovered that heat distribution can be improved in certain embodiments by stacking multiple bubbles. These embodiments can be more beneficial when using an internal heating filament **215.** FIG. **12A** shows a longitudinal cross-section of a top portion of another composite tube. FIG. **12A** shows a cross section of a composite tube **201** without any stacking.

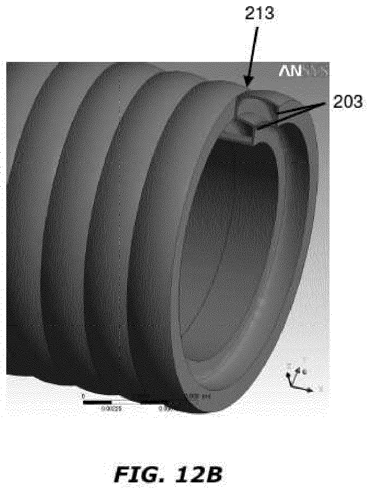

FIG. **12B** shows a longitudinal cross-section of a top portion of another composite tube. FIG. **12B** shows another example composite tube **201** with stacked bubbles. In this example, two bubbles are stacked on top of each other to form the first elongate member **203.** As compared to FIG. **12A****,** the total bubble height is maintained, but the bubble pitch is half of FIG. **12A****.** Also, the embodiment in FIG. **12B** has only a slight reduction in air volume. The stacking of the bubbles reduces natural convection and heat transfer in the gap between bubbles **213** and lowers the overall thermal resistance. The heat flow path increases in the stacked bubbles allowing heat to more easily distribute through the composite tube **201.**

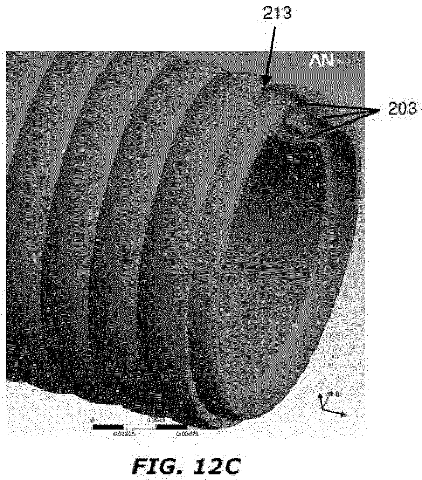

FIG. **12C** shows a longitudinal cross-section of a top portion of another composite tube. FIG. **12C** shows another example of a composite tube **201** with stacked bubbles. In this example, three bubbles are stacked on top of each other to form the first elongate member **203.** As compared to FIG. **12A****,** the total bubble height is maintained, but the bubble pitch is a third of FIG. **12A****.** Also, the embodiment in FIG. **12B** has only a slight reduction in air volume. The stacking of the bubbles reduces natural convection and heat transfer in the gap between bubbles **213.**

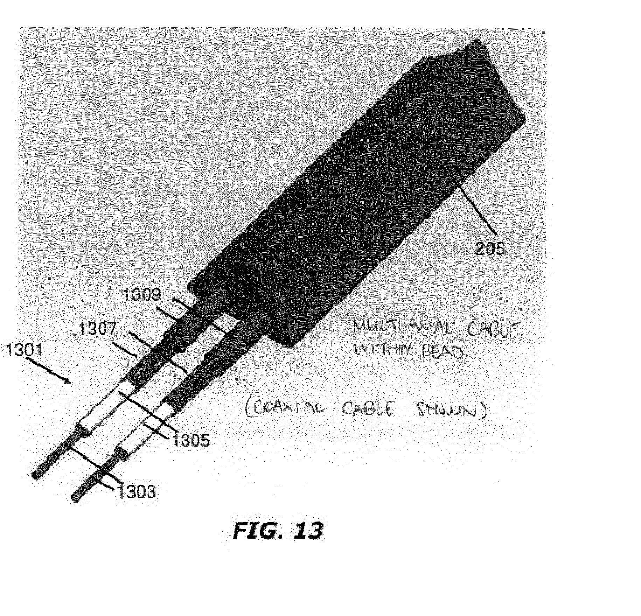

Referring now to FIG. **13****,** additional possible properties of the second elongate member **205** are shown. The second elongate member **205** comprises one or more coaxial cables **1301** having a conductor **1303** surrounded by an insulation layer **1305,** a shield layer **1307,** and a sheath layer **1309.** In certain embodiments, one or more of cables **1301** can be a multi-axial cable, that is, have multiple conductors **1303** arranged within the insulation layer **1305.** In this manner, a single assembly containing multiple wires (including heater wires and/or sensor wires) can be used in the second elongate member **205,** thereby simplifying assembly and providing some shielding (via the shield layer **1307**) from RF interference and the like.

In some embodiments, one or more data transmission cables can be included in the second elongate member **205.** The data transmission cables can comprise fiber optic cables. In at least one embodiment, a single fiber optic cable is included in the second elongate member **205** and used in a passive mode. In a passive mode, at a first end of the cable, a light source and a light sensor are provided. At a second end, a reflector is provided. In use, the light source provides a quantity of light having certain properties towards the reflector. The reflector then reflects the light towards the light sensor, which can analyze the reflected light to determine the properties of the light. The reflector can be adapted to change the property of the reflected light depending on a property of the system. For example, the reflector can be used to monitor condensation within the interface. The reflector can comprise a material which, for example, changes color depending on the presence of condensation at the second end. The reflector can alternatively or additionally include a material which changes color or the like depending on the level of humidity (either relative humidity or absolute humidity), and/or the temperature of gas at the second end, and/or gas composition such as inhaled O₂ or exhaled CO₂.

Referring again to FIG. **2B****,** in some embodiments, a fluid (gas or liquid) flow can be passed along the space inside the first elongate member **203.** In such embodiments, it is desirable that at least a portion of the first elongate member **203** is formed of a breathable material. Breathable is used herein to mean appreciably permeable to water vapor and substantially impermeable to liquid water and the bulk flow of gases. Suitable breathable materials include an activated perfluorinated polymer material with extreme hydrophilic properties, such as NAFION^{®}, or a hydrophilic polyester block copolymer, such as SYMPATEX^{®}. Other suitable materials include polymers commercially embodied in EVAQUA^{™} and EVAQUA 2^{™} conduits (Fisher & Paykel Healthcare Ltd., Auckland, New Zealand). Suitable materials are further described in PCT Publication WO 2011/077250, filed Dec. 22, 2010 and published June 30, 2011, and U.S. Patent No. 6,769,431, filed May 8, 2001 and issued Aug. 3, 2003.

The flow through the first elongate member **203** can be useful for drying or humidifying the gas flow through the tube **201** lumen **207,** as desired. Conversely, the flow through the tube **201** lumen **207** can be useful for drying or humidifying the gas flow through the first elongate member **203,** as desired. Exhaled respiratory gases can be carried through the first elongate member **203.** As another example, a liquid such as liquid water can be carried. As yet another example, a humidified or saturated gas stream can be carried. As yet another example, a dry gas stream or a stream of compressed ambient air can be carried. In the foregoing embodiments, the first elongate member **203** can be open on both ends, to facilitate the flow of fluid through the first elongate member **203.** One end of the first elongate member **203** can be connected to a suitable source, such as a source of exhaled respiratory gas, liquid water, humidified gas, dry gas, or compressed air, as desired. The other end can be connected to a suitable outlet or allowed to vent to the atmosphere.

For example, with reference to FIG. **2B****,** the portion **211** of the first elongate member **203** that forms the lumen **207** of the tube **201** can be formed of the breathable material, as described above. The outward-facing portion **219** (facing the ambient atmosphere and facing away from the lumen) of the first elongate member **203** can be formed of an impermeable material, that is, a material that is not appreciably permeable to water vapor, liquid water, or the bulk flow of gases, as described elsewhere in this disclosure. In use, a quantity of humidification fluid (such as water) can be passed through the space formed by the first elongate member **203.** As the humidification fluid is heated (for example, by the heating filaments **215** disposed in the second elongate member **205**), a portion of the humidification fluid will tend to evaporate. The water vapor can then pass through the breathable portion **211** into the bulk gas flow through the lumen **207,** thereby humidifying the bulk gas flow. In such an embodiment, the combination of the humidification fluid, first elongate member **203,** and heating filaments **215** can provide a means for humidifying the gas flow within the lumen **207** such that a standalone humidifier can be omitted from the system.

As another example, a gas flow can be passed along the space inside the first elongate member **203.** For example, exhaled respiratory gases can be carried. Referring again to FIG. **2B****,** the first elongate member **203** or at least the outward-facing portion **219** of the first elongate member **203** is made of a breathable material, as described above. In this manner, as the exhaled gas travels along the length of the first elongate member **203,** it will tend to dry from about 100% relative humidity at the patient-end to reduced humidity level at the opposite end.

Co-extrusion is a suitable method for forming a first elongate member **203** comprising a portion (**211** or **219,** depending on the desired application) formed of a breathable material and a portion (**219** or **211,** depending on the desired application) formed of an impermeable material.

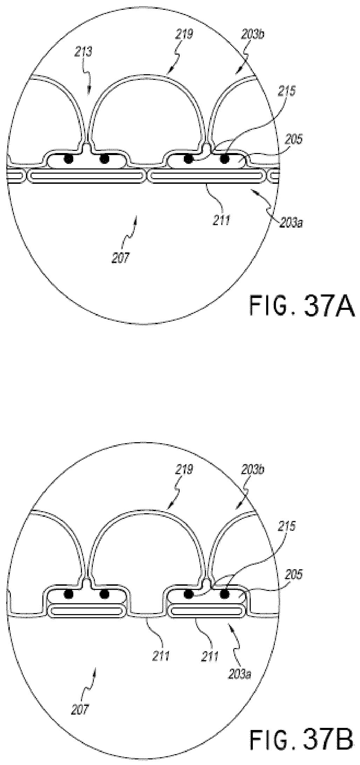

In addition, although certain foregoing embodiments have been described with reference to a single first elongate member **203** comprising breathable and impermeable portions, it should be appreciated that desired results (such as humidification of the gas flow within the lumen **207**) also can be achieved using a plurality of first elongate members **203.** Suitable embodiments are shown in FIGS. **12B****,** **12C****,** **37A** and **37B****.**

FIG. **37A** shows a cross section of tube comprising two first elongate members. A first first-elongate-member **203*a*** is disposed proximal the tube lumen **207.** A second first-elongate-member **203*b*** faces the ambient atmosphere and faces away from the lumen **207.** The inner portion of the first first-elongate-member **203*a*** forms the lumen **207** wall. The first first-elongate-member **203*a*** can define a conduit for a humidification fluid, such as liquid water. The first first-elongate-member **203*a*** can be formed from a breathable material. As the humidification fluid is heated (for example, by the heating filaments **215** disposed in the second elongate member **205**), a portion of the humidification fluid will tend to evaporate. The vapor can then pass through the walls of the first first-elongate-member **203*a*** into the bulk gas flow through the lumen **207,** thereby humidifying the bulk gas flow. In such an embodiment, the combination of the humidification fluid, first first-elongate-member **203a,** and heating filaments **215** can provide a means for humidifying the gas flow within the lumen **207** such that a standalone humidifier can be omitted from the system. It should be appreciated that the dimensions shown in FIG. **37A** are not necessarily to scale. For example, the first first-elongate-member **203*a*** can be relatively larger, and the second first-elongate-member **203*b*** can be relatively smaller as shown in FIG. **12B****.** It should also be appreciated that the heating filaments **215** need not necessarily be housed in the second elongate member **205.** For example, as shown in FIG. **12B****,** the second elongate member can be omitted. The heating filaments **215** can be housed, for instance, in a portion of the second first-elongate-member **203*b*** proximal the first first-elongate-member ***203a.***

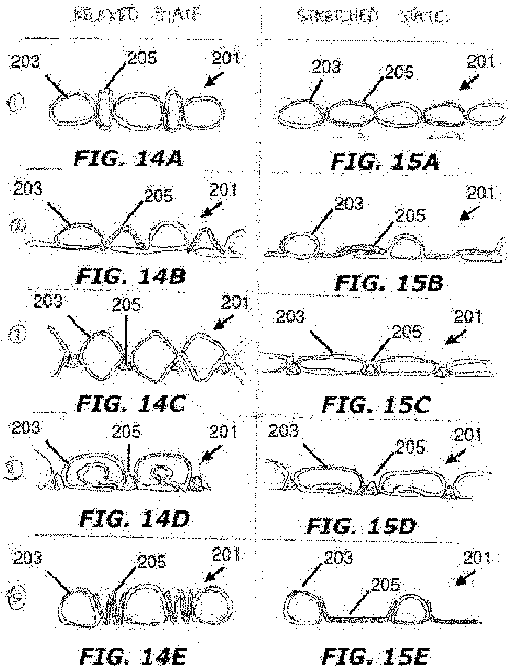

FIG. **37B** also shows a cross section of tube comprising two first elongate members. A first first-elongate-member **203*a*** is disposed proximal the tube lumen **207.** A second first-elongate-member **203*b*** faces the ambient atmosphere and faces away from the lumen **207.** The inner portion of the first first-elongate-member **203*a*** forms part of the lumen **207** wall. An inner portion of the second first-elongate-member **203*b*** also forms part of the lumen **207** wall. As discussed above with reference to FIG. **37A****,** the first first-elongate-member **203*a*** can define a conduit for a humidification fluid, such as liquid water, and the combination of the humidification fluid, first first-elongate-member **203,** and heating filaments **215** can provide a means for humidifying the gas flow within the lumen **207** such that a standalone humidifier can be omitted from the system. Again, it should be appreciated that the dimensions shown in FIG. **37B** are not necessarily to scale. For example, the first first-elongate-member **203*a*** can be relatively larger, and the second first-elongate-member **203*b*** can be relatively smaller as shown in FIG. **12B****.** It should also be appreciated that the heating filament need not necessarily be housed in the second elongate member. For example, as shown in FIG. **12B****,** the second elongate member can be omitted. The heating filament can be housed, for instance, in a portion of the second first-elongate-member **203*b*** proximal the first first-elongate-member **203*a*.** [0235] Referring now to FIGS. **14A-****14E** and **15A-15E,** some variations of the composite tube **201** configuration are shown which are adapted to provide increased lateral stretch in the composite tube **201.** FIGS. **15A-****15E** show a stretched state of the composite tubes shown in FIGS. **14A-14E****,** respectively.

Certain embodiments include the realization that the tubes shown in FIGS. **14A, 14B,** and **14E** comprise a second elongate member **205** having a shape that increases stretch capability. For example, in FIG. **14A****,** the second elongate member **205** is substantially oblate having a profile substantially the same height as the first elongate member **203.** As shown in FIG. **15A****,** this allows the second elongate member **205** to deform outwards to at least twice the width compared to the second elongate member **205** at rest. In FIG. **14B** and **14E****,** the second elongate member **205** is shaped so as to have an accordion-like shape. On stretching, the second elongate member **205** can therefore accommodate an increase amount of stretching by flattening (as shown in FIGS. **15B** and **15E****,** respectively).

In FIGS. **14C** and **14D****,** the first elongate member **203** is given a shape that allows it to deform outward, thereby allowing an increased lateral stretch (as shown in FIGS. **15C** and **15D****,** respectively).

### Medical Circuits

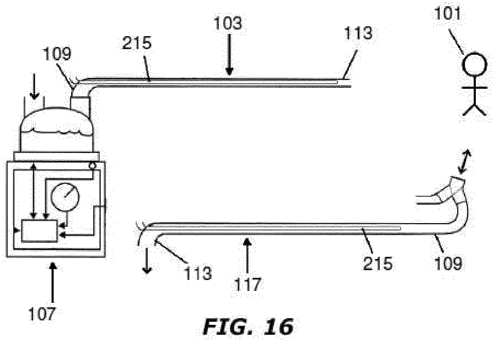

Reference is next made to FIG. **16****,** which shows an example medical circuit according to at least one embodiment. The circuit comprises one or more composite tubes as described above, namely for the inspiratory tube **103** and/or the expiratory tube **117.** The properties of the inspiratory tube **103** and the expiratory tube **117** are similar to the tubes described above with respect to **FIG. 1****.** The inspiratory tube **103** has an inlet **109,** communicating with a humidifier **107,** and an outlet **113,** through which humidified gases are provided to the patient **101.** The expiratory tube **117** also has an inlet **109,** which receives exhaled humidified gases from the patient, and an outlet **113.** As described above with respect to FIG. **1****,** the outlet **113** of the expiratory tube **117** can vent exhaled gases to the atmosphere, to the ventilator/blower unit **105,** to an air scrubber/filter (not shown), or to any other suitable location.

As described above, heating filaments **215** can be placed within the inspiratory tube **103** and/or the expiratory tube **117** to reduce the risk of rain out in the tubes by maintaining the tube wall temperature above the dew point temperature.

### Component of an Insufflation System

Laparoscopic surgery, also called minimally invasive surgery (MIS), or keyhole surgery, is a modern surgical technique in which operations in the abdomen are performed through small incisions (usually 0.5 to 1.5 cm) as compared to larger incisions needed in traditional surgical procedures. Laparoscopic surgery includes operations within the abdominal or pelvic cavities. During laparoscopic surgery with insufflation, it may be desirable for the insufflation gas (commonly CO₂) to be humidified before being passed into the abdominal cavity. This can help prevent "drying out" of the patient's internal organs, and can decrease the amount of time needed for recovery from surgery. Insufflation systems generally comprise humidifier chambers that hold a quantity of water within them. The humidifier generally includes a heater plate that heats the water to create a water vapour that is transmitted into the incoming gases to humidify the gases. The gases are transported out of the humidifier with the water vapor.

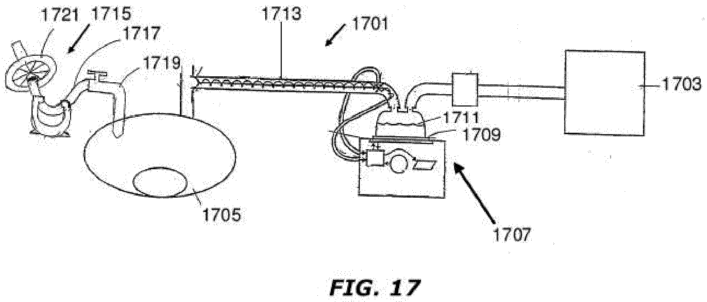

Reference is next made to FIG. **17****,** which shows an insufflation system **1701,** according to at least one embodiment. The insufflation system **1701** includes an insufflator **1703** that produces a stream of insufflation gases at a pressure above atmospheric for delivery into the patient **1705** abdominal or peritoneal cavity. The gases pass into a humidifier **1707,** including a heater base **1709** and humidifier chamber **1711,** with the chamber **1711** in use in contact with the heater base **1709** so that the heater base **1709** provides heat to the chamber **1711.** In the humidifier **1707,** the insufflation gases are passed through the chamber **1711** so that they become humidified to an appropriate level of moisture.

The system **1701** includes a delivery conduit **1713** that connects between the humidifier chamber **1711** and the patient **1705** peritoneal cavity or surgical site. The conduit **1713** has a first end and second end, the first end being connected to the outlet of the humidifier chamber **1711** and receiving humidified gases from the chamber **1711.** The second end of the conduit **1713** is placed in the patient **1705** surgical site or peritoneal cavity and humidified insufflation gases travel from the chamber **1711,** through the conduit **1713** and into the surgical site to insufflate and expand the surgical site or peritoneal cavity. The system also includes a controller (not shown) that regulates the amount of humidity supplied to the gases by controlling the power supplied to the heater base **1709.** The controller can also be used to monitor water in the humidifier chamber **1711.** A smoke evacuation system **1715** is shown leading out of the body cavity of the patient **1705.**

The smoke evacuation system **1715** can be used in conjunction with the insufflation system **1701** described above or may be used with other suitable insufflation systems. The smoke evacuation system **1715** comprises a discharge or exhaust limb **1717,** a discharge assembly **1719,** and a filter **1721.** The discharge limb **1717** connects between the filter **1721** and the discharge assembly **1719,** which in use is located in or adjacent to the patient **1705** surgical site or peritoneal cavity. The discharge limb **1717** is a self-supporting tube (that is, the tube is capable of supporting its own weight without collapsing) with two open ends: an operative site end and an outlet end.

At least one embodiment includes the realization that the use of a composite tube as the conduit **1713** can deliver humidified gases to the patient **1705** surgical site with minimized heat loss from the humidified gases.

### Coaxial Tube

A coaxial breathing tube can also comprise a composite tube as described above. In a coaxial breathing tube, a first gas space is an inspiratory limb or an expiratory limb, and the second gas space is the other of the inspiratory limb or expiratory limb. One gas passageway is provided between the inlet of said inspiratory limb and the outlet of said inspiratory limb, and one gas passageway is provided between the inlet of said expiratory limb and the outlet of said expiratory limb. In one embodiment, the first gas space is said inspiratory limb, and the second gas space is said expiratory limb. Alternatively, the first gas space can be the expiratory limb, and the second gas space can be the inspiratory limb.

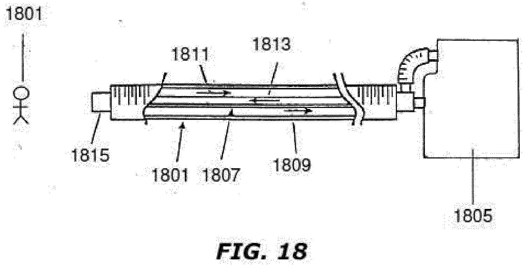

Reference is next made to FIG. **18****,** which shows a coaxial tube **1801** according to at least one embodiment. In this example, the coaxial tube **1801** is provided between a patient **1801** and a ventilator **1805.** Expiratory gases and inspiratory gases each flow in one of the inner tube **1807** or the space **1809** between the inner tube **1807** and the outer tube **1811.** It will be appreciated that the outer tube **1811** may not be exactly aligned with the inner tube **1807.** Rather, "coaxial" refers to a tube situated inside another tube.

For heat transfer reasons, the inner tube **1807** can carry the inspiratory gases in the space **1813** therewithin, while the expiratory gases are carried in the space **1809** between the inner tube **1807** and the outer tube **1811.** This airflow configuration is indicated by arrows. However, a reverse configuration is also possible, in which the outer tube **1811** carries inspiratory gases and the inner tube **1807** carries expiratory gases.

In at least one embodiment, the inner tube **1807** is formed from a corrugated tube, such as a Fisher & Paykel model RT100 disposable tube. The outer tube **1811** can be formed from a composite tube, as described above.

With a coaxial tube **1801,** the ventilator **1805** may not become aware of a leak in the inner tube **1807.** Such a leak may short circuit the patient **1801,** meaning that the patient **1801** will not be supplied with sufficient oxygen. Such a short circuit may be detected by placement of a sensor at the patient end of the coaxial tube **1801.** This sensor may be located in the patient end connector **1815.** A short circuit closer to the ventilator **1805** will lead to continued patient **1801** re-breathing of the air volume close to the patient **1801.** This will lead to a rise in the concentration of carbon dioxide in the inspiratory flow space **1813** close to the patient **1801,** which can be detected directly by a CO₂ sensor. Such a sensor may comprise any one of a number of such sensors as is currently commercially available. Alternatively, this re-breathing may be detected by monitoring the temperature of the gases at the patient end connector **1815,** wherein a rise in temperature above a predetermined level indicates that re-breathing is occurring.

In addition to the above to reduce or eliminate the formation of condensation within either the inner tube **1807** or outer tube **1811,** and to maintain a substantially uniform temperature in the gases flow through the coaxial tube **1801,** a heater, such as a resistance heater filament, may be provided within either the inner tube **1807** or outer tube **1811,** disposed within the gases spaces **1809** or **1813,** or within the inner tube **1807** or outer tube **1811** walls themselves.

### Nasal Cannulas and Other Patient Interfaces

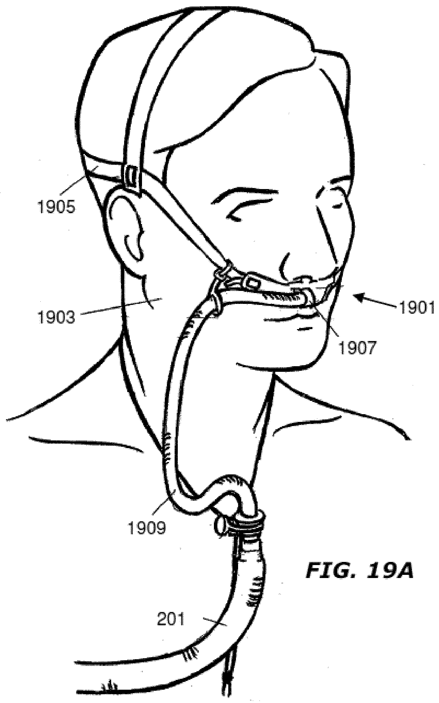

Reference is next made to FIG. **19A****,** which shows composite tube **201** in use with a nasal cannula patient interface **1901.** In this example, the patient interface **1901** is positioned on the face of the patient **1903** with headgear **1905** secured around the back of the head of the patient **1901.** The patient interface includes a cannula body **1907** and a delivery tube **1909.** A composite tube **201,** as described, communicates with the delivery tube **1909** to supply inspiratory gases to the patient interface **1901.**

In the past, the delivery tube **1909** has been used to decouple the weight of heated respiratory tubing from the patient interface **1901.** Previously-used delivery tubes **1909** consisted of a length of flexible tubing. It was important that the delivery tube **1909** be lightweight so that the mass of the delivery tube **1909** did not drag the patient interface **1901** off a patient's face. Heated tubes were substantially bulkier and heavier than unheated tubes. Thus, previously-used delivery tubes **1909** were unheated. In order to achieve satisfactory flexibility, previously-used delivery tubes **1909** also had poor insulation properties. Without good insulating and heating, rainout in the delivery tubes **1909** was a problem. Thus, delivery tubes **1909** were kept as short as possible to minimize rainout. The short length did not consistently prevent the weight of heated respiratory tubing from dragging the patient interface **1901,** however. Thus, previously-used delivery tubes have a number of drawbacks.

The composite tubes **201** described herein provide good insulation while maintaining good flexibility and light weight. Thus, in some embodiments, the delivery tube **1909** can be a composite tube **201.** Composite tubes **201** can provide improved insulation properties over delivery tubes previously known in the art. In addition, the length of the delivery tube can be longer and provide better decoupling of tube drag. The composite-tube **201** delivery tube **201** can optionally have heating filaments (not shown) in the second elongate member (not shown). The heating filaments, if present, can provide heat input. Alternatively, the heating filaments can provide structural support for the second elongate member without being energized.

The length of the unheated composite-tube **201** delivery tube **1909** can be greater than the length of a normal unheated extension while still maintaining the same or less amount of heat loss because of the better insulation properties of the composite tube **201.** An increased length of delivery tube **1909** is beneficial to keep the patient's movement from dragging on the tube connections. An increased extension length will also allow for better head movement without compromising patient comfort.

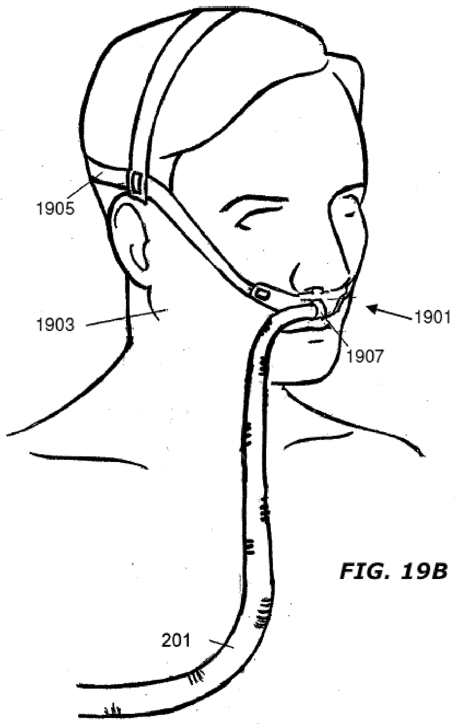

In addition, certain embodiments include the realization that eliminating a separate delivery tube **1909** can have a number of benefits, discussed below. Thus, as shown in FIG. **19B****,** the delivery tube **1909** and composite tube **201** can desirably be a unitary component, which extends to the cannula body **1907.**

In a typical patient interface **1901,** a heated tube (in place of composite tube **201** of FIG. **19A**) supplies inspiratory gases to an unheated delivery tube **1909.** The temperature of the inspiratory gases can experience significant thermal losses (for example, 20°C or more or thereabout) along the length of the unheated delivery tube **1909.** To compensate, the temperature at the patient end of the heated tube is held higher than the required temperature actually delivered to the patient **1901.** In addition, condensation can rain out as the temperature drops in the delivery tube **1909.** It was realized that extending a heated composite tube **201** to the cannula body **1907** in place of the delivery tube **1909,** as shown in FIG. **19B****,** can reduce input energy requirements, as the patient end of the composite tube **201** can be held at a lower temperature. This configuration can also reduce rainout by eliminating the unheated delivery tube **1909** from the patient interface.

Desirably, the composite tube **201** can be tapered. In at least one embodiment, the patient-end portion of the composite tube **201** is tapered to fit the inlet of the cannula body **1907.** In at least one embodiment, the diameter of a length of the composite tube **201** near the patient end is smaller than the diameter of the remainder of the composite tube **201.** For example, the length of the composite tube **201** near the patient end can be in the range of 50 to 300 mm (or about 50 to 300 mm). A smaller diameter tube near the patient end can advantageously reduce tube weight near the cannula body.

The composite tube **201** can comprise a temperature sensor (not shown) near at least the patient end of the composite tube **201.** In addition to, or instead of, a temperature sensor, the composite tube **201** can comprise another kind of sensor (not shown) near at least the patient end of the composite tube **201.** For example, the composite tube **201** can comprise a pressure sensor (not shown) near at least the patient end of the composite tube **201.** Pressure sensors can be particularly advantageous for CPAP control and nasal high-flow therapy. When the composite tube **201** and delivery tube **1909** are a unitary component, the sensor(s) are close to the patient's **1903** nostrils, which can provide more accurate information relating to the delivered gas. Example patient-end sensor configurations are described in greater detail below.

A unitary configuration is also desirable because it can reduce wiring on a patient **1901.** If the cannula body **1907** is equipped with one or more sensors or other electrical components, it is necessary to provide an electrical connection to the cannula body **1907.** If the composite tube **201** and the delivery tube are a unitary component, electrical lines can run along the composite tube **201,** as described above, to the patient end of the composite tube **201** at the cannula body **1907.** No separate electrical connection to the cannula body **1907** is required.

The unitary configuration can incorporate a variable-pitch composite tube **201,** as described above. In a tube that has no or little unheated extension, the heating would continue to the cannula body **1907** where the sensing element would be located. These tubes require reduced end-of-tube temperatures to ensure the delivery of saturated gases at 37 C This is because, ordinarily, the end of tube temperature is set to much higher than 37°C to account for heat loss in the unheated extension. However, a configuration without an unheated extension is more likely to suffer condensation near the unit end. Redistributing the heating to a region proximal to the unit end of the tube will help to boost *T*_{gas} > *T*_{dew}, and hence reduce the occurrence of condensation, without delivering excessively high end of tube temperature.

It should be appreciated that, although the configurations in FIG. **19B** may be preferred in certain embodiments, other configurations, including the configuration of FIG. **19A****,** may be utilized in other embodiments as may be desired.

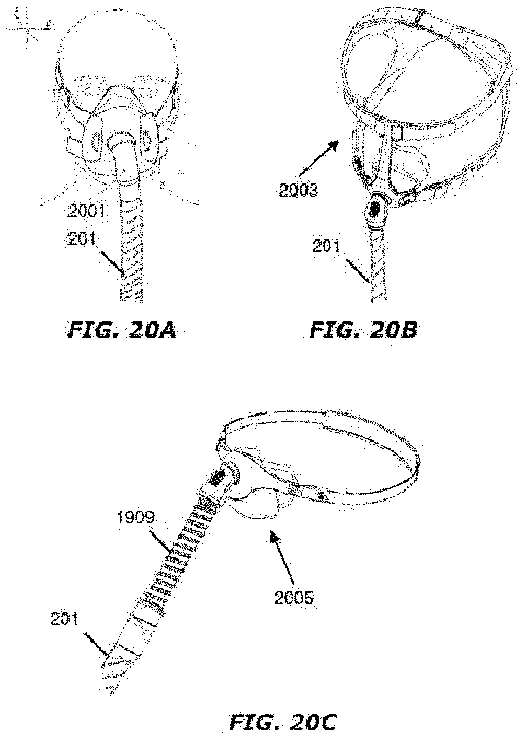

The composite tubes **201** of this disclosure can also be incorporated in and/or used with other patient interfaces, such as a full face mask **2001** (FIG. **20A**), a nasal mask **2003** (FIG. **20B**), and a nasal/pillow mask **2005** (FIG. **20C**). As discussed above, the composite tube **201** can serve as a delivery tube **1909** or eliminate the need for a delivery tube entirely.

### Cleaning

Returning again to FIG. **2A****,** in at least one embodiment, materials for a composite tube can be selected to handle various methods of cleaning. In some embodiments, high level disinfection (around 20 cleaning cycles) can be used to clean the composite tube **201.** During high level disinfection, the composite tube **201** is subject to pasteurization at about 75°C for about 30 minutes. Next, the composite tube **201** is bathed in 2% glutaraldehyde for about 20 minutes. The composite tube **201** is removed from the glutaraldehyde and submerged in 6% hydrogen peroxide for about 30 minutes. Finally, the composite tube **201** is removed from the hydrogen peroxide and bathed in 0.55% orthophthalaldehyde (OPA) for about 10 minutes.

In other embodiments, sterilization (around 20 cycles) can be used to clean the composite tube **201.** First, the composite tube **201** is placed within autoclave steam at about 121 °C for about 30 minutes. Next, the temperature of the autoclave steam is increased to about 134 °C for about 3 minutes. After autoclaving, the composite tube **201** is surrounded by 100% ethylene oxide (ETO) gas. Finally, the composite tube **201** is removed from the ETO gas and submerged in about 2.5% glutaraldehyde for about 10 hours.

The composite tube **201** may be made of materials to withstand the repeated cleaning process. In some embodiments, part or all of the composite tube **201** can be made of, but is not limited to, styrene-ethylene-butene-styrene block thermo plastic elastomers, for example Kraiburg TF6STE. In other embodiments, the composite tube **201** can be made of, but is not limited to, hytrel, urethanes, or silicones.

### Methods Of Manufacture

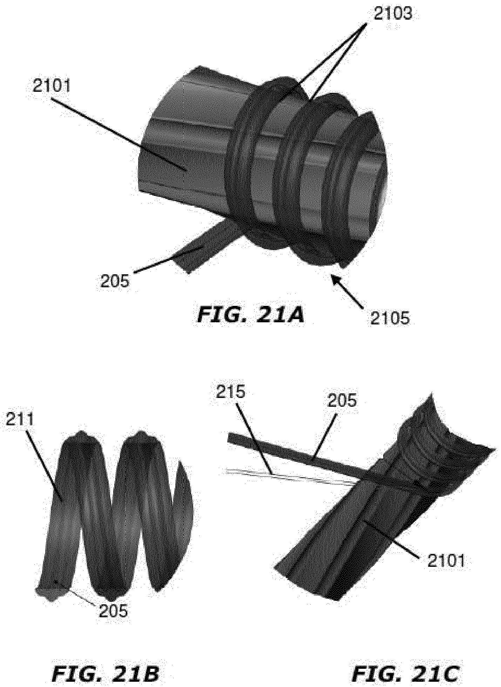

Reference is next made to FIGS. **21A** through **21F** which demonstrate example methods for manufacturing composite tubes.

Turning first to FIG. **21A****,** in at least one embodiment, a method of manufacturing a composite tube comprises providing the second elongate member **205** and spirally wrapping the second elongate member **205** around a mandrel **2101** with opposite side edge portions **2103** of the second elongate member **205** being spaced apart on adjacent wraps, thereby forming a second-elongate-member spiral **2105.** The second elongate member **205** may be directly wrapped around the mandrel in certain embodiments. In other embodiments, a sacrificial layer may be provided over the mandrel.

In at least one embodiment, the method further comprises forming the second elongate member **205.** Extrusion is a suitable method for forming the second elongate member **205.** The second extruder can be configured to extrude the second elongate member **205** with a specified bead height. Thus, in at least one embodiment, the method comprises extruding the second elongate member **205.**

As shown in FIG. **21B****,** extrusion can be advantageous because it can allow heating filaments **215** to be encapsulated in the second elongate member **205** as the second elongate member is formed **205,** for example, using an extruder having a cross-head extrusion die. Thus, in certain embodiments, the method comprises providing one or more heating filaments **215** and encapsulated the heating filaments **215** to form the second elongate member **205.** The method can also comprise providing a second elongate member **205** having one or more heating filaments **215** embedded or encapsulated in the second elongate member **205.**

In at least one embodiment, the method comprises embedding one or more filaments **215** in the second elongate member **205.** For example, as shown in FIG. **21C****,** filaments **215** can be pressed (pulled or mechanically positioned) into the second elongate member **205** to a specified depth. Alternatively, cuts can be made in the second elongate member **205** to a specified depth, and the filaments **215** can be placed into the cuts. Preferably, pressing or cutting is done shortly after the second elongate member **205** is extruded and the second elongate member **205** is soft.

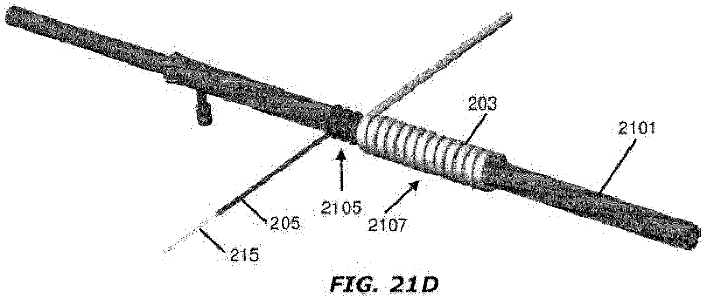

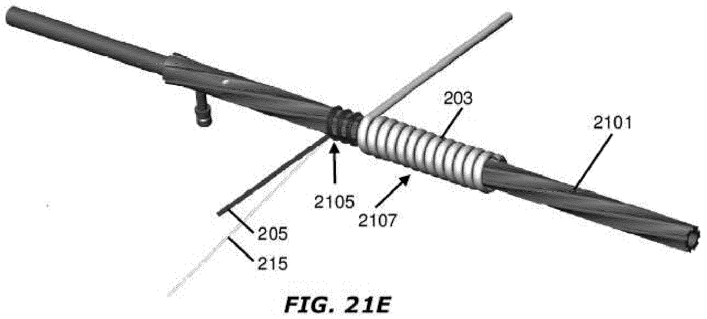

As shown in FIGS. **21D** and **21E****,** in at least one embodiment, the method comprises providing the first elongate member **203** and spirally wrapping the first elongate member **203** around the second-elongate-member spiral **2105,** such that portions of the first elongate member **203** overlap adjacent wraps of the second-elongate-member spiral **205** and a portion of the first elongate member **203** is disposed adjacent the mandrel **2101** in the space between the wraps of the second-elongate-member spiral **2105,** thereby forming a first-elongate-member spiral **2107.** FIG. **21D** shows such an example method, in which heating filaments **215** are encapsulated in the second elongate member **205,** prior to forming the second-elongate-member spiral. FIG. **21E** shows such an example method, in which heating filaments **215** are embedded in the second elongate member **205,** as the second-elongate-member spiral **2105** is formed. An alternative method of incorporating filaments **215** into the composite tube comprises encapsulating one or more filaments **215** between the first elongate member **203** and the second elongate member **205** at a region where the first elongate member **203** overlaps the second elongate member **205.**

As discussed above, at least one embodiment comprises a tube having multiple wraps of the first elongate member **203** between wraps of the second elongate member **205.** Accordingly, in certain embodiments, the method comprises providing the first elongate member **203** and spirally wrapping the first elongate member **203** around the second-elongate-member spiral **2105,** such that a first side portion of the first elongate member **203** overlaps a wrap of the second-elongate-member spiral **2105** and a second side portion of the first elongate member **203** contacts an adjacent side portion of the first elongate member **203.** A portion of the first elongate member **203** is disposed adjacent the mandrel **2101** in the space between the wraps of the second-elongate-member spiral **2105,** thereby forming a first-elongate-member spiral **2107** comprising multiple wraps of the first elongate member **203** between wraps of the second elongate member **205.**

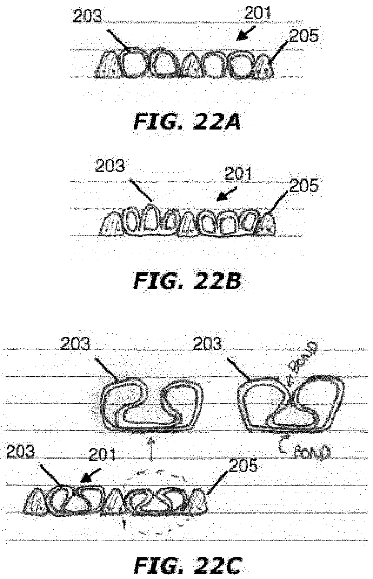

In at least one embodiment, the first elongate member **203** is wrapped multiple times between winds of the second elongate member **205.** An example schematic of the resulting longitudinal cross-section is shown in FIG. **22A****.** Adjacent wraps of the first elongate member **203** can be fused using any suitable technique, such as heat fusing, adhesive, or other attachment mechanism. In at least one embodiment, adjacent molten or softened bubbles can be touched together and thereby bonded while hot and subsequently cooled with an air jet. Adjacent wraps of the first elongate member **203** can also be joined by winding them on the mandrel in a softened state and allowing them to cool.

In at least one embodiment, the first elongate member **203** is wrapped a single time or multiple times between winds of the second elongate member **205,** and the bubble or bubbles between winds of the second elongate member **205** are further collapsed into additional discrete bubbles using an appropriate technique such as a heat treatment. An example schematic of the resulting longitudinal cross-section is shown in FIG. **22B****.** As shown in FIG. **22B****,** one bubble of the first elongate member **203** can be collapsed into two or three or more discrete bubbles using any suitable technique, such as application of a mechanical force with an object or application of a force with a directed air jet. Another example schematic of a resulting longitudinal cross-section is shown in FIG. **22C****.** In this example, a center portion of a bubble is collapsed such that the top of the bubble is bonded to the bottom of the bubble to form two discrete bubbles separated by a flat bottom portion. Then, adjacent side portions of the two discrete bubbles are bonded to form a structure comprising three discrete bubbles.

The above-described alternatives for incorporating one or more heating filaments 215 into a composite tube have advantages over the alternative of having heating filaments in the gas path. Having the heating filament(s) **215** out of the gas path improves performance because the filaments heat the tube wall where the condensation is most likely to form. This configuration reduces fire risk in high oxygen environments by moving the heating filament out of the gas path. This feature also reduces performance as it reduces the heating wires effectiveness at heating the gases that are passing through the tube. Nevertheless, in certain embodiments, a composite tube **201** comprises one or more heating filaments **215** placed within the gas path. For example, heating filaments can be emplaced on the lumen wall (tube bore), for example, in a spiral configuration. An example method for disposing one or more heating filaments **215** on the lumen wall comprises bonding, embedding, or otherwise forming a heating filament on a surface of the second elongate member **205** that, when assembled, forms the lumen wall. Thus, in certain embodiments, the method comprises disposing one or more heating filaments **215** on the lumen wall.

Regardless of whether the heating filaments **215** are embedded or encapsulated on the second elongate member **205** or disposed on the second elongate member **205,** or otherwise placed in or on the tube, in at least one embodiment, pairs of filaments can be formed into a connecting loop at one end of the composite tube to form a circuit.

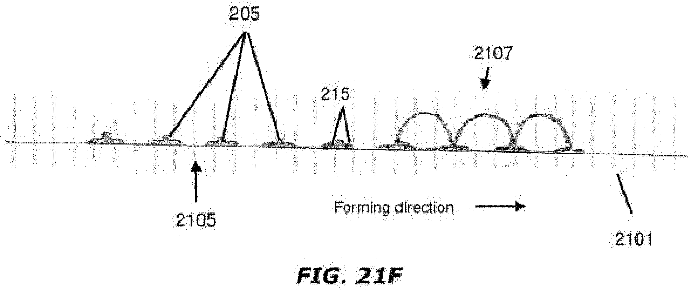

FIG. **21F** shows a longitudinal cross-section of the assembly shown in FIG. **21E****,** focusing on a top portion of the mandrel **2101** and a top portion of the first-elongate-member spiral **2107** and second-elongate-member spiral **2105.** This example shows the second-elongate-member spiral **2105** having a T-shaped second elongate member **205.** As the second-elongate member is formed, heating filaments **215** are embedded in the second elongate member **205.** The right side of FIG. **21F** shows the bubble-shaped profile of the first-elongate-member spiral, as described above.

The method can also comprise forming the first elongate member **203.** Extrusion is a suitable method for forming the first elongate member **203.** Thus, in at least one embodiment, the method comprises extruding the first elongate member **203.** The first elongate member **203** can also be manufactured by extruding two or more portions and joining them to form a single piece. As another alternative, the first elongate member **203** can also be manufactured by extruding sections that produce a hollow shape when formed or bonded adjacently on a spiral-tube forming process.

The method can also comprise supplying a gas at a pressure greater than atmospheric pressure to an end of the first elongate member **203.** The gas can be air, for example. Other gases can also be used, as explained above. Supplying a gas to an end of the first elongate member **203** can help maintain an open, hollow body shape as the first elongate member **203** is wrapped around the mandrel **2101.** The gas can be supplied before the first elongate member **203** is wrapped around the mandrel **2101,** while the first elongate member **203** is wrapped around the mandrel **2101,** or after the first elongate member **203** is wrapped around the mandrel **2101.** For instance, an extruder with an extrusion die head/tip combination can supply or feed air into the hollow cavity of the first elongate member **203** as the first elongate member **203** is extruded. Thus, in at least one embodiment, the method comprises extruding the first elongate member **203** and supplying a gas at a pressure greater than atmospheric pressure to an end of the first elongate member **203** after extrusion. A pressure of 15 to 30 cm H₂O (or about 15 to 30 cm H₂O) has been found to be suitable.

In at least one embodiment, the first elongate member **203** and the second elongate member **205** are spirally wound about the mandrel **2101.** For example, the first elongate member **203** and second elongate member **205** may come out of an extrusion die at an elevated temperature of 200°C (or about 200°C) or more and then be applied to the mandrel after a short distance. Preferably, the mandrel is cooled using a water jacket, chiller, and/or other suitable cooling method to a temperature of 20 °C (or about 20 °C) or less, e.g., approaching 0 °C (or about 0 °C). After 5 (or about 5) spiral wraps, the first elongate member **203** and second elongate member **205** are further cooled by a cooling fluid (liquid or gas). In one embodiment, the cooling fluid is air emitted from a ring with jets encircling the mandrel. After cooling and removing the components from the mandrel, a composite tube is formed having a lumen extending along a longitudinal axis and a hollow space surrounding the lumen. In such an embodiment, no adhesive or other attachment mechanism is needed to connect the first and second elongate members. Other embodiments may utilize an adhesive or other attachment mechanism to bond or otherwise connect the two members. In another embodiment, the second elongate member **205** after extrusion and placement of the heating filaments may be cooled to freeze the location of the heating filaments. The second elongate member **205** may then be re-heated when applied to the mandrel to improve bonding. Example methods for re-heating include using spot-heating devices, heated rollers, etc.

The method can also comprise formed pairs of heating or sensing filaments into a connecting loop at one end of the composite tube. For example, end sections of two heating or sensing filaments can be extricated from the second elongate member **205** and then formed into a connecting loop e.g., by tying, bonding, soldering, adhering, fusing, etc. the two filaments together. As another example, end sections of the heating filaments can be left free from the second elongate member **205** during the manufacturing process and then formed into a connecting loop when the composite tube is assembled.

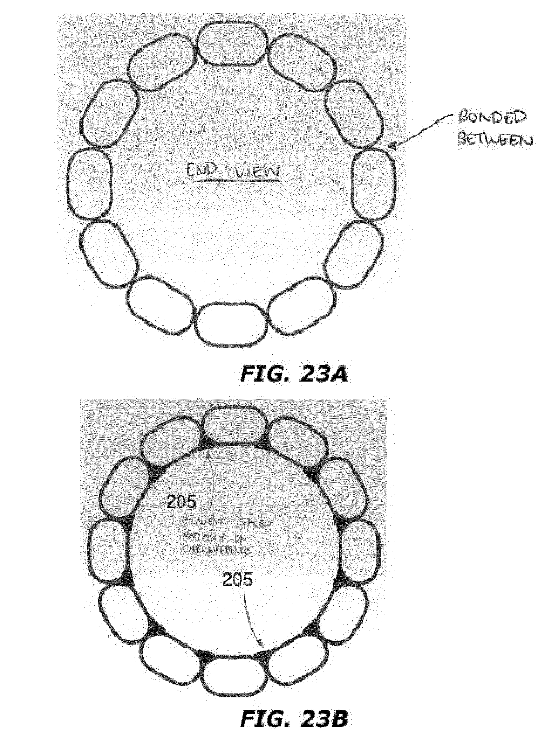

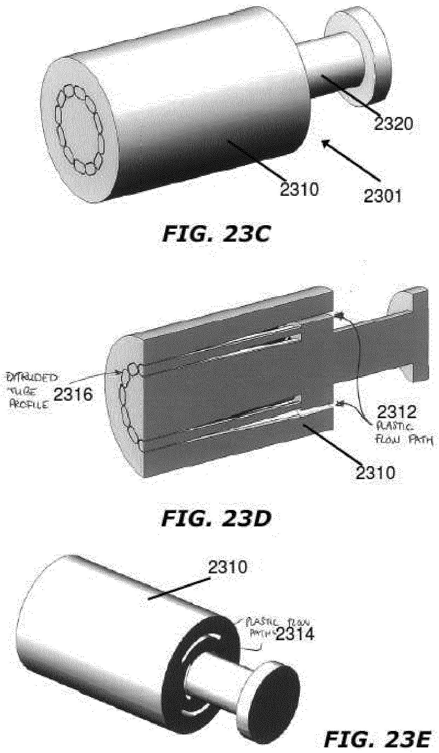

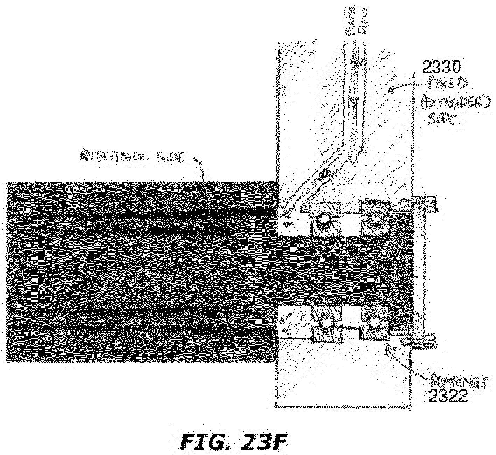

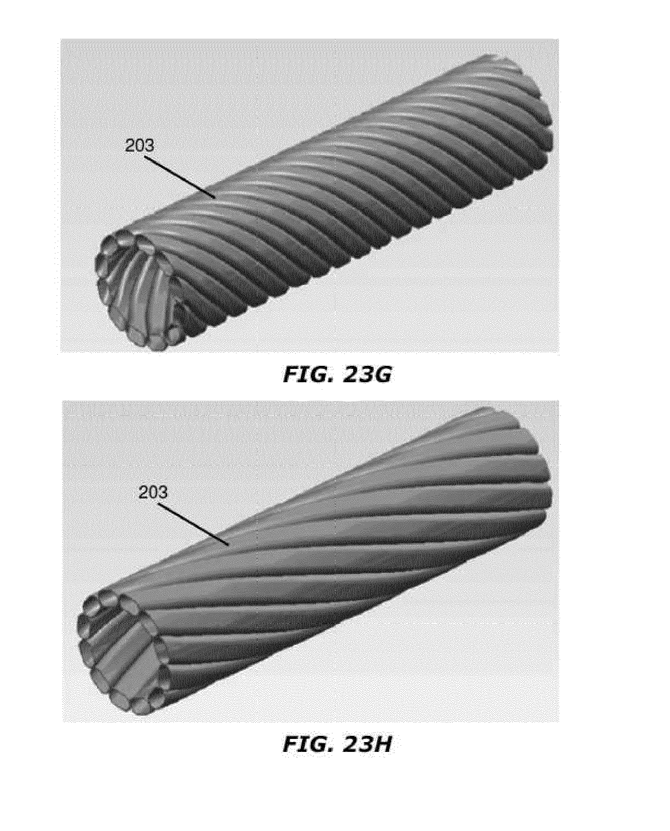

With reference now to FIGS. **23A-23H****,** an alternative method of forming a tube **201** involves an extrusion tool **2301** having a series of flow paths running therealong. The extrusion tool **2301** can be used to form tubes such as the example tubes shown in FIGS. **23G** and **23H****.** As shown, tubes produced using the extrusion tool **2301** can include a plurality of first elongate members **203** extending generally along the longitudinal axis of the tube. In some embodiments, the extrusion tool **2301** includes a body **2310** and a central extension **2320.** In some embodiments, the body **2310** and extension **2320** are generally cylindrical. The body **2310** can include one or more flow paths **2312** that allow for the passage of a molten plastic or another material through the body **2310** from an input end **2314** to an output or extrusion end **2316.** In some embodiments, the flow paths have a substantially conical longitudinal cross-section (that is, are wider where the molten plastic first enters at the input **2314** and narrower near the extrusion end **2316**). The flow paths can have various configurations to produce tubes **201** having various profiles. For example, the flow path configuration shown at the output or extrusion end **2316** in FIGS. **23C** and **23D** can produce a tube **201** having an end view profile as shown in FIG. **23A****.** FIG. **21B** shows an end view of the tube of FIG. **23A** including second elongate members **205,** which may include heating filaments **215,** disposed between adjacent bubbles or first elongate members **203.** In use, the tool **2301** is adapted to rotate so as to induce the tube **201** to be helically formed. As shown in FIG. **23F****,** the central extension **2320** can couple the extrusion tool **2301** to an extruder **2330.** Bearings **2322** disposed between the central extension **2320** and the extruder **2330** can allow the central extension **2320** and body **2310** to rotate relative to the extruder **2330.** The rate of rotation of the tool **2301** can be adjusted to change the pitch or helix angle of the first elongate members **203.** For example, a faster rate of rotation can produce a smaller helix angle, as shown in FIG. **23G****.** A slower rate of rotation can produce a larger helix angle, as shown in FIG. **23H****.**

As discussed above with reference to FIG. **8A** and **8B****,** certain embodiments can comprise a composite tube with a variable pitch. When manufacturing such embodiments, a mandrel **2101** and control system is preferably provided that can alter the effective pitch of the first elongate member **203** and second elongate member **205** (that is, the "ropes"). This can be achieved, for example, by controlling the ratio of rope speed to mandrel **2101** precession rate while maintaining a constant tangential speed at the critical dimension, that is, the pitch center diameter of the ropes. Pitch center diameter determines the pitch center going through the middle of the ropes. This value depends on speed. It is also predictable, so if the pitch center diameter is different from expected, speed can be adjusted to bring the pitch center diameter to the expected value. Altering the effective pitch can also be achieved, for example, by controlling the ratio of rope speed to mandrel **2101** precession while maintaining a constant rotational rate for the spiral composite tube **201** so formed. By controlling the rope speed, any changes in extrudate output are compensated for.

Yet another approach for manufacturing a variable-pitch composite tube **201** uses an integrated system in which extrusion rate and mandrel **2101** precession rate are altered in unison. For example, in this mode, the rope speed may stay the same, but the precession of the mandrel **2101** when enabled will require a slowdown in extrusion rate to match the extrudate output with the tangential speed of the spiral tube **201** so formed.

Still another approach for manufacturing a variable-pitch composite tube **201** moves the incident angle of the second elongate member **205** and first elongate member **203** to alter the pitch of the tube **201.** In these embodiments, the extruders can be on a slide way, which will allow a change in angle, such as a rotary table where the center of rotation is where the second elongate member **205** and first elongate member **203** meet the mandrel **2101.** This method can allow up to 3-5 mm (or about 3-5 mm) of variation in pitch.

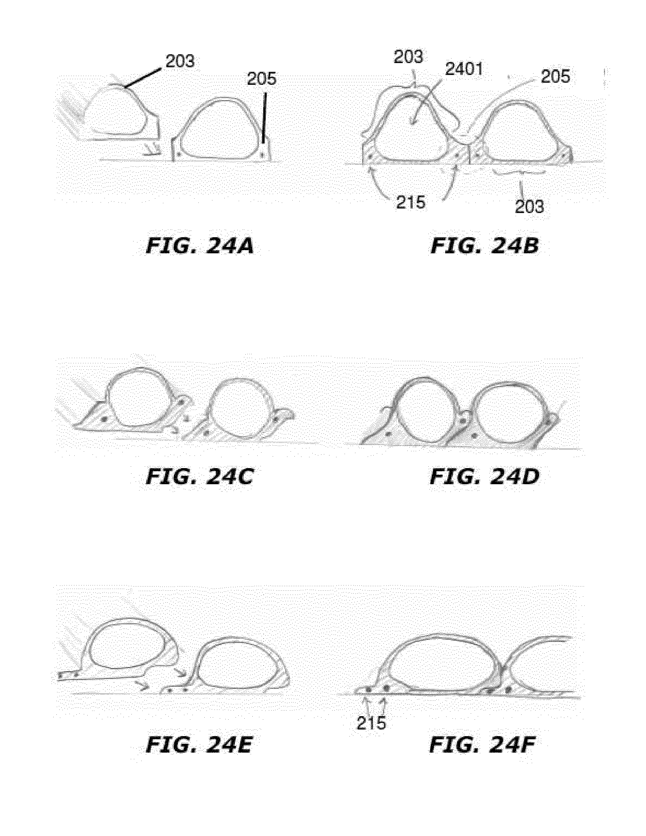

Reference is next made to FIG. **24A** through **24F** which show transverse cross-sections of tubes comprising a single tube-shaped element having a first elongate member or portion **203** and a second elongate member or portion **205.** As illustrated, the second elongate portions **205** are integral with the first elongate portions **203,** and extend along the entire length of the single tube-shaped element. In the embodiments illustrated, the single tube-shaped element is an elongate hollow body having in transverse cross-section a relatively thin wall defining in part the hollow portion **2201,** with two reinforcement portions **205** with a relatively greater thickness or relatively greater rigidity on opposite sides of the elongate hollow body adjacent the relatively thin wall. These reinforcement portions form a portion of the inner wall of the lumen **207** after the elongate hollow body is spirally wound, such that these reinforcement portions are also spirally positioned between adjacent turns of the elongate hollow body.

In at least one embodiment, the method comprises forming an elongate hollow body comprising the first elongate portion **203** and the reinforcement portion **205.** Extrusion is a suitable method for forming the elongate hollow body. Suitable cross-sectional shapes for the tube-shaped element are shown in FIG. **24A** through **24F****.**

The elongate hollow body can be formed into a medical tube, as explained above, and the foregoing discussion is incorporated by this reference. For example, in at least one embodiment, a method of manufacturing a medical tube comprises spirally wrapping or winding the elongate hollow body around a mandrel. This may be done at an elevated temperature, such that the elongate hollow body is cooled after being spirally wound to join adjacent turns together As shown in FIG. **24B****,** opposite side edge portions of the reinforcement portions **205** can touch on adjacent turns. In other embodiments, opposite side edge portions of the second elongate member **205** can overlap on adjacent turns, as shown in FIGS. **24D** and **24E****.** Heating filaments **215** can be incorporated into the second elongate member as explained above and as shown in FIG. **24A** through **24F****.** For example, heating filaments may be provided on opposite sides of the elongate hollow body such as shown in FIGS. **24A-24D****.** Alternatively, heating filaments may be provided on only one side of the elongate hollow body, such as shown in FIGS. **24E-24F****.** Any of these embodiments could also incorporate the presence of sensing filaments.

### Placement of Chamber-End Connector with Electrical Connectivity

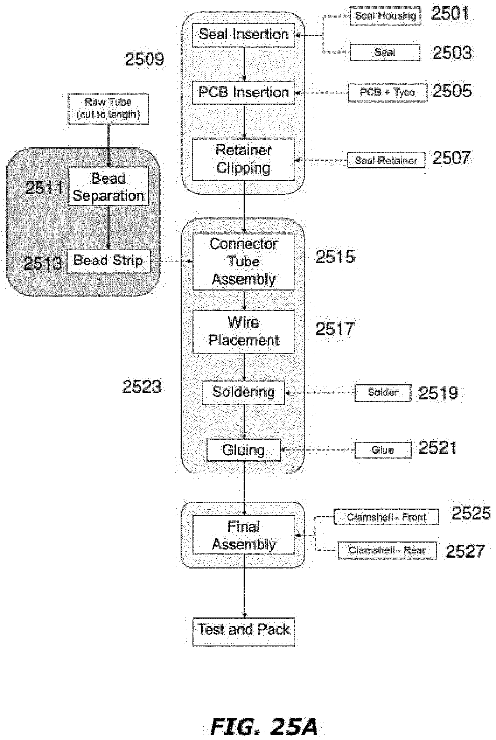

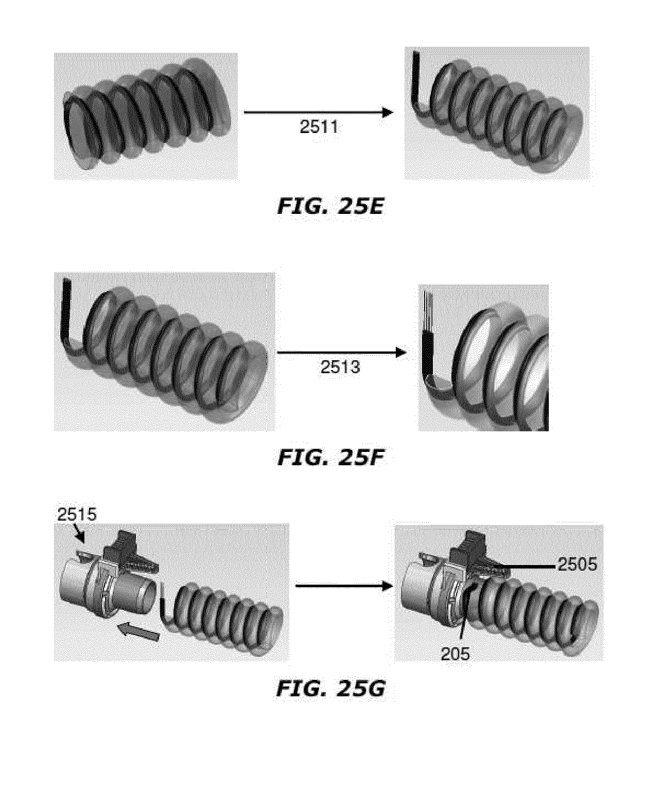

Reference is next made to FIG. **25A****,** which shows an example flow chart for attaching a connector to the end of the tube that is configured in use to connect to a humidifier. For example, as described above with reference to FIG. **1****,** inlet **109** of the inspiratory tube **103** connects to humidifier **107** via port **111.** The example flow chart of FIG. **25A** can make an inlet **109** capable of physically and electrically connecting to the humidifier **107.**

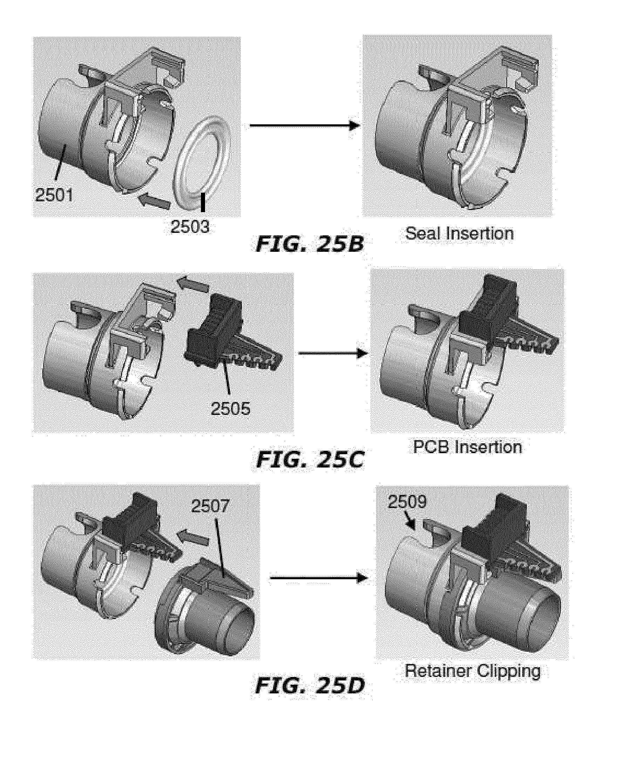

In this example, a seal **2503** is inserted into a seal housing **2501.** The act of seal insertion is also shown in greater detail in FIG. **25B****.** The seal housing **2501** is made of a molded plastic. One open end is sized and configured for connecting to a humidifier. The seal **2503** can be an o-ring, as shown in FIG. **25B****.** A suitable configuration for the o-ring can be a double-toric configuration comprising thicker concentric toruses connected by a thinner web. In this example, the o-ring is molded from a single elastomeric material, such as rubber or silicone. The seal **2503** is seated in a compliant ridge in the seal housing **2501.** The seal **2503** is designed to seal against an outer surface of the port of the humidifier chamber. The seal **2503** can deflect to extend along the outer surface of the port. In other words, the double o-ring configuration includes an inner O-ring and an outer O-ring connected by a flange. The outer O-ring will be sealed within the connector while the inner O-ring can deflect along the flange portion and squeeze against the outer surface of the port. In such a position, a horizontal plane extending through a center axis of the inner O-ring may be in a different plane than a horizontal plane extending through a center axis of the outer O-ring.

Turning again to the example of FIG. **25A****,** a printed circuit board (PCB) is inserted into a compliant dock on the seal housing **2501.** The act of PCB insertion is shown in greater detail in FIG. **25C****.** In FIG. **25C****,** an assembly **2505** comprising a PCB and a PCB electrical connector is inserted into a compliant dock on the seal housing **2501.** A variety of PCBs of suitable size and configuration can be used. A variety of PCB electrical connectors can also be used. For example, the PCB electrical connector can be a straight-through connector or a bi-directional connector. The PCB comprises four connection pads suitable for receiving four conductive filaments encased in the second elongate member of the tube. However, the PCB can be configured to receive a suitable number of conductive filaments, if the second elongate member contains more or fewer than four conductive filaments.

Turning again to the example of FIG. **25A****,** and as shown in greater detail in FIG. **25D****,** a seal retainer **2507** is clipped onto one open end of the seal housing **2501** with the seal **2503** seated on the compliant ridge. Clipping the seal retainer **2507** in place compresses the seal **2503** and thereby forms a liquid- and gas-resistant connection between the seal housing **2501** and the seal retainer **2507.** In this example, the seal retainer **2507** is made from a molded plastic. In this example, the seal retainer **2507** also comprises a protruding portion sized and shaped to fit around the PCB. The protruding portion serves to support and protect the more flexible and fragile PCB. The protruding portion can be omitted in certain embodiments, however. The resulting assembly comprising the seal housing **2501,** seal **2503,** PCB and PCB connector assembly **2505,** and the seal retainer **2507** is referred to herein as a connector tube assembly **2515.**

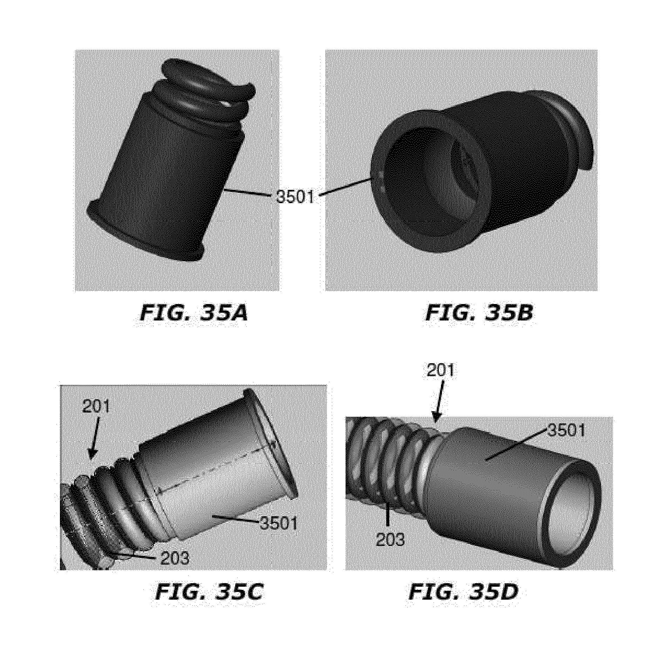

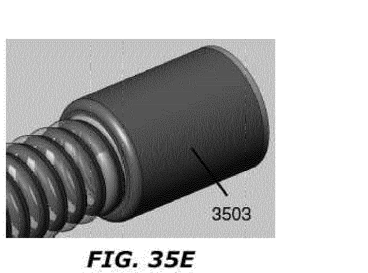

Turning again to the example of FIG. **25A****,** the tube is prepared for connection to the connector tube assembly **2515.** As shown FIG. **25A** and in greater detail in FIG. **25E****,** in step **2511,** a portion of the second elongate member at one end of the tube is separated from the first elongate member. Then, in step **2513,** a length of the separated second elongate member is stripped away to reveal four conductive filaments (or the number of conductive filaments contained in the second elongate member). Step **2513** is shown in greater detail in FIG. **35F.**

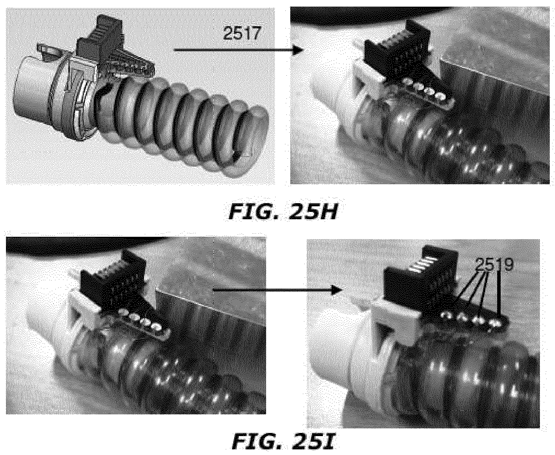

As explained in FIG. **25A** and as shown in greater detail in FIG. **25G****,** the portion of the tube with the stripped length of the second elongate member is inserted in the connector tube assembly **2515.** In FIG. **25G****,** the second elongate member **205** has a bent shape to accommodate the position of the PCB connector assembly **2505.** The PCB connector assembly **2505** can also be sized and positioned to reduce or eliminate the bent shape, for example, by shifting the PCB connector assembly further toward the connector end. As shown in step **2517** of FIG. **25A** and FIG. **25H****,** the four conductive filaments are inserted in the four connection pads of the PCB. Then, as shown in FIGS. **25A** and **25I****,** a bead of solder **2519** is placed over each filament-connection pad connection to secure the filament to the connection pad and ensure a good electrical connection between each filament and its corresponding connection pad.

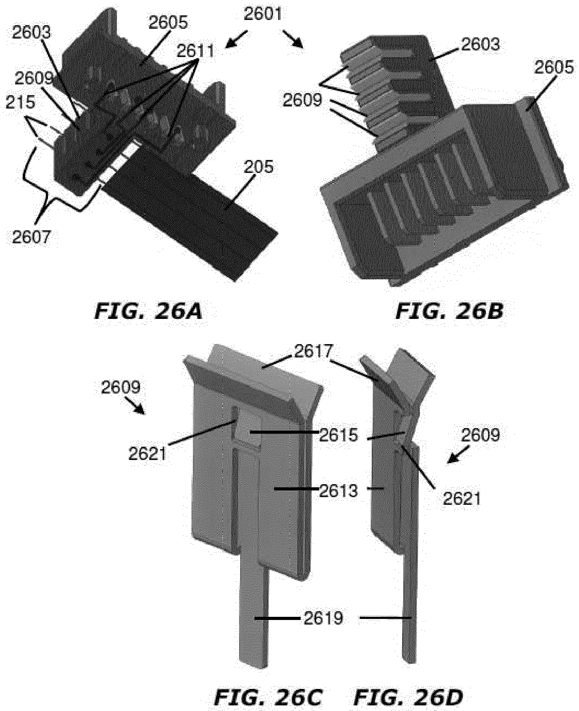

The foregoing step of placing the bead of solder **2519** can be omitted in certain embodiments. FIGS. **26A-26E** show an example connector assembly configuration that does not require soldering to connect the filaments to the connector assembly.

FIG. **26A** shows a connector assembly **2601** comprising a clip housing **2603** and a circuit connector **2605.** A stripped length **2607** of the second elongate member **205** exposes the heating filaments **215** that can be inserted in the clips **2609** in the clip housing **2403.** Each clip **2609** is electrically conductive. Suitable materials for a clip **2609** include, for example, aluminum, copper, and gold. A clip **2609** retains a heating filament **215** without the need for solder. An electrical lead **2611** can run between each clip **2609** and the circuit connector **2605.**

FIG. **26B** shows a top-down view of the connector assembly **2601** showing the clips **2609** positioned in the clip housing **2603.**

FIG. **26C** shows a clip **2609** in greater detail. The clip **2609** comprises a folded portion **2613,** a retention tab portion **2615,** a flanged portion **2617,** and elongated portion **2619.** A heating filament (not shown) is inserted into the flanged portion **2617** so that the folded portion **2613** accepts and retains the heating filament. The shape of the flanged portion **2617** facilitates insertion of the heating filament and guides the heating filament into place. Nevertheless, the flanged portion **2617** can have a straight shape, if desired. The flanged portion **2617** can also have another suitable shape, such as a partial flange. The folded portion has a catch portion **2621** that is compliant with the retention tab portion **2615.** The retention tab portion **2615** is angled so that a heating filament is able to slide past the retention tab portion **2615** in one direction into the folded portion **2613.** The retention tab portion **2615** also catches the heating filament to prevent it from inadvertently falling out of the folded portion **2613.** The elongated portion **2619** is electrically conductive and transmits the electrical current from the heating filament into and/or across the clip housing **2603.**

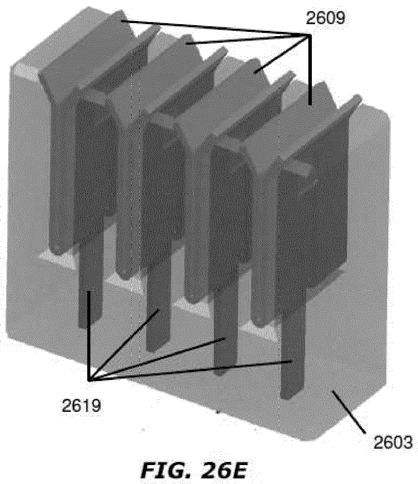

FIG. **26D** is a cross section of the view of FIG. **26C** and demonstrates the position of the tab portion **2615** and the catch portion **2621** in greater detail. FIG. **26E** shows how the clips **2609** are positioned in the clip housing **2603.** The clip housing **2603** is shown transparent to demonstrate the position of the elongated portion **2619.**

Referring again to FIG. **25A****,** to ensure that all pieces of the connector tube assembly **2515** are securely fixed to each other, a layer of glue **2521** is then applied. Glue is a broad term and refers to a material for joining, fixing, or attaching other materials. A glue can be adhesive or sticky to the touch when it is in a liquid or semi-solid state. When the glue has dried or otherwise cured into a solid state, the glue can be adhesive or non-adhesive or non-sticky to the touch. The glue can be a resin, such as an epoxy resin, or an elastomer (thermoset or thermoplastic). Use of TPE materials can be advantageous because they are generally flexible and can accommodate twisting, bending, or pressure without shattering.

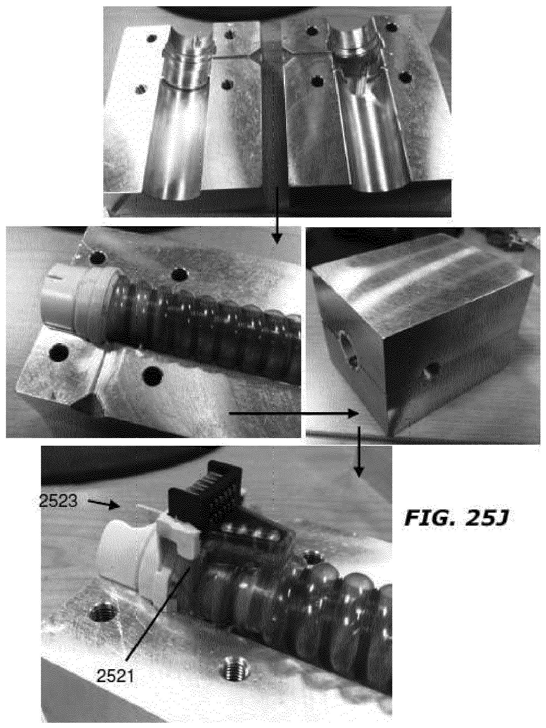

An example method for applying the glue **2521** is shown in FIG. **25J****.** In this method, a two-block mold is provided. In this example, the mold is made of a metal, such as aluminum or stainless steel, however any suitable material can be used. For instance, the mold can be made from Teflon^{®} PTFE blocks. One block is configured to accommodate the protruding PCB and PCB connector assembly **2505** of the connector tube assembly **2515** and the adjacent tube, and the other block is configured to accommodate the opposite portion of the tube and connector tube assembly **2515.** The tube is placed in the compliant mold portions such that the blocks stack one on top of the other. A liquid glue is introduced into an inlet hole in the mold, and the glue is allowed to harden. Then, the mold is removed to expose the glued tube-and-connector assembly **2523,** which includes a layer of hardened glue **2521** covering the PCB and the joint between the tube and the connector tube assembly **2515.** The glue layer can cover the PCB and all of the soldered connections on the PCB. In this manner, the layer of glue can protect the PCB and the connections from corrosion. In other words, the glue serves at least three functions: sealing the connector and the conduit, holding the PCB in place and potting the PCB; the glue layer forms a pneumatic seal, a mechanical bond and a PCB pot. In addition, the glue layer can act as an electrically insulating barrier, for example, by keeping out moisture and liquids from reaching the electrical components and creating a conductive path to a user of the device.

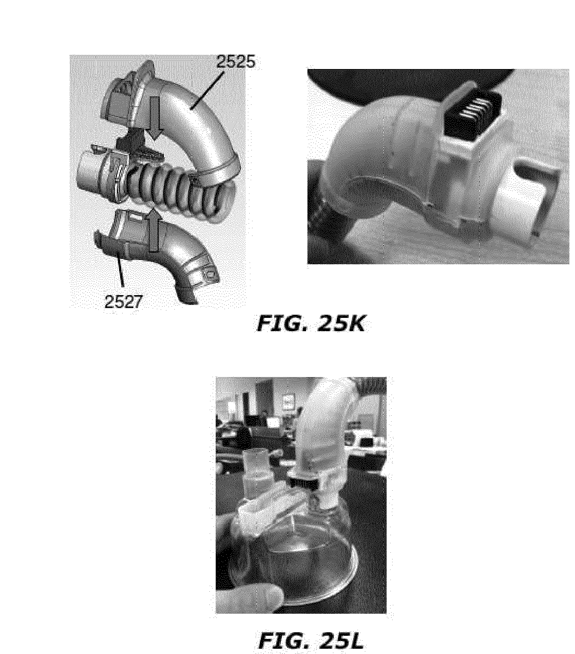

Returning again to FIG. **25A****,** the tube-and-connector assembly **2523** is then in condition for final assembly. As shown in greater detail in FIG. **25K****,** a first clamshell **2525** and a second clamshell **2527** are snapped together around the tube-and-connector assembly **2523** such that a portion of the PCB connector is left exposed. The first clamshell **2523** and second clamshell **2527** shown in FIG. **25K** are top and bottom clamshells, respectively.

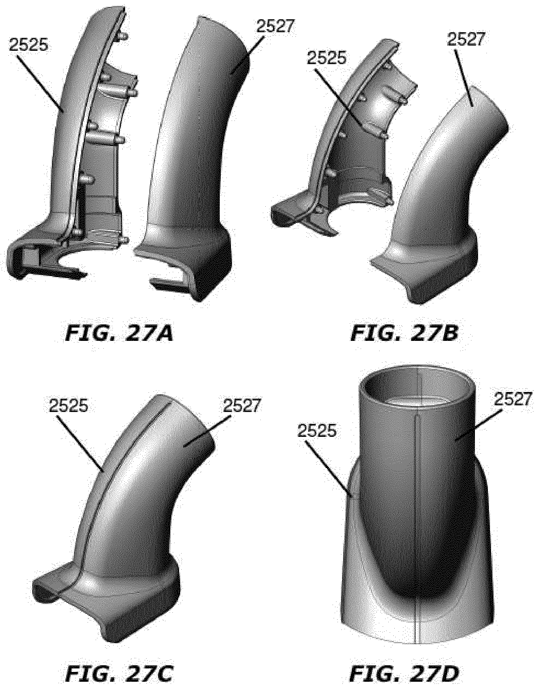

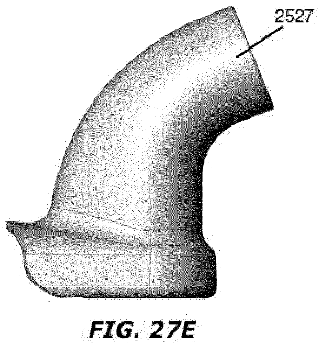

An alternative clamshell design is shown in FIGS. **27A-27E****,** in which the first **2525** and second **2527** clamshells are left and right clamshells, respectively. The clamshell **2525, 2527** portions (FIG. **25K** or FIGS. **27A****-27E**) can be made of molded plastic or any other suitable material. The clamshell **2525, 2527** portions (FIG. **25K** or FIGS. **27A-****27E**) serve to further protect the tube-and-connector assembly **2523** (FIGS. **25A** and **25J**) and to maintain the tube-and-connector assembly in a bent position that promotes the return of condensate to the humidifier unit when in use. As shown in FIG. **25L****,** the final assembly can readily snap into a humidifier with a compliant electrical connector near the connection port.

Although the foregoing manufacturing method has been described with reference to a flow chart, the flow chart merely provides an example method for attaching a connector to the end of the tube that is configured in use to connect to a humidifier. The method described herein does not imply a fixed order to the steps. Nor does it imply that any one step is required to practice the method. Embodiments may be practiced in any order and combination that is practicable.

### Placement of Alternative Device-End Connectors

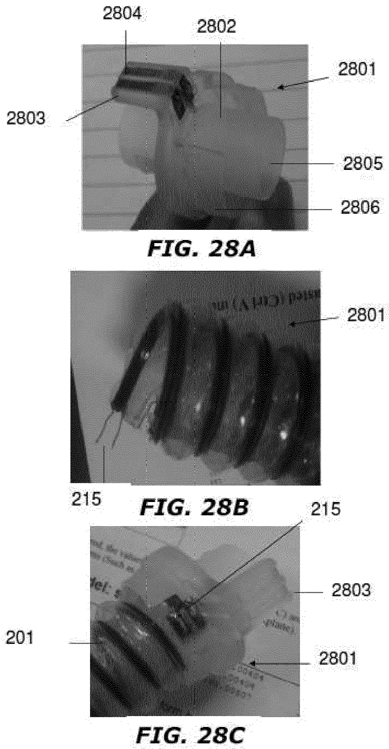

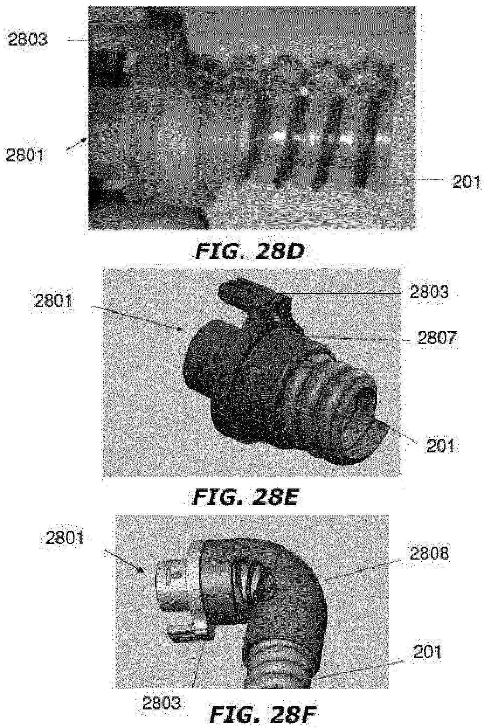

Reference is next made to FIGS. **28A-28F** which show a connector which can be used for medical circuits having electrical wires running therethrough. The connector **2801** comprises a cut-out **2802,** which in certain embodiments is 30 mm (or about 30 mm) across. In certain embodiments, on one end of the cut-out **2802** is a L-shaped arm **2803** which extends in part outward from the connector **2801** and in part parallel to the longitudinal axis of the connector **2801.**

The arm **2803** can have one or more electrical conductors **2804** embedded therein. The conductors **2804** can be made of copper or brass or another suitably conductive material and can be formed as flat L-shaped pieces running substantially along the length of the arm **2803.**

The connector **2801** can further comprise an inner portion **2805** adapted to sit substantially inside a portion of the tube **201** and an outer portion **2806** adapted to substantially surround a portion of the tube **201.**

A portion of the second elongate member **205** is stripped away to reveal the one or more filaments **215** embedded therein. Preferably about 5 mm of the filaments **215** are revealed. The connector **2801** is then attached to the tube **215** such that the inner portion **2805** sits within tube **201** and the outer portion **2806** sits around the tube **201.** Preferably the connector **2801** is oriented such that the revealed ends of the filaments **215** are located at or near the cut-out **2802.**

The revealed ends of the filaments **215** are then electrically and/or physically connected to the conductors **2804.** This can be done by soldering the ends to the conductors **2804,** or any other method known in the art.

A member **2807** can be inserted or molded on top of at least a portion of connector **2801** and, optionally, tube **201** to promote the attachment between the connector **2801** and the tube **201.** The member **2807** can be a hard material or a soft material, such as a soft rubber or elastomer.

In some embodiments, a substantially L-shaped elbow **2808** can be placed over the assembly. The elbow **2808** can provide some additional strength to the connection and can provide a predetermined bend in the tube **201** (such that the connector **2801** can tend to sit at an angle of about 90° from the body of the tube **201**).

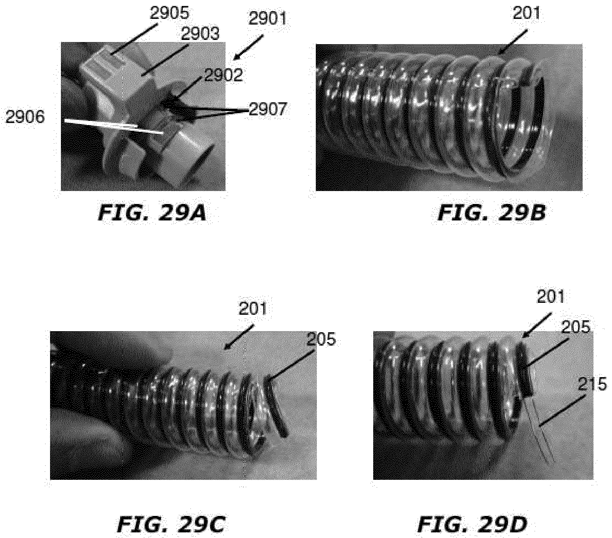

Reference is next made to FIGS. **29A-29L** which shows another connector **2901** which can be used for medical circuits having electrical wires running therethrough. Referring first to FIG. **29A****,** the connector **2901** permits a composite tube to be connected to a device, such as a CPAP device (not shown). The connector **2901** carries an electrical terminal on an L-shaped arm **2903,** which engages a complementary electrical terminal of the device to permit electrical signals or electrical energy to be transmitted between the device and a composite tube. In the illustrated arrangement, the electrical terminal of the connector **2901** is a plug **2905** that complies with a receptacle or port of the device. This arrangement could also be reversed, however, if desired. In this example, the plug electrically communicates with electrical contacts **2906** for establishing an electrical connection with a composite tube. Here, the electrical contacts **2906** are molded into the connector **2901.** The connector **2901** further comprises filament holders **2907** that are also molded into the connector **2901.** The connector **2901** also comprises a cut-out **2902,** which in certain embodiments is 30 mm (or about 30 mm) across.

As shown in FIGS. **29B** and **29C****,** a portion (e.g., a 10-mm portion) of the second elongate member **205** is stripped away to reveal a small length of the one or more filaments **215** embedded therein. Preferably, about 5 mm or 10 mm of the filaments **215** are revealed.

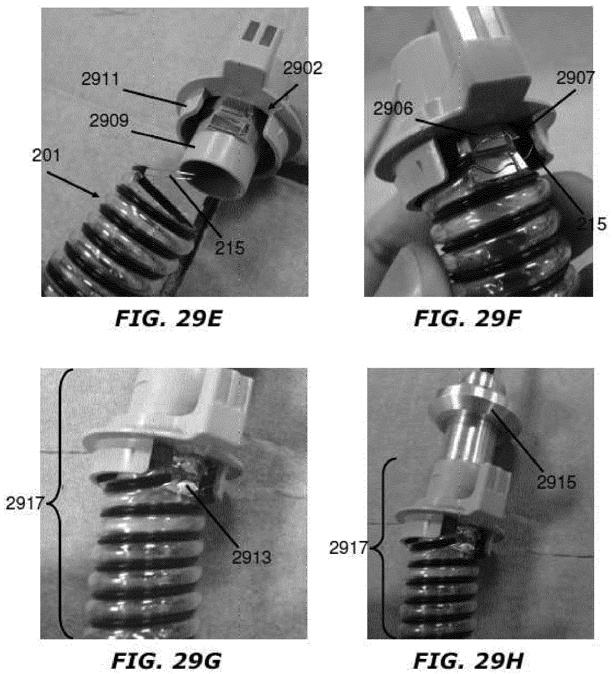

As shown in FIG. **29E****,** the connector **2901** is then attached to the tube **215** such that an inner portion **2909** of the connector **2901** sits within tube **201** and an outer portion **2911** of the connector **2901** sits around the tube **201.** Preferably the connector **2901** and composite tube **201** are oriented such that the revealed ends of the filaments **215** are located at or near the cut-out **2902** and the filaments **215** are aligned to meet near the contacts **2906.**

As shown in FIG. **29F****,** the heating filaments **215** are positioned under the wire holders **2907** so that each heating filament **215** is positioned over a contact **2906.**

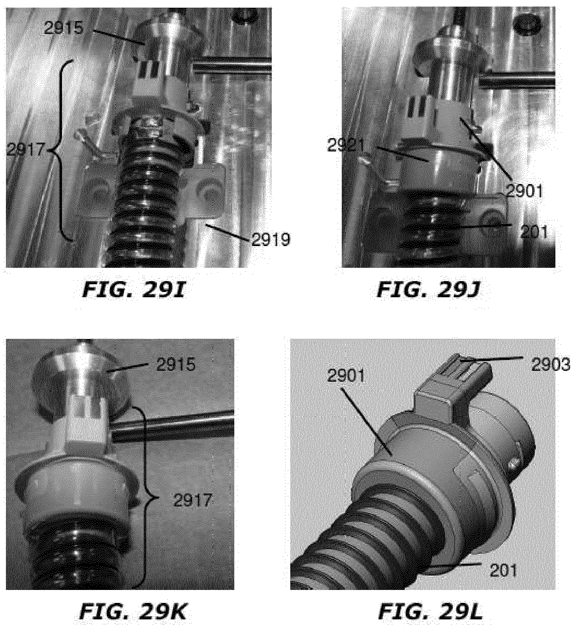

As shown in FIG. **29G****,** a bead of solder **2913** is placed over each heating filament **215** at the respective contact **2906.** The combination of connector **2901** and composite tube **201** is designated here as a connector-tube assembly **2917.** As shown in FIG. **29H****,** a mold tool core **2915** is inserted into the connector **2901.** As shown in FIG. **29I****,** the connector-tube assembly **2917** and core **2915** are placed in an injection mold tool **2919.** In FIG. **29J****,** a molded material **2921** is molded over the cut-out (not shown), thereby bonding the connector **2901** and composite tube **201.** Suitable molded materials **2921** include plastic and rubber. The connector-tube assembly **2917** and core **2915** are removed from the injection mold tool (not shown), as in FIG. **29K****.**

As shown in FIG. **29L****,** the core **2915** is removed, thereby providing a composite tube **201** with a device-end connector **2901.** The method of FIGS. **29A-29J** allows the plug **2903** to be electrically connected to the heating filament and/or other electrical element(s) (not shown) of the composite tube **201.** Preferably, the heating circuit of the device provides electrical energy to the heating filament of the composite tube **201** so that the heating filament can provide heat energy to the flow of humidified air passing through the composite tube **201.** As discussed herein, such an arrangement can prevent or limit condensation within the composite tube **201.** In addition, or in the alternative, the plug **2903** and device port could provide for other electrical signals, such as data signals, to be communicated between the device and the composite tube **201.** For example, a sensor at the patient interface-end of the composite tube **201** could provide data regarding one or more parameters of the flow of air (e.g., temperature, humidity level) for use by the control system of the device. Any other desirable electrical signals could also be transmitted.

The foregoing methods of attaching connectors to composite tubes are provided by way of example. The methods described do not imply a fixed order to the steps. Nor do they imply that any one step is required to practice the methods. Embodiments may be practiced in any order and combination that is practicable.

### Placement of Patient-End Connector with Electrical Connectivity

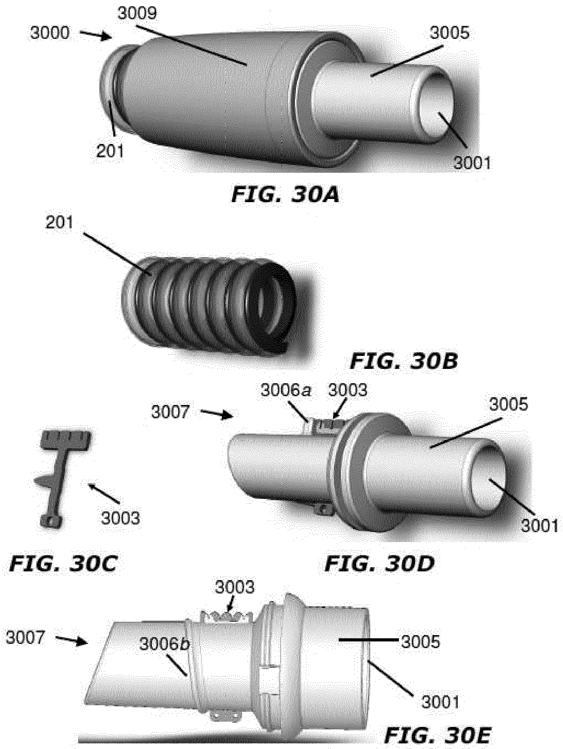

Reference is next made to FIGS. **30A-30O****,** which show an example connector **3000** for connecting one end of the tube **201** to a patient interface (not shown). The end of the connector **3000** that connects to the patient interface is indicated by reference **3001.**

FIG. **30A** shows a side perspective view of the connector **3000.**

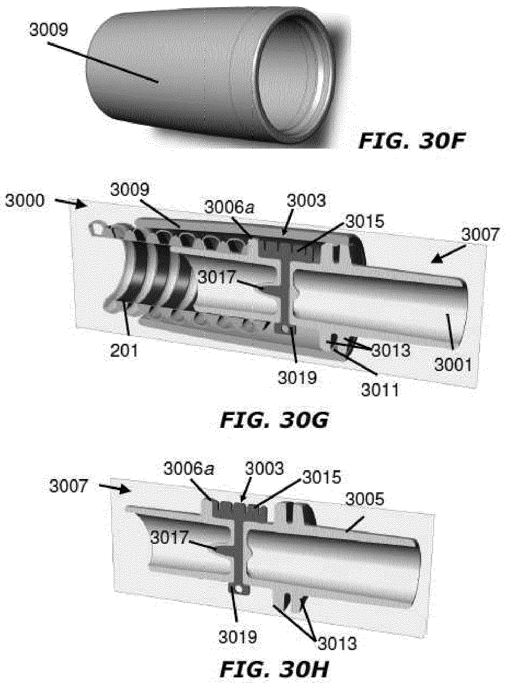

As shown in FIG. **30B-30F****,** the connector **3000** comprises a PCB assembly **3003** and an insert **3005,** designated together as an insert assembly **3007** when assembled together, and a cover **3009.** Each of FIGS. **30B-30D** and **30F** shows a side-perspective view that generally corresponds with the view of FIG. **30A.** FIG. **30E** shows a side-plan view.

The insert **3005** and cover **3009** are preferably molded plastic components. The insert **3005** can serve one or more purposes, including providing a receptor for the tube **201,** providing a suitable conduit for the gas flow path, providing a housing for the PCB assembly **3003,** and providing a housing for a sensor (not shown), such as a thermistor. The cover **3009** protects and covers the relatively fragile PCB assembly **3003** and protects the connection between the tube **201** and the insert **3005.** As shown in FIGS. **30D** and **30E****,** the end of the insert **3005** that is inserted in the tube **201** (that is, the end that is opposite end **3001**) can be angled, which can aid insertion into the tube **201.** In some embodiments, however, the end that is opposite end **3001** can be blunt or tapered.

As shown in FIG. **30D****,** the insert desirably includes a stop portion **3006*a*.** The stop portion **3006*a*** can promote correct placement of the tube **201** with respect to the insert **3005.** The stop portion **3006*a*** can also serve to protect the PCB assembly **3003** from directly contacting the tube **201.** An alternative configuration is shown in FIG. **30E****.** In FIG. **30E****,** stop portion **3006*b*** is formed as a spiral or helical component, such as a spiral or helical rib. This configuration is advantageous because the shape complements the spirally wound tube **201,** thereby providing a secure connection between insert **3005** and tube **201.**

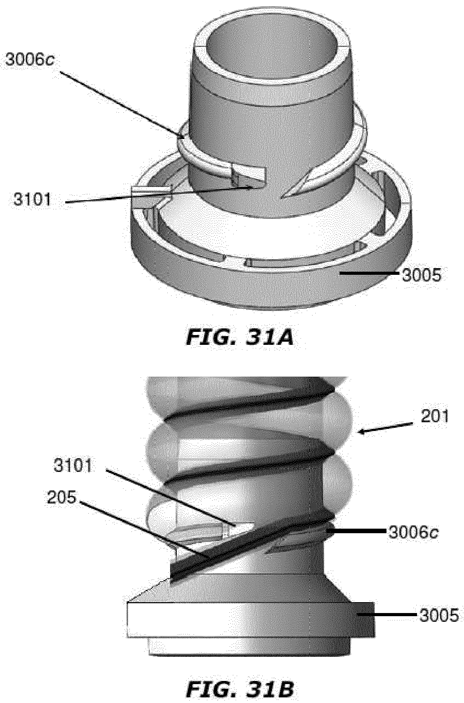

Yet another alternative configuration is shown in FIGS. **31A** and **31B****.** In these figures, stop portion **3006*c*** is formed as a spiral or helical component, such as a spiral or helical rib. Again, this configuration is advantageous because the shape complements the spirally wound tube **201** (FIG. **31B**), thereby providing a secure connection between insert **3005** and tube **201.** In this configuration, the stop portion **3006*c*** comprises an orientational stopping feature **3101.** As shown in FIG. **31B****,** a surface of the orientation stopping feature **3101** is tapered such that the orientational stopping feature **3101** resembles a fin The shape of the orientational stopping feature **3101** can pinch, grasp, or otherwise retain the second elongate member **205** of the tube **201.** The orientational stopping feature **3101** thus can serve to better hold the tube **201** in the correct position by preventing the tube **201** from shifting and/or turning.

Returning to FIG. **30E****,** the patient end **3001** of the insert **3005** is larger than it is in FIG. **30D** and illustrates how the size can be modified for different applications (for example, connecting to infant or adult patient interfaces).

FIG. **30G** shows a cross section of the connector **3000** and generally corresponds with the same side perspective view as FIG **30A****.** In certain embodiments, there is an insulating gap, such as an air gap, between the tube **201** and the insert **3005** to protect the sensor (discussed below) from heat radiating from the heating filament(s) in the tube **201,** which can induce sensor error at low flows. In FIG. **30G****,** such a gap would appear above and below sensor portion **3017.** Alternative, in certain embodiments, the insert **3005** is formed such that air bubbles are encapsulated in the insert **3005.** For example, the insert **3005** can comprise a foamed plastic.

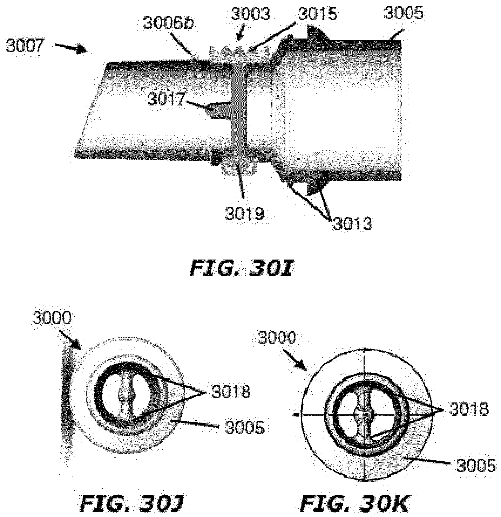

FIG. **30H** shows a cross section of the insert assembly **3007** and generally corresponds with the side perspective view of FIG. **30D****.** FIG. **30I** shows an alternative cross section of the insert assembly **3007** and generally corresponds with the side plan view of FIG. **30E****.** These figures show greater details regarding the relative placement of the tube **201,** insert assembly **3007,** and/or cover **3009.**

As shown in FIGS. **30G-30I****,** a generally annular catch structure **3013,** which comprises two molded rings extending radially outward from the body of the insert **3005.** The molded rings comply with a notch **3011,** which comprises a molded ring extending radially inward from the cover **3009.** The notch **3011** and catch structure **3013** hold the cover **3009** on the insert **3005.**

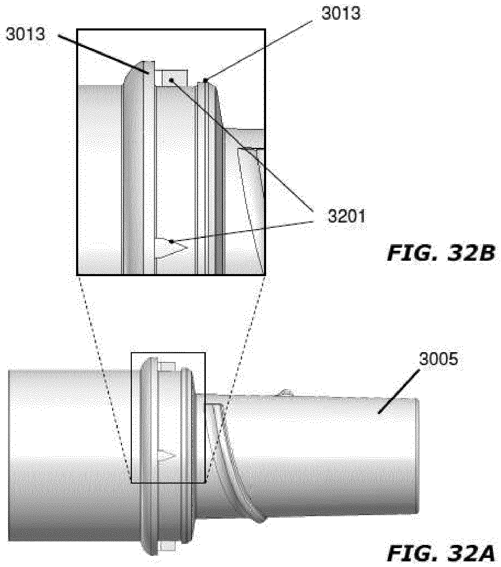

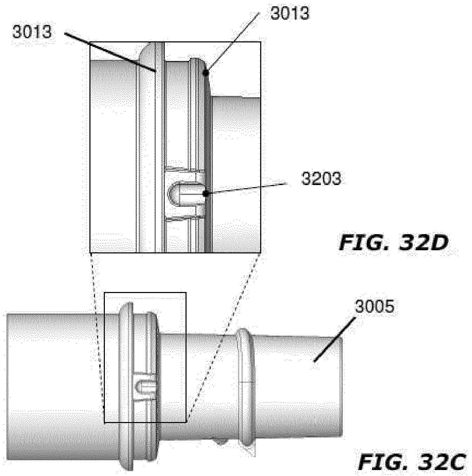

An alternative configuration for the catch structure **3013** is shown in FIGS. **32A** and **32B****.** Again, the catch structure **3013** is generally annular and comprises two molded rings extending radially outward from the body of the insert **3005.** A plurality of anti-rotation projections **3201** extends perpendicularly between the rings. In this example, there are four projections **3201** evenly spaced around the circumference of the catch structure **3013** (e.g., at 90° intervals). The projections **3201** engage compliant notches in the cover (not shown) and prevent the cover from rotating on the insert assembly. Yet another alternative configuration for the catch structure **3013** is shown in FIGS. **32C-32D****.** Again, the catch structure **3013** is generally annular and comprises two molded rings extending radially outward from the body of the insert **3005.** Anti-rotation notches **3203** are disposed between the rings. In this example, there are four notches **3203** evenly spaced around the circumference of the catch structure **3013** (e.g., at 90° intervals). These notches **3203** engage compliant projections in the cover (not shown) and prevent the cover from rotating on the insert assembly.

FIGS. **30G-30I** further illustrate that PCB assembly **3003** comprises a PCB **3015,** a sensor portion **3017,** and a locating portion **3019.** The PCB assembly **3003** is located such that, in use, the sensor portion **3017** is within the fluid flow path through the insert **3005.**

The sensor portion **3017** includes one or more sensors, such as a temperature sensor. The sensor is preferably located on the projecting portion of the sensor portion **3017.** A suitable temperature sensor includes a thermistor, thermocouple, resistance temperature detector, or bi-metallic thermometer.

The PCB **3015** completes the heating and/or sensing circuit of the composite tube **201.**

The locating portion **3019** improves stability and facilitates locating the PCB assembly **3003** during manufacturing. Nevertheless, the locating portion **3019** can be omitted in certain embodiments.

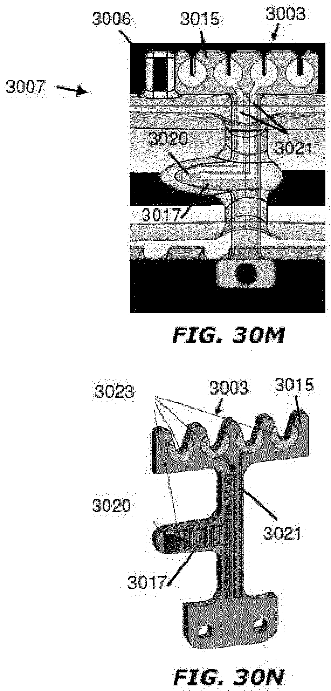

FIG. **30I** also illustrates that the PCB assembly **3003** can be further stabilized in the insert **3005** by recessing at least part of the PCB **3015** and/or the locating portion **3019** in the outer surface of the insert **3005.** The recessed configuration is also shown in FIG. **30N****.**

The configurations of FIGS. **30G-30I** have a number of advantages. For example, certain embodiments include the realization that placing the sensor portion **3017** within the fluid flow path promotes accurate measurements, regardless of flow rate, ambient temperatures, and so forth. In addition, certain embodiments include the realization that there is less potential for fluid leak than a configuration having a separate sensor attached to a connector, due to poor user setup of the circuit.

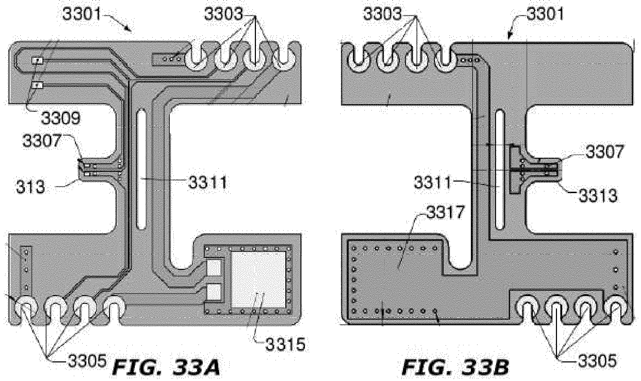

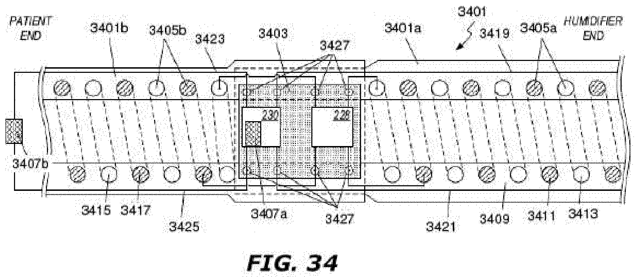

Further, certain embodiments include the realization that, because the PCB assembly **3003** passes across the full width of the insert **3005,** the PCB assembly **3003** can be used to pass connection leads across a tube **201.** As discussed below, FIGS. **33A-33D** illustrate a PCB assembly **3301** design capable of passing connection leads across a tube, the respective figures illustrating two sides of the PCB assembly **3301.** The concept of passing connection leads across a tube **201** is further discussed below with reference to FIG. **34****,** in the context of an intermediate connector between two tube **201** segments.

Turning first to FIGS. **33A** and **33B****,** the PCB assembly **3301** includes connection pads **3303, 3305** for the heating filament and/or sensor connections. The connection pads **3303, 3305** are configured to be on opposite sides of the PCB assembly **3303** to facilitate connections with spirally-wound heating filaments.

The PCB assembly **3301** includes sensor connection pads **3307** for the sensor. The sensor can be coupled to a diode through signal connection pads **3309** on the PCB assembly **3301.** As illustrated, the PCB assembly **3301** includes a gap **3311** configured to thermally insulate the sensor from the other electrical components and tracks. In some embodiments, the gap **3311** can be filled with an insulating material to further thermally isolate the sensor connected to sensor connection pads **3307.** In addition, the PCB assembly **3301** can be configured to position the sensor apart from the other active and/or passive electrical components, such as with the protruding feature **3313.**

The PCB assembly **3301** includes power connection pad **3315** for a diode electrically coupled to the heating filaments through conductive tracks on the PCB assembly **3301.** The power connection pad **3315** can be electrically and thermally coupled to heat sink **3317** to aid in dissipating heat, to reduce or minimize effects on the accuracy of the temperature reading of the thermistor coupled to the sensor connection pads **3307.**

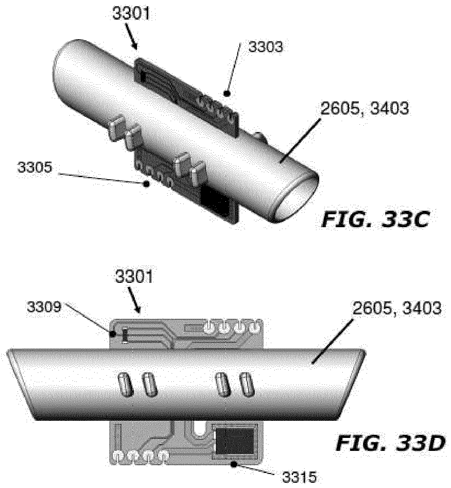

FIGS. **33C** and **33D** show the PCB assembly **2901** of FIGS. **33A** and **33B** in place across insert **2605,** discussed above with respect to FIGS. **30A-30O****,** or an intermediate connector **3403,** discussed below with respect to FIG. **34****.**

Thus, in at least one embodiment, a respiratory conduit segment, such as the insert **2605** or intermediate connector **3403,** comprises a lumen extending along a longitudinal axis and a wall surrounding the lumen, the lumen defining a gas-flow path when in use; and a PCB assembly **3301** comprising a printed circuit board and further comprising a first portion extending across the lumen along a diameter or chord line, such that a portion of the printed circuit board assembly generally bisects at least part of the flow path, the first portion being overmolded by an overmolding composition, a second portion adjacent the first portion projecting outward from the wall in a direction away from the lumen, the second portion comprising one or more connection pads **3303** on the printed circuit board configured to receive one or more wires from a first assembly, a third portion adjacent the first portion projecting outward from the wall in a direction away from the lumen and in a direction opposite the second portion, the third portion comprising one or more connection pads **3305** on the printed circuit board configured to receive one or more wires from a second assembly that is distinct from the first assembly, and one or more conductive tracks on the printed circuit board electrically coupled to the one or more connection pads of the second portion and to the one or more connection pads of the third portion and configured to provide electrical connectivity between the first assembly and the second assembly.

The first assembly and second assembly can each be a breathing tube. Or the first assembly can be a breathing tube and the second assembly can be a patient interface, for example.

Returning again to the examples of FIGS. **30G-30I****,** the sensor portion **3017** is mounted or formed such that the sensor portion **3017,** PCB **3015,** and locating portion **3019** form a unit. For example, sensor portion **3017,** PCB **3015,** and locating portion **3019** can be mounted to each other using a suitable process such as soldering. Sensor portion **3017,** PCB **3015,** and locating portion **3019** can be unitarily formed from a suitable material, such as a circuit board substrate.

The sensor portion **3017** can be electrically connected to the PCB **3015** using a suitable technique, such as circuit printing. For example, the electrical connection can comprise conductive tracks, such as copper tracks. To electrically connect the conductive filaments in the second elongate member of the tube **201** to the connection pads of the PCB assembly **3003,** a procedure similar to that shown and described above with respect to FIGS. **25E-25I** can be used. Additional electrical components, such as diodes (not shown), can be located at either side of the PCB **3015** inside and/or outside of the gas path. Locating a diode outside the gas path is discussed above with reference to sensor connection pads **3307** and signal connection pads **3309** and as shown in FIGS. **33A-33B****.**

Returning again to the examples of FIGS. **30G-30I****,** the PCB assembly **3003** can be mounted within the insert **3005** using, for example, an overmolding process, as known in the art. A material having a thermal conductivity in the range of 0.03-0.6 W/m·K or thereabout, such as polypropylene (thermal conductivity 0.1-0.22 W/m·K), can be used for at least a portion of the overmolding. Use of a material with low thermal conductivity can advantageously reduce interference from the ambient environment during sensor measurements, as it poorly conducts heat from the sensor portion **3017** to the insert **3005** walls. Certain embodiments include the realization that overmolding of a unitary PCB assembly **3003** allows more consistent placement of a sensor than overmolding a sensor alone. Furthermore, certain embodiments include the realization that overmolding a sensor placed inside center of the tube may make the sensor less sensitive to radiant effects.

As shown in FIGS. **30G-30I****,** the PCB assembly **3003** passes through the width of the insert **3005** and is supported by opposite walls of the insert **3005.** Because the PCB assembly **3003** is supported on opposite sides of the insert **3005,** the PCB assembly **3003** can be relatively thin (that is, having less thickness and less width than a PCB with one support on the tube). The thin profile can promote fluid flow by providing less resistance to flow than a thicker profile.

The overmolding around the sensor portion **3017** is preferably configured to reduce the drag on fluids flowing around the sensor portion **3017.** The overmolding can have an aerodynamically efficient tapered shape, such as an airfoil shape, for example, a wing shape, a fully tapered torpedo shape (as shown in FIGS. **30F** and **30G**), or bullet shape that is partially tapered with one blunt edge (as shown in FIG. **30H**). These tapered shapes promote fluid flow. In addition, when placed within the fluid flow, these tapered shapes reduce turbulence and vortices at the trailing edge of the tapered shape, which can cause unwanted cooling of humidified gas and forming of condensation. Condensation formation can lead to inaccurate measurement, as well as unwanted temperature drop in the gas delivered to a patient. Thus, the tapered shape can promote more accurate readings. In addition, the tapered shape can reduce collection of condensate that does form and also reduce buildup of patient secretions, by promoting runoff.

The tapered shape can also be selected to reduce turbulent flow by reducing the formation of vortices in the flow and increase the likelihood that the flow remains laminar.

The distance between the tapered shape and the inner wall of the insert **3005** is preferably selected to allow for more space. In at least one embodiment, the distance between tapered shape and the inner wall of the insert **3005** is at least 10% (or about 10%) or at least 30% (or about 30%), such as 33% (or about 33%) or 40% (or about 40%) of the inner diameter. In at least one embodiment, the distance between the tapered sjape and the inner wall of the insert **3005** is greater than 2 mm (or about 2 mm). Allowing for more space decreases the likelihood that condensate becomes trapped in the space.

The overmolding promotes reading a more averaged temperature. There is some temperature variation across the insert **3005,** with higher temperatures toward the center of the insert **3005** and lower temperatures along the insert **3005** walls. An asymmetric temperature profile, in which the highest temperature is offset from the center line of the insert **3005,** is especially prevalent with bent tubes **203.** The overmolding has a larger surface area than the sensor portion **3017** of the PCB assembly **3003** and the overmolding material distributes the heat so that that the sensor of the sensor portion **3017** measures a more averaged temperature across the fluid path.

FIG. **30J** shows an end view of the connector **3000** taken along the width of the connector, as seen from the patient end portion **3001** of the connector **3000,** and looking toward the tube (not shown). In this view, the overmolded tapered shape housing the PCB assembly **3003**(not shown) is generally centered. FIG. **30K** shows an alternative configuration. In this view, the tapered shape is offset from the center line. As shown in FIGS. **30J** and **30K****,** the junctions **3018** between the inner wall of the insert **3005** and the overmolded tapered shape housing the PCB assembly optionally can have fillets to reduce flow disturbance and reduce areas for fluid build-up. The fillets of the junctions **3018** can be 1 mm (or about 1 mm) in radius, for example.

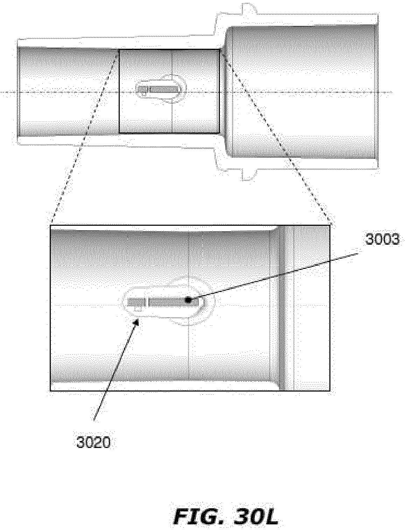

FIG. **30L** shows the offset positioning of the tapered shape of FIG. **30K** in greater detail. Because the sensor **3020** projects outward from the PCB assembly **3003,** the offset configuration can improve accuracy by placing the sensor **3020** closer to the center line. In addition, the offset configuration can also be desirable because the PCB assembly **3003** can be housed in one side of the mold tool during manufacturing, thereby simplifying the manufacturing process.

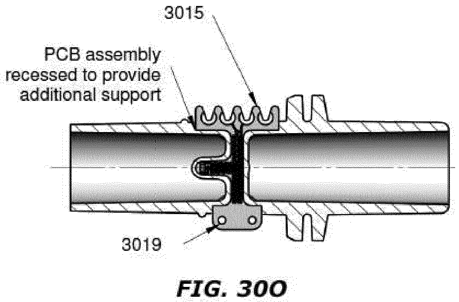

FIG. **30M** shows a longitudinal cross section of the insert assembly **3007** showing additional details of the PCB assembly **3003.** A sensor **3020** is placed into the flow path. The sensor **3020** can provide temperature and/or gas flow information to allow assessment of conditions near the patient interface. The sensor **3020** is preferably located close to the edge of the projecting part of the sensor portion **3017.** The thickness of the overmolding proximal the sensor **3020** is preferably thinner than the thickness of the overmolding around the other portions of the PCB assembly **3003,** as shown in FIG. 30**O**. Reducing the overmolding thickness increases heat transfer to promote more accurate temperature measurements.

With reference again to FIG. **30M****,** conductive tracks **3021** electrically connect the sensor **3020** to the PCB **3015.** (Note that sensor **3020** is not specifically shown in FIG. **30M****;** rather the structure designated **3020** represents the general position of the sensor. The structure designated **3020** shows two conductive pads that the sensor would bridge across. The structure is designated as a sensor for sake of illustration.) Through holes **3023** allow components to make contact with the required conductive layers. FIG. **30N** shows an alternative configuration of the PCB assembly **3003.** In FIG. **30N****,** the conductive tracks **3021** have a tortuous path. It was realized that increasing the length of the conductive tracks **3021** within the fluid path allows the temperature of the conductive tracks **3021** to more closely reflect the temperature within the fluid path, thereby reducing ambient effects on the sensor **3020** through the conductive tracks **3021.** Preferably, there is increased surface area of copper near the sensor **3020.** The increased copper promotes accurate detection of temperature around the sensor **3020** area.

In certain embodiments, the tapered shape can extend upstream along the gas path towards the source of the gas flow. This configuration promotes more accurate measurement by ensuring the sensor **3020** projects into the fluid flow, before the fluid is cooled as it passes the overmolding. This configuration can also promote more accurate measurements by reducing the "stem effect." All contact-type temperature sensors are subject to stem effect. When a probe is immersed in a fluid flow, a thermally conductive path is created by the probe's stem. In a case where the ambient temperature is cooler than the temperature of the measured fluid stream, heat is conducted away from the probe tip via the probe's stem to the outer atmosphere. This results in the sensing tip reading a temperature that is lower than the actual surrounding fluid. And in a case where the ambient temperature is hotter than the temperature of the measured fluid stream, heat is conducted toward the probe tip via the probe's step from the outer atmosphere. This results in the sensing tip reading a temperature that is higher than the actual surrounding fluid. The tapered shape configuration reduces the stem effect by projecting the sensor **3020** away from the part of the sensor portion **3017** connecting the PCB **3015** and locating portion **3019** (that is, away from the "stem"). In certain embodiments, the tapered shape extends upstream by at least 6 mm (or about 6 mm) from the part of the sensor portion **3017** connecting the PCB **3015** and locating portion **3019.**

In certain embodiments, the tapered shape can extend downstream away from the source of the gas flow. This configuration can be advantageous, for example, when the design of the overmolded PCB assembly **3003** significantly alters the average downstream fluid properties such that it is desirable to accurately measure the fluid properties leaving the tube.

Heating filaments (not shown here but described above) in the second elongate member can be connected to the PCB **3015,** which can provide termination points to complete the heating filament circuit. The PCB **3015** can also be used to provide additional termination points to provide power to additional heating filaments in a secondary tube, such as in a segmented inspiratory limb configuration for use with a humidification system, the segmented inspiratory limb having a connector configured to couple heating filaments and sensors in two segments. A suitable PCB assembly configuration is discussed above with reference to FIGS. **33A-33D****.**

Returning again to FIG. **30M****,** this configuration eliminates the need to have a separate power line running to the heating filaments. This configuration further ensures that the heating filaments run along the tube **203** and terminate at approximately the same position on the tube **203** as the sensor **3020.** Thus, the configuration minimizes temperature drop from the end of the heating filaments to the sensor **3020.** This configuration can also reduce temperature drop from the end of the heating filaments and a second heating filament in an additional section of tube. The configuration can also be used to heat the covering connector of the sensor **3020,** thereby reducing heat losses to a cold ambient, and further improving accuracy in temperature measurement.

Although the foregoing describes placing one or more sensors at the patient end of a tube **201,** it should be appreciated that this sensor configuration can be applied along any part of the fluid pathway of a tube **201.**

For example, FIG. **34** illustrates a portion of a segmented inspiratory limb **3401** for use with a respiratory humidification system, the segmented inspiratory limb **3401** comprising a first segment **3401a** and a second segment **3401b** and having an intermediate connector **3403** configured to couple first heater wires **3405a** to second heater wires **3405b** and a first temperature sensor **3407a** to a second temperature sensor **3407b** in the respective segments **3401a** and **3401b.** Coupling the two segments **3401a** and **3401b** can comprise mechanically coupling the segments to form a single can be conduit through which humidified gases can be delivered to a user wherein mechanically coupling the segments **3401a** and **3401b** can result in electrically coupling the respective heater wires **3405a, 3405b** and the respective temperature sensors **3407a, 3407b** through the intermediate connector **3403.** The PCB assembly **3301** shown in FIGS. **33A** and **33B** is suitable for use with the intermediate connector **3403** of FIG. **34****.**

Returning again to FIG. **34****,** the segmented inspiratory limb **3401** can comprise a structure **3409** forming a lumen through which humidified gases can pass. The structure **3409** can include paths formed within walls of the structure **3409** configured to house heater wires **3405a** or **3405b** such that the heater wires **3405a** or **3405b** are shielded from the humidified gases travelling through the lumen and/or are covered by an external surface of the structure **3409** so that they are not exposed. For example, the structure **3409** can be a composite tube wherein the heater wire paths are coils molded into the tube, as discussed above. The structure **3409** can comprise any type of suitable material and can include insulating material and/or flexible material. In some embodiments, the structure **3409** and the intermediate connector **3403** can be configured such that, when the first and second segments **3401a** and **3401b** are mechanically coupled, the heater wires **3405a** and **3405b** wrap over the intermediate connector **3403** in such a way as to be electrically coupled to the intermediate connector **3403.** In some embodiments, the first segment **3401a** and/or the intermediate connector **3403** can exclude any flying leads for connecting to the second segment **3401b,** thereby facilitating connection of the second segment **3401b** to the first segment **3401a.**

The structure **3409** at complementary ends of the first and second segments **3401a** and **3401b** can be configured to house the intermediate connector **3403.** Thus, the intermediate connector **3403** can be internal to the inspiratory limb **3401.** In some embodiments, the complementary ends of the first and second segments **3401a** and **3401b** can be configured to shield the intermediate connector **3403** from humidified gases travelling through the inspiratory limb **3401.** In some embodiments, the intermediate connector **3403** is both internal to the inspiratory limb **3401** and shielded from humidified gases in the conduit, thereby reducing or eliminating exposure of electrical connections on the intermediate connector **3403.**

In some embodiments, the first heater wires **3405a** can comprise two wires **3411** and **3413** and the second heater wires **3405b** can comprise two wires **3415** and **3417.** The two wires **3411** and **3413** in the first segment **3401a** can be electrically coupled to one another through electrical components **3419** wherein the electrical coupling creates an electrical path through the wire **3411,** at least a portion of the electrical components **3419,** and the wire **3413.** Similarly, the two wires **3415** and **3417** in the second segment **3401b** can be electrically coupled to one another through electrical components **3419** and/or electrically shorted together at an end of the segment **3401b** opposite the intermediate connector **3401b,** such as through a patient-end connector (not shown). By coupling the wires **3415** and **3417** of the second segment **3401b** at the intermediate connector **3403,** electrical connections at the patient-end of the inspiratory limb **3401** are reduced or eliminated which can reduce cost, system complexity, and/or risk to the patient.

The intermediate connector **3403** can be configured to allow a single controller to control power to the heater wires **3405a, 3405b,** such as a humidifier controller. In some embodiments, the humidifier controller controls the heater wires **3405a, 3405b** without any additional control functionality located on the intermediate connector **3403.** For example, the intermediate connector **3403** can include passive components without any logic circuitry wherein the passive components direct power to heater wires **3405a** and/or **3405b** as selected by the controller. This can allow the intermediate connector **3403** to be designed using relatively inexpensive components and can reduce the complexity of the design.

In some embodiments, heating of the two segments **3401a** and **3401b** can be accomplished using a maximum of four wires in each segment **3401a, 3401b.** For example, in the first segment **3401a** the four wires can include a first heater wire **3411,** a second heater wire **3413,** a signal temperature sensor wire **3419,** and a return temperature sensor wire **3421.** In the second segment **3401b** the four wires can include a first heater wire **3415,** a second heater wire **3417,** a signal temperature sensor wire **3423,** and a return temperature sensor wire **3425.** By coupling the second heater wires **3415, 3417** to the first heater wires **3411, 3413** at connection points **3427,** and by coupling the second temperature sensor wires **3423, 3425** to the first temperature sensor wires **3419, 3421** at connection points **3427,** a controller can be configured to provide power independently to the first heater wires **3405a** and the second heater wires **3405b** and to read temperature sensor data independently from the temperature sensors 204a and 204b without including more than four wires in either segment **3401a** or **3401b.** In some embodiments, control of the heater wires **3405a** and **3405b** and reading of the temperature sensors **3407a** and **3407b** can be accomplished using less than four wires in each segment (*e.g*., using three wires or using two wires) or more than four wires in each segment (*e.g*., using five wires, using six wires, using seven wires, using eight wires, or using more than eight wires).

The intermediate connector **3403** can include electrical components **3419** configured to allow a controller to selectively control heater wires **3405a, 3405b.** The controller can be configured to control heating of the inspiratory limb **3401** using two modes wherein a first control mode comprises providing power to the heater wires **3405a** in the first segment, and a second control mode comprises providing power to the heater wires **3405a** and **3405b** in the first and second segments **3401a** and **3401b.** Thus, the controller can be configured to independently control heater wire sections. This ability allows for the controller to control heating of the inspiratory limb **3401** when the second segment **3401b** is not present by solely controlling the heating of the inspiratory limb according to the first control mode, thereby allowing for the respiratory humidification system to be used in a variety of circumstances without modifying the controller or humidification unit. In some embodiments, the control modes can include a mode where power is delivered only to the heater wires **3405b** in the second segment **3401b.** In some embodiments, the controller includes an electrical power source that provides electrical current. The first and second control modes can be based at least in part on the voltage supplied by the power source wherein a positive voltage or positive current can trigger the first control mode and a negative voltage or a negative current can trigger the second control mode. In some embodiments, the power source provides rectified AC or DC power to the heater wires **3405a, 3405b** and a change in the rectification or polarity triggers a change in the control mode. By switching control modes, control of heating in the breathing circuit can be accomplished with any power supply that can switch the polarity of the output signal. In some embodiments, the amount of power provided to the heater wires **3405a, 3405b** can be adjusted by adjusting a duty cycle of power applied to the heater wires **3405a, 3405b.** For example, pulse-width modulation (PWM) can be used to power the heater wires **3405a, 3405b** and the duty cycle of the PWM signal can be adjusted to control the power delivered. In another example, the amount of power provided to the heater wires **3405a, 3405b** can be adjusted by controlling the amplitude of the power signal.

The intermediate connector **3403** can include electrical components **3421** configured to allow a controller to selectively read temperature sensors **3407a, 3407b.** Selective reading can be accomplished through the use of a source of electrical current wherein applying a positive current across the wires **3419** to **3421** can result in the controller measuring a temperature-related signal from the first temperature sensor **3407a** and applying a negative current across the wires **3419** and **3421** can result in the controller measuring a temperature-related signal from the second temperature sensor **3407b** or from both the first and second temperature sensors **3407a, 3407b.** The controller can use the readings from the temperature sensors **3407a, 3407b** to adjust power to the heater wires **3405a, 3405b,** using, for example pulse-width modulation. The first temperature sensor **3407a** can be positioned near the connection or intersection of the first and second segments **3401a** and **3401b** to provide to the controller a temperature of gases entering the second segment **3401b,** which can correspond to entering an incubator or other such region having a different ambient temperature. The second temperature sensor **3407b** can be positioned at a patient-end of the second segment **3401b** to provide to the controller a temperature of gases delivered to the patient or the temperature prior to the final piece before the patient, such as a wye-piece. The controller can use these temperature readings to adjust power to the heater wires **3405a, 3405b** to maintain the temperature of the gas at the patient-end of the inspiratory limb **3401** at a targeted or suitable temperature. The targeted or suitable temperature can vary depending at least in part on the application and environment it is being used in, and can be about 37°C, about 40 °C, at least about 37°C and/or less than or equal to about 38°C, at least about 36.5°C and/or less than or equal to about 38.5°C, at least about 36°C and/or less than or equal to about 39°C, at least about 35°C and/or less than or equal to about 40°C, at least about 37°C and/or less than or equal to about 41°C, or at least about 39.5°C and/or less than or equal to about 40.5°C. In some embodiments, the second temperature sensor **3407b** can be positioned inside the incubator but not attached to the breathing circuit. By measuring the temperature inside the incubator, the temperature of the second segment **3401b** can be calculated.

The controller can independently control the amount of power delivered in the first and second control modes, as described herein. Based at least in part on feedback from the temperature sensors **3407a** and/or **3407b,** the controller can independently adjust power delivered in the first and second control modes, thereby resulting in varying heater power ratios between the first and second segments **3401a** and **3401b.**

In some embodiments, the first temperature sensor **3407a** is positioned within the flow of gas within the inspiratory limb **3401.** In some embodiments, the intermediate connector **3403** or the first segment **3401a** can include a mechanical component that decreases turbulence in the flow of the gas across the first temperature sensor **3407a** which can increase accuracy in the temperature sensor's **3407a** readings. In some embodiments, the mechanical component (e.g., a cross-member feature within the inspiratory conduit) that decreases turbulence also secures the temperature sensor **3407a** within the flow of the gases. In some embodiments, the intermediate connector **3403** and the mechanical component are configured to thermally isolate the temperature sensor **3407a** from the electrical components on the intermediate connector **3403.**

In some embodiments, the intermediate connector **3403** includes additional connection points in addition to the connection points **3427** illustrated in FIG. **34****.** The additional connection points can be used to incorporate further functionality into the breathing circuit such as, for example, incorporating a memory device (PROM), a microcontroller, additional circuits, and the like.

Moreover, the composite tube **201** can be an inspiratory tube or an expiratory tube.

### Placement of Spiral-Style Connector

Reference is next made to FIGS. **35A-35F** which show a connector without electrical connectivity to a PCB. However, as will be appreciated by those skilled in the art, that the connector could be equally adapted to have electrical connectivity to a PCB. The connector is suitable for connecting to, for example, a patient interface or a humidifier. It is particularly suited for use as a patient-end connector and/or device-end connector in an obstructive-sleep apnea environment.

A spiral-ended molded insert **3501** is provided. The end of the insert **3501** opposite the spiral end is molded for insertion on or attachment to a humidifier port, and/or a patient interface port, and/or any other desired component. The insert **3501** can be a hard material, such as a hard plastic, for example, polypropylene.

As shown in FIG. **35C****,** the spiral end of the insert **3501** is screwed into the compliant turns of the tube **201.** In this example, the spiral turns of the insert **3501** are sized and configured to fit into the turns of the first elongate member **203** of the tube **201.**

It should be noted that, in the case of a tube having one or more electrically powered wires therein, an electrical connection can be provided on at least a portion of the insert **3501.** When the insert **3501** is installed, the electrical connector will preferably align with the wires, thereby facilitating electrical connection. Solder or the like can then be used to secure the connection.

A member **3503** can be inserted or molded on top of at least a portion of insert **3501** and, optionally, tube **201** to promote the attachment between the insert **3501** and the tube **201.** The member **3503** can be a hard material or a soft material, such as a soft plastic, rubber, or PTFE, for example polypropylene. In some cases, the insert **3501** (or at least the spiral end of the insert **3501**) provides sufficient lateral crush resistance to enable high-pressure molding techniques to be used, where the pressure can exceed the lateral crush resistance of the tube **201** without the insert **3501.** Member **3503** can also advantageously provide a soft surface to grip on when inserting and removing tube from a component.

The foregoing method of attaching a connector to a spiral-wound tube is provided by way of example. The method described herein does not imply a fixed order to the steps. Nor does it imply that any one step is required to practice the method. Embodiments may be practiced in any order and combination that is practicable.

### Placement of Alternative Patient-End Connector

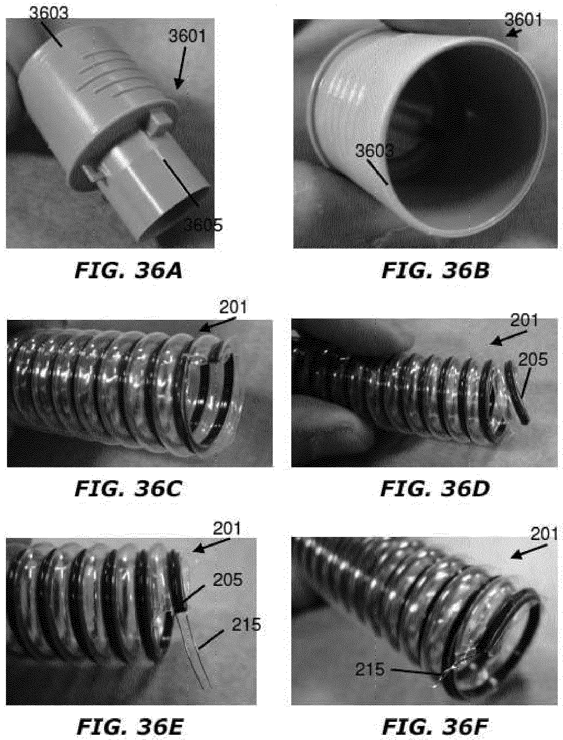

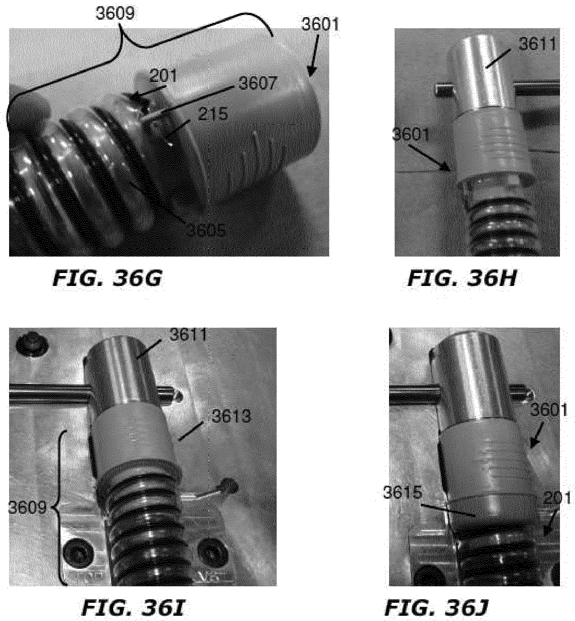

Reference is next made to FIGS. **36A-36K****.** FIGS. **36A** and **36B** show a patient-end connector **3601** without electrical connectivity. The connector **3601** has a patient end **3603** with a standard-size medical taper suitable for use with a patient interface. The tube end **3605** of the connector **3601** is suitable for connection to a composite tube **201,** as described below. The connector **3601** is preferably a pre-molded component formed from a suitable material such as plastic, rubber, or PTFE.

As shown in FIGS. **36C** and **36D****,** a portion (e.g., a 10-mm portion) of the second elongate member **205** is stripped away to reveal a small length of the one or more filaments **215** embedded therein. Preferably, about 5 mm or 10 mm of the filaments **215** are revealed. As shown in FIG. **36D****,** the filaments **215** are twisted together and optionally secured, for example, by soldering, thereby creating a closed loop circuit.

Turning next to FIG. **36G****,** the tube end **3605** of the connector **3601** is inserted into the tube **201** and the twisted filaments **215** are placed under a retaining loop **3607.** The retaining loop **3607** diminishes movement of the filaments **215** during molding. The retaining loop **3607** also advantageously aligns the rotational pitch of the composite tube **201** to the connector **3601,** which in turn promotes proper alignment of the tube **201** in the mold. The combination of connector **3601** and composite tube **201** is designated here as a connector-tube assembly **3609.**

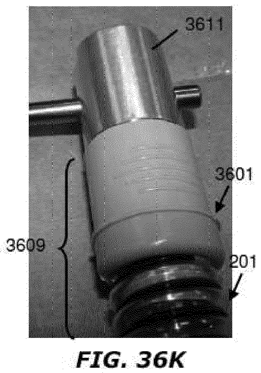

As shown in FIG. **36H****,** a mold tool core **3611** is inserted into the connector **3601.** As shown in FIG. **361,** the connector-tube assembly **3609** and core **3611** are placed in an injection mold tool **3613.** In FIG. **36J****,** a molded material **3615** is molded over junction region between the composite tube **201** and the connector **3601,** thereby bonding composite tube **201** and the connector **3601.** Suitable molded materials **3615** include plastic and rubber. The connector-tube assembly **3609** and core **3611** are removed from the injection mold tool (not shown), as in FIG. **36K****.** The core **3611** is removed, thereby providing a composite tube **201** with a patient-end connector **3601.**

The foregoing method of attaching a connector to a composite tube is provided by way of example. The method described does not imply a fixed order to the steps. Nor does it imply that any one step is required to practice the methods. Embodiments may be practiced in any order and combination that is practicable.

The foregoing description of the invention includes preferred forms thereof. Modifications may be made thereto without departing from the scope of the invention. To those skilled in the art to which the invention relates, many changes in construction and widely differing embodiments and applications of the invention will suggest themselves without departing from the scope of the invention as defined in the appended claims. The disclosures and the descriptions herein are purely illustrative and are not intended to be in any sense limiting.

Through the description and the claims, the terms "comprises," "comprising," and the like are to be construed in an inclusive sense, that is, in the sense of "including but not limited to," unless the context clearly requires otherwise.

Although the invention has been described by way of example and with reference to possible embodiments thereof, it is to be understood that modifications or improvements may be made thereto without departing from the spirit and scope of the invention and without diminishing its attendant advantages. Furthermore, where reference has been made to specific components or integers of the invention having known equivalents, such equivalents are herein incorporated as if individually set forth.

Any discussion of the prior art throughout the specification should in no way be considered as an admission that such prior art is widely known or forms part of the common general knowledge in the field anywhere in the world.

It will be appreciated that there is thus disclosed:
According to the present invention there is provided a breathing tube comprising: a first elongate member comprising a hollow body spirally wound to form at least in part an elongate tube having a longitudinal axis, a lumen extending along the longitudinal axis, and a hollow wall surrounding the lumen, the wall having an inner portion proximal the lumen and an outer portion facing away from the lumen, wherein the inner portion of the wall has a smaller thickness than the outer portion of the wall.

Preferably further comprising a second elongate member spirally wound and joined between adjacent turns of the first elongate member, the second elongate member forming at least a portion of the lumen of the elongate tube.

Preferably the thickness of the outer portion of the wall is in the range of about 0.14 mm and about 0.44 mm.

Preferably the thickness of the outer portion of the wall is about 0.24 mm.

Preferably the thickness of the inner portion of the wall is in the range of about 0.05 mm and about 0.30 mm.

Preferably the thickness of the inner portion of the wall is about 0.10 mm.

According to the present invention there is provided a breathing tube comprising: a first elongate member comprising a hollow body component, wherein the weight per length of the tube within at least a portion of the 300 mm nearest an end of the tube is less than about 0.08 g/mm.

Preferably the first elongate member comprises a hollow body spirally wound to form at least in part an elongate tube having a longitudinal axis, a lumen extending along the longitudinal axis, and a hollow wall surrounding the lumen.

Preferably further comprising a second elongate member spirally wound and joined between adjacent turns of the first elongate member, the second elongate member forming at least a portion of the lumen of the elongate tube.

Preferably comprising one or more conductive filaments embedded or encapsulated within the second elongate member.

Preferably at least one of the one or more conductive filaments is a heating filament.

Preferably at least one of the one or more conductive filaments is a sensing filament.

Preferably the tube mass in the 300 mm nearest an end of the tube is less than about 24 g.

Preferably the weight per length of the tube within at least a portion of the 300 mm nearest an end of the tube is less than about 0.06 g/mm.

Preferably the tube mass in the 300 mm nearest an end of the tube is less than about 16 g.

Preferably the thickness of the wall is at most about 0.50 mm.

According to the present invention there is provided a breathing tube comprising: a first elongate member comprising a hollow body spirally wound to form at least in part an elongate tube having a longitudinal axis, a lumen extending along the longitudinal axis, and a hollow wall surrounding the lumen, the wall having an inner portion proximal the lumen and an outer portion facing away from the lumen, wherein, in at least a portion of the composite tube, when force is applied to the outer portion of the wall with a 2.5-mm probe and until the outer portion of the wall contacts the inner portion., the outer portion deflects by a vertical distance that satisfies the equation: D > 0.5×F2.5, where D represents the vertical distance in millimeters, and F2.5 represents the force in Newtons applied by the 2.5-mm probe.

Preferably further comprising a second elongate member spirally wound and joined between adjacent turns of the first elongate member, the second elongate member forming at least a portion of the lumen of the elongate tube.

Preferably the outer portion deflects more than about 1 mm when a force of about 1 N is applied with the 2.5-mm probe.

According to the present invention there is provided a respiratory conduit comprising: a lumen extending along a longitudinal axis and a wall surrounding the lumen, the lumen defining a gas-flow path when in use; and an overmolded printed circuit board assembly secured to the wall, the printed circuit board assembly comprising a printed circuit board with a sensor portion disposed in the lumen of the conduit and projecting along the longitudinal axis, and a temperature sensor on a surface of the sensor portion.

Preferably the temperature sensor is a thermistor.

According to the present invention there is provided a respiratory conduit comprising: a lumen extending along a longitudinal axis and walls surrounding the lumen, the lumen defining a gas-flow path when in use; and a component secured to the walls and extending across the lumen along a diameter or chord line, such that the component generally bisects at least part of the flow path, the component comprising a sensor portion disposed in the lumen and projecting along the longitudinal axis, and a temperature sensor on a surface of the sensor portion.

Preferably the temperature sensor is a thermistor.

Preferably the component is a printed circuit board.

Preferably the electrical connection spans the component's length along the diameter or chord line.
Also disclosed herein are the following items:
1. A respiratory conduit comprising:
   a lumen extending along a longitudinal axis and a wall surrounding the lumen, the lumen defining a gas-flow path when in use; and,
   a component securely connected to the wall and extending across the lumen, the component comprising:
      at least a Printed Circuit Board (PCB) with a sensor portion disposed in the lumen and projecting along the longitudinal axis; and,
      a temperature sensor provided on a surface of the sensor portion, wherein the temperature sensor is positioned longitudinally upstream along the gas-flow path from a connection of the component to the wall.
2. The respiratory conduit of item 1, wherein the component comprises a dividing portion embedded in the wall and extending across the lumen along a diameter or chord line, such that the dividing portion generally bisects at least part of the gas-flow path, at least part of the dividing portion being overmolded by an overmolding composition.
3. The respiratory conduit of item 2, wherein the component comprises a wiring portion adjacent the dividing portion and projecting outward from the wall in a direction away from the lumen, the wiring portion being configured to provide an electrical connection to the temperature sensor.
4. The respiratory conduit of item 3, wherein the component comprises a support portion adjacent the dividing portion and projecting outward in a direction away from the lumen and in a direction opposite to the wiring portion.
5. The respiratory conduit of any one of items 2-4, wherein the sensor portion projects from the dividing portion along the longitudinal axis, the sensor portion being overmolded by the overmolding composition.
6. The respiratory conduit of any one of items 1-5, wherein the sensor portion projects upstream of the gas-flow path and the temperature sensor is disposed adjacent an upstream leading edge of the sensor portion.
7. The respiratory conduit of item 5 or item 6 when dependent on item 5, wherein the overmolding composition proximal the sensor portion comprises a tapered shape extending along the longitudinal axis and/or an airfoil shape, a wing shape, a torpedo shape, or a bullet shape.
8. The respiratory conduit of item 6 when dependent on item 5, or item 7, wherein a thinnest part of the overmolding composition is proximal the upstream leading edge of the sensor portion.
9. The respiratory conduit of any one of items 1-8, wherein a vertical distance between the sensor portion and the wall is at least 30% of a diameter of the lumen.
10. The respiratory conduit of any one of items 1-9, wherein the temperature sensor is a thermistor.
11. The respiratory conduit of any one of items 1-10, comprising a patient-end connector configured to connect a tube to a patient interface, wherein the patient-end connector comprises the component.
12. The respiratory conduit of item 11, wherein the patient-end connector comprises an insert configured to be connected at one end to the tube and at another end to the patient interface, the insert forming at least a part of the lumen defining the gas-flow path and configured to provide a housing for the PCB and the temperature sensor of the component.
13. The respiratory conduit of item 12, wherein the patient-end connector comprises a cover configured to protect and cover the PCB and the connection of the insert to the tube.
14. The respiratory conduit of any one of items 1-13, comprising a/the tube, wherein the PCB is configured to be connected to one or more conductive filaments provided in a wall of the tube.
15. The respiratory conduit of item 14, wherein the tube comprises:
   a first elongate member having a hollow body;
   a second elongate member spirally wound and joined between adjacent turns of the first elongate member; and,
   the one or more conductive filaments comprises at least one heating filament and/or at least one sensing filament embedded or encapsulated within the second elongate member.

## Claims

1. A breathing tube for delivering humidified gases to a patient, the breathing tube comprising:
a first end and a second end;
a connector at the first end of the tube comprising a lumen extending along a longitudinal axis and a wall surrounding the lumen, the lumen configured to define a gas-flow path when in use;
a circuit board assembly comprising:
a wiring portion comprising electrical contacts configured to be coupled to at least two conductive wires of the breathing tube, the wiring portion projecting outwardly of the connector in a direction away from the lumen;
an extending portion extending across the lumen along a diameter or chord line and comprising a sensor portion, the sensor portion being positioned within the gas-flow path and comprising at least one temperature sensor;
one or more conductive tracks configured to electrically connect the at least one temperature sensor to the electrical contacts; and,
an overmolding composition provided over at least over the sensor portion; and,
at least one diode electrically coupled to the at least one temperature sensor;
the circuit board assembly being configured to position the at least one temperature sensor apart from the at least one diode to thermally insulate the at least one temperature sensor from the at least one diode.

2. The breathing tube of claim 1, wherein the printed circuit board assembly is configured to position the at least one temperature sensor apart from the at least one diode by inclusion of a gap.

3. The breathing tube of claim 1 or 2, wherein the printed circuit board assembly is overmolded by the overmolding composition.

4. The breathing tube of any one of claims 1 to 3, wherein the at least one temperature sensor is located on a surface of the sensor portion and/or close to an edge of the sensor portion, is the at least one temperature sensor being configured to assess conditions near a patient interface.

5. The breathing tube of any one of claims 1 to 4, wherein the overmolding composition comprises a thermal conductivity in the range of 0.03 to 0.6 W/m-K.

6. The breathing tube of any one of claims 1 to 5, wherein the circuit board assembly is configured to position the at least one temperature sensor apart from other active and/or passive electrical components.

7. The breathing tube of any one of claims 1 to 6, wherein the one or more conductive tracks have a tortuous path.

8. The breathing tube of any one of claims 1 to 7, wherein the circuit board assembly is a Printed Circuit Board assembly.

9. The breathing tube of any one of claims 1 to 8, wherein a thinnest part of the overmolding composition is proximal an edge of the sensor portion.

10. The breathing tube of any one of claims 1 to 9, wherein the at least one temperature sensor is a thermistor.

11. The breathing tube of any one of claims 1 to 10, wherein the circuit board assembly is supported by at least one of the walls of the connector.

12. The breathing tube of any one of claims 1 to 11, wherein the at least two conductive wires comprise heating wires and/or sensing wires.

13. The breathing tube of claim 12, wherein the at least two conductive wires are heating filaments, the heating filaments being connected to the electrical contacts of the wiring portion to provide termination points and wherein the heating filaments:
comprise two heating filaments embedded or encapsulated in walls of the breathing tube; and/or,
run along the breathing tube and terminate at approximately the same position on the tube as the at least one temperature sensor.

14. The breathing tube of claim 12 or 13, wherein the circuit board assembly completes: a heating circuit of the tube formed by the heating wires; and/or, a sensing circuit of the tube formed by the sensing wires.

15. The breathing tube of any one of claims1 to 14, wherein the circuit board assembly is configured such that the electrical contacts of the wiring portion are positioned outside of the gas-flow path.

16. The breathing tube of any one of claims 1 to 15, comprising:
an additional connector at the second of the breathing tube; and,
an elongate body defining a lumen and being in fluid communication with the connector and the additional connector such that the connector, the additional connector and the elongate body form a gas-flow path between the first end and the second end for the humidified gases, when in use.

17. The breathing tube of claim 16, wherein the at least two conductive wires are spirally wound along the length of the elongate body.

18. The breathing tube of claim 16 or 17, wherein the additional connector is configured to removably connect the breathing tube in use to a flow generator, wherein the at least two conductive wires terminate at least two electrical conductors of the additional connector.

19. The breathing tube of claim 16 or 17, wherein an electrical connector of the additional connector electrically couples the at least one temperature sensor to at least one controller of a breathing assistance apparatus or a humidification device when the additional connector is coupled to the breathing assistance apparatus or humidification device.

20. The breathing tube of any one of claims 1 to 19, wherein the connector is configured to at least pneumatically connect the tube in use to a patient interface.

21. The breathing tube of any one of claims 16 to 20, wherein the at least two conductive wires are connected to: an electrical connector of the additional connector and the electrical contacts of the circuit board assembly of the connector to be in electrical communication with the at least one temperature sensor.
